(19) Europäisches Patentamt
European Patent Office
Office européen des brevets

(11) Veröffentlichungsnummer: **0 309 746**
**A1**

(12) **EUROPÄISCHE PATENTANMELDUNG**

(21) Anmeldenummer: **88114016.4**

(22) Anmeldetag: **27.08.88**

(51) Int. Cl.[4]: **C12N 15/00 , C07K 13/00 , C07H 21/04 , C12N 1/20 , C12P 21/00 , A61K 39/015 , A61K 37/02**

Der Mikroorganismus ist bei Deutsche Sammlung von Mikroorganismen unter der Nummer DSM 4232 hinterlegt worden.

Patentanspruch für folgenden Vertragsstaat:ES.

(30) Priorität: **08.09.87 CH 3486/87**

(43) Veröffentlichungstag der Anmeldung: **05.04.89 Patentblatt 89/14**

(84) Benannte Vertragsstaaten: **AT BE CH DE ES FR GB IT LI NL SE**


(71) Anmelder: **F. HOFFMANN-LA ROCHE & CO. Aktiengesellschaft**

**CH-4002 Basel(CH)**

(72) Erfinder: **Certa, Ulrich, Dr.**
**Steinbühlweg 21**
**CH-4123 Allschwil(CH)**

(74) Vertreter: **Mezger, Wolfgang, Dr. et al**
**Grenzacherstrasse 124 Postfach 3255**
**CH-4002 Basel(CH)**


(54) **Antimalaria-Vakzine.**


(57) Die Erfindung betrifft Polypeptide, die in mindestens einem spezifischen Epitop mit einem Plasmodium falciparum Merozoitenantigen mit einem Molekulargewicht von etwa 41'000 Dalton übereinstimmen, sowie ein Verfahren zu deren Herstellung. Weiter betrifft die Erfindung immunogene Kompositionen, die ein solches Polypeptid und ein geeignetes Adjuvans enthalten, eine DNA Sequenz, die für ein solches Polypeptid codiert, replizierbare mikrobielle Vektoren, die eine solche DNA Sequenz enthalten, Mikroorganismen, die einen solchen replizierbaren Vektor enthalten sowie Antikörper gegen ein erfindungsgemässes Polypeptid. Ferner betrifft die Erfindung Verfahren zur Herstellung der immunogenen Kompositionen, der Mikroorganismen und der Antikörper sowie die Verwendung der Polypeptide und der immunogenen Komposition zur Immunisierung von Säugern gegen Malaria.


Fig. 6

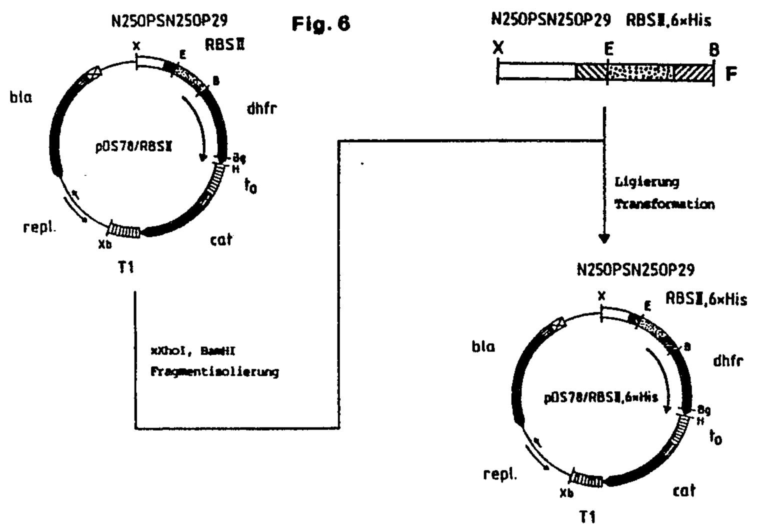


EP 0 309 746 A1

## Antimalaria-Vakzine

Malaria wird beim Menschen durch vier Spezies von Plasmodium verursacht, nämlich durch P. falciparum, P. vivax, P. ovale und P. malariae. Gemäss einem Bericht der Weltgesundheitsorganisation (WHO) aus dem Jahre 1986, gibt es weltweit fast 100 Millionen Fälle von Malariainfektionen. Davon haben etwa 1 Million, meistens Fälle von Kleinkindern, die mit P. falciparum infiziert sind, tödlichen Ausgang. Wegen dem Auftauchen von drogenresistenten Parasiten und von insektizidresistenten Moskitovektoren breitet sich die Malaria weiter aus. So berichteten die Gesundheitsbehörden Indiens 1962 von 100'000 Fällen von Malaria und 1980 schon von 3 Millionen Fällen, meistens verursacht durch P. vivax (vergl. Bruce-Chwatt, Essential Malariology, 2. Auflage, Heinemann, London [1986]).

Neuere technische Fortschritte haben die Hoffnung genährt, dass es bald möglich sein wird Antimalaria-Vakzine herzustellen, die der wachsenden Ausbreitung der Malaria entgegen wirken können. Erstens können neue Methoden bei der Entwicklung von Malariavakzinen angewendet werden, so z.B. die Klonierung von Genen und deren Expression in mikrobiellen Wirtsorganismen sowie die Verwendung von monoklonalen Antikörpern zur Antigenidentifizierung. Zweitens liefern Langzeitkulturen von P. falciparum in menschlichen roten Blutzellen (Trager et al., Science 193, 673-675 [1976]) auf bequeme Art und Weise Material zum Studium des Malaria-Parasiten. In neuerer Zeit wurde es möglich alle Stadien im Lebenszyklus des Parasiten im Labor zu züchten (Ponnudurai et al., Trans. R. Soc. Trop. Med. Hyg. 76, 812-818 [1982]; Mazier et al., Science 227, 440-442 [1985]).

Der natürliche Lebenszyklus von P. falciparum umfasst drei verschiedene Stadien. Im ersten Stadium führen Moskitos während der Nahrungsaufnahme Sporozoiten in die Blutbahn von Wirbeltieren ein. Diese Sporozoiten wandern über die Blutbahn in die Leber und dringen in die Hepatozyten des Wirtes ein. Im zweiten Stadium entwickeln sich aus diesen Sporozoiten Merozoiten. Diese Merozoiten durchlaufen mehrere Multiplikationszyklen in Erythrozyten des Wirtes und entwickeln sich dann zu Gametozyten. Die Gametozyten, die das sexuelle Stadium des Parasiten darstellen, werden von Moskitos bei der Nahrungsaufnahme aufgenommen. Nach der Befruchtung im Darm des Insektes entwickeln sich aus den Gametozyten Sporozoiten, die dann in die Speicheldrüsen des Insektes wandern. Von dort aus kann der Zyklus von neuem beginnen.

Sporozoiten, Merozoiten und Gametozyten weisen verschiedenen Antigene auf. Vakzine können prinzipiell gegen jedes der verschiedenen Stadien des Malariaparasiten hergestellt werden, doch ist bekannt, dass viele Polypeptide des Parasiten genetisch polymorph sind, d.h. dass sich die Polypeptide von Generation zu Generation leicht verändern. Dies erschwert die Immunisierung von Wirbeltieren gegen Malaria unter Verwendung dieser Polypeptide als Antigene, da die einmal gebildeten Antikörper mit der Zeit das sich verändernde Antigen nicht mehr erkennen können. Deshalb wäre ein ideales Vakzin gegen ein Polypeptid des Parasiten gerichtet, dessen Aminosäuresequenz nicht variabel ist, d.h. gegen ein Polypeptid das genetisch stabil ist. Es ist bekannt, dass die Aminosäuresequenz (= Primärstruktur) von Polypeptiden, die eine bestimmte Funktion ausüben, wie z.B. Enzyme, zumindest in denjenigen Bereichen der Primärstruktur, die für die Funktion wichtig sind, konstant ist.

Ein Beispiel für ein genetisch stabiles Polypeptid von P. falciparum ist das Merozoitenantigen mit der Aminosäuresequenz (I)

MetAsnAlaProLysLysLeuProAlaAspValAlaGluGluLeuAlaThrThrAla-W-LysLeuValGlnAlaGlyLysGlyIleLeuAlaAlaAspGluSerThrGlnThrIleLysLysArgPheAspAsnIleLysLeuGluAsnThrIleGluAsnArgAlaSerTyrArgAspLeuLeuPheGlyThrLysGlyLeuGlyLysPheIleSerGlyAlaIleLeuPheGluGluThrLeuPheGlnLysAsnGluAlaGlyValPro-X-ValAsnLeuLeuHisAsnGluAsnIleIleProGlyIleLysValAspLys-Y-LeuValAsnIleProCysThrAspGluGluLysSerThrGln-Z-LeuAspGlyLeuAlaGluArgCysLysGluTyrTyrLysAlaGlyAlaArgPheAlaLysTrpArgThrValLeuValIleAspThrAlaLysGlyLysProThrAspLeuSerAsnHisGluThrAlaTrpGlyLeuAlaArgTyrAlaSerIleCysGlnGlnAsnArgLeuValProIleValGluProGluIleLeuAlaAspGlyProHisSerIleGluValCysAlaValValThrGlnLysValLeuSerCysValPheLysAlaLeuGlnGluAsnGlyValLeuLeuGluGlyAlaLeuLeuLysProAsnMetValThrAlaGlyTyrGluCysThrAlaLysThrThrThrGlnAspValGlyPheLeuThrValArgThrLeuArgArgThrValProProAlaLeuProGlyValValPheLeuSerGlyGlyGlnSerGluGluAlaSerValAsnLeuAsnSerIleAsnAlaLeuGlyProHisProTrpAlaLeuThrPheSerTyrGlyArgAlaLeuGlnAlaSerValLeuAsnThrTrpGlnGlyLysLysGluAsnValAlaLysAlaArgGluValLeuLeuGlnArgAlaGluAlaAsnSerLeuAlaThrTyrGlyLysTyrLysGlyGlyAlaGlyGlyGluAsnAlaGlyAlaSerLeuTyrGluLysLysTyrValTyr

worin
-W- Gln ist oder fehlen kann;
-X- Met oder Gln ist;
-Y- Gly oder Cys ist und
-Z- Gly oder Cys ist.

Die vorliegende Erfindung betrifft Polypeptide, die in mindestens einem spezifischen Epitop mit dem Plasmodium flaciparum Merozoiten Antigen mit der Aminosäuresequenz (I) übereinstimmen. Ein spezifisches Epitop ist eine immunogene Determinante auf einem Polypeptid, die sich durch eine spezifische molekulare Konfiguration einer Teilsequenz des Polypeptids ergibt. Die Erfindung betrifft auch Polypeptide wie sie oben definiert sind und die zusätzlich noch kovalent mit einem Affinitätspeptid verknüpft sind.

Unter einem Affinitätspepid werden solche Peptidreste verstanden, die eine Aminosäuresequenz enthalten, die bevorzugt an ein Affinitätschromatographieträgermaterial bindet. Beispiele für solche Affinitätspeptidreste sind Peptidreste, die mindestens zwei Histidinreste enthalten. Solche Affinitätspeptidreste binden selektiv an Nitrilotriessigsäure-Nickelchelatharze (siehe z.B. europäische Patentanmeldung, Publ.-Nr. 253 303). Polypeptide, die einen solchen Affinitätspeptidrest enthalten, können mittels solcher Harze selektiv von den übrigen Polypeptiden abgetrennt werden. Das Affinitätspeptid kann entweder mit dem C-Terminus oder dem N-Terminus des oben definierten Polypeptids verknüpft sein, bevorzugt ist jedoch die Verknüpfung mit dem N-Terminus, insbesondere dann, wenn das natürliche Stop-Codon des Malaria Antigens bei der Expression des erfindungsgemässen Polypeptids mitverwendet wird.

Das bevorzugte Polypeptid gemäss der vorliegenden Erfindung kann durch die allgemeinen Formel

A-B

dargestellt werden, worin

A ein Affinitätspeptid ist oder fehlen kann.

B ein Polypeptid ist, das in mindestens einem spezifischen Epitop mit dem P. falciparum Merzoiten-Antigen mit der Aminosäuresequenz (I) übereinstimmt.

Die am meisten bevorzugten erfindungsmässen Polypeptide haben die Aminosäuresequenz

MetArgGlySerHisHisHisHisHisHisGlySerGlyAsnIleProCysThrAspGlu
GluLysSerThrGlnGlyLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu
AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr (II),

MetArgGlySerHisHisHisHisHisHisGlySerGlyAsnIleProCysThrAspGlu
GluLysSerThrGlnCysLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu
AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr (II'),

MetArgGlySerHisHisHisHisHisHisGlySerGluLeuAlaCysGlnTyrMetAsn
AlaProLysLysLeuProAlaAspValAlaGluGluLeuAlaThrThrAlaLysLeuVal
GlnAlaGlyLysGlyIleLeuAlaAlaAspGluSerThrGlnThrIleLysLysArgPhe
AspAsnIleLysLeuGluAsnThrIleGluAsnArgAlaSerTyrArgAspLeuLeuPhe

GlyThrLysGlyLeuGlyLysPheIleSerGlyAlaIleLeuPheGluGluThrLeuPhe
GlnLysAsnGluAlaGlyValProGlnValAsnLeuLeuHisAsnGluAsnIleIlePro
GlyIleLysValAspLysCysLeuValAsnIleProCysThrAspGluGluLysSerThr
GlnGlyLeuAspGlyLeuAlaGluArgCysLysGluTyrTyrLysAlaGlyAlaArgPhe
AlaLysTrpArgThrValLeuValIleAspThrAlaLysGlyLysProThrAspLeuSer
AsnHisGluThrAlaTrpGlyLeuAlaArgTyrAlaSerIleCysGlnGlnAsnArgLeu
ValProIleValGluProGluIleLeuAlaAspGlyProHisSerIleGluValCysAla
ValValThrGlnLysValLeuSerCysValPheLysAlaLeuGlnGluAsnGlyValLeu
LeuGluGlyAlaLeuLeuLysProAsnMetValThrAlaGlyTyrGluCysThrAlaLys
ThrThrThrGlnAspValGlyPheLeuThrValArgThrLeuArgArgThrValProPro
AlaLeuProGlyValValPheLeuSerGlyGlyGlnSerGluGluGluAlaSerValAsn
LeuAsnSerIleAsnAlaLeuGlyProHisProTrpAlaLeuThrPheSerTyrGlyArg
AlaLeuGlnAlaSerValLeuAsnThrTrpGlnGlyLysLysGluAsnValAlaLysAla
ArgGluValLeuLeuGlnArgAlaGluAlaAsnSerLeuAlaThrTyrGlyLysTyrLys
GlyGlyAlaGlyGlyGluAsnAlaGlyAlaSerLeuTyrGluLysLysTyrValTyr (III),

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProGlnValAsnLeuLeuHisAsnGluAsnIleIle
ProGlyIleLysValAspLysGlyLeuValAsnIleProCysThrAspGluGluLysSer
ThrGlnGlyLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr
(III'),

MetArgGlySerHisHisHisHisHisHisGlySerGluLeuAlaCysGlnTyrMetAsn AlaProLysLysLeuProAlaAspValAlaGluGluLeuAlaThrThrAlaGlnLysLeu ValGlnAlaGlyLysGlyIleLeuAlaAlaAspGluSerThrGlnThrIleLysLysArg PheAspAsnIleLysLeuGluAsnThrIleGluAsnArgAlaSerTyrArgAspLeuLeu PheGlyThrLysGlyLeuGlyLysPheIleSerGlyAlaIleLeuPheGluGluThrLeu PheGlnLysAsnGluAlaGlyValProMetValAsnLeuLeuHisAsnGluAsnIleIle ProGlyIleLysValAspLysGlyLeuValAsnIleProCysThrAspGluGluLysSer ThrGlnGlyLeuAspGlyLeuAlaGluArgCysLysGluTyrTyrLysAlaGlyAlaArg PheAlaLysTrpArgThrValLeuValIleAspThrAlaLysGlyLysProThrAspLeu SerAsnHisGluThrAlaTrpGlyLeuAlaArgTyrAlaSerIleCysGlnGlnAsnArg LeuValProIleValGluProGluIleLeuAlaAspGlyProHisSerIleGluValCys AlaValValThrGlnLysValLeuSerCysValPheLysAlaLeuGlnGluAsnGlyVal LeuLeuGluGlyAlaLeuLeuLysProAsnMetValThrAlaGlyTyrGluCysThrAla LysThrThrThrGlnAspValGlyPheLeuThrValArgThrLeuArgArgThrValPro ProAlaLeuProGlyValValPheLeuSerGlyGlyGlnSerGluGluGluAlaSerVal AsnLeuAsnSerIleAsnAlaLeuGlyProHisProTrpAlaLeuThrPheSerTyrGly ArgAlaLeuGlnAlaSerValLeuAsnThrTrpGlnGlyLysLysGluAsnValAlaLys AlaArgGluValLeuLeuGlnArgAlaGluAlaAsnSerLeuAlaThrTyrGlyLysTyr LysGlyGlyAlaGlyGlyGluAsnAlaGlyAlaSerLeuTyrGluLysLysTyrValTyr (III"),

MetArgGlySerHisHisHisHisHisHisGlySerGluLeuAlaCysGlnTyrMetAsn AlaProLysLysLeuProAlaAspValAlaGluGluLeuAlaThrThrAlaGlnLysLeu ValGlnAlaGlyLysGlyIleLeuAlaAlaAspGluSerThrGlnThrIleLysLysArg PheAspAsnIleLysLeuGluAsnThrIleGluAsnArgAlaSerTyrArgAspLeuLeu PheGlyThrLysGlyLeuGlyLysPheIleSerGlyAlaIleLeuPheGluGluThrLeu PheGlnLysAsnGluAlaGlyValProGlnValAsnLeuLeuHisAsnGluAsnIleIle ProGlyIleLysValAspLysGlyLeuValAsnIleProCysThrAspGluGluLysSer ThrGlnCysLeuAspGlyLeuAlaGluArgCysLysGluTyrTyrLysAlaGlyAlaArg PheAlaLysTrpArgThrValLeuValIleAspThrAlaLysGlyLysProThrAspLeu SerAsnHisGluThrAlaTrpGlyLeuAlaArgTyrAlaSerIleCysGlnGlnAsnArg LeuValProIleValGluProGluIleLeuAlaAspGlyProHisSerIleGluValCys AlaValValThrGlnLysValLeuSerCysValPheLysAlaLeuGlnGluAsnGlyVal LeuLeuGluGlyAlaLeuLeuLysProAsnMetValThrAlaGlyTyrGluCysThrAla LysThrThrThrGlnAspValGlyPheLeuThrValArgThrLeuArgArgThrValPro ProAlaLeuProGlyValValPheLeuSerGlyGlyGlnSerGluGluGluAlaSerVal AsnLeuAsnSerIleAsnAlaLeuGlyProHisProTrpAlaLeuThrPheSerTyrGly

ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr
(III"'),

oder

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProMetValAsnLeuLeuHisAsnGluAsnIleIle
ProGlyIleLysValAspLysGlyLeuValAsnIleProCysThrAspGluGluLysSer
ThrGlnCysLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr
(III"").

Die Erfindung betrifft auch Polypeptide der allgemeinen Formel A-B mit einer Aminosäuresequenz, die durch Additionen, Deletionen, Insertionen oder Aminosäuresubstitutionen aus den oben gezeigten Aminosäuresequenzen hervorgegeangen sind, vorausgesetzt, dass diese Polypeptide noch fähig sind, eine Immunantwort gegen das Merozoitenstadium der Malariaparasiten auszulösen, vorzugsweise gegen das Merozoiten-Antigen mit der Aminosäuresequenz (I) von P. falciparum. Die Erfindung betrifft ferner DNA Sequenzen, die für ein erfindungsgemässes Polypeptid kodieren, sowie replizierbare mikrobielle Vektoren, die solche DNA Sequenzen enthalten, insbesondere Expressionsvektoren, d.h. replizierbare mikrobielle Vektoren, bei denen eine DNA Sequenz, die für ein erfindungsgemässes Polypeptid kodiert, so an eine Expressionskontrollsequenz gebunden ist, dass das von der DNA Sequenz kodierte Polypeptid exprimiert werden kann. Ausserdem betrifft die vorliegende Erfindung Mikroorganismen, die einen solchen replizierbaren Vektor bzw. einen Expressionsvektor enthalten sowie Verfahren zu deren Herstellung. Im weiteren betrifft die vorliegende Erfindung Verfahren zur Herstellung der Polypeptide sowie deren Verwendung zur Immunisierung von Säugern gegen Malaria.

Da gewisse Substitutionen in der Aminosäuresequenz eines Polypeptides keinen Einfluss auf die räumliche Struktur bzw. die biologische Aktivität des Polypeptides haben, kann die Aminosäuresequenz der erfindungsgemässen Polypeptide von den oben gezeigten Aminosäuresequenzen abweichen. Beispiele solcher Aminosäuresubstitutionen sind Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu und vice versa (vgl. Doolittle, in "The Proteins", ed. Neurath, H, und Hill, R.L., Academic Press, New York [1979]).

Die erfindungsgemässen Polypeptide können kovalent an ein Trägermaterial gebunden oder daran adsorbiert sein. Geeignete Trägermaterialien sind natürliche oder snythetische Polymerverbindungen, wie z.B. Copolymere einer oder mehrerer Aminosäuren (z.B. Polylysin) oder Zucker (z.B. Polysaccharid). Weitere geeignete Trägermaterialien sind natürliche Polypeptide wie Hämocyanine (z.B. KLH = "keyhole limpet hemocyanin"), Serumproteine (z.B. Gammaglobulin, Serumalbumin) und Toxoide (z.B. Diphtherie- oder Tetanustoxoid). Weitere geeignete Trägermaterialien sind dem Fachmann bekannt.

Die kovalente Bindung der erfindungsgemässen Polypeptide an die Trägermaterialien kann auf bekannte Art und Weise erfolgen, z.B. direkt durch die Bildung einer Peptid- oder Esterbindung zwischen freien Carboxyl-, Amino- oder Hydroxylgruppen der Polypeptide und den entsprechenden Gruppen auf dem Trägermaterial, oder indirekt durch Verwendung von konventionellen, bifunktionellen Reagenzien wie z.B. m-Maleimidobenzoyl-N-hydroxysuccinimidester (MBS) oder Succinimidyl 4-(p-maleimidophenyl)butyrat (SMPB). Diese und weitere bifunktionellen Reagenzien sind käuflich erhältlich, z.B. von Pierce Chemical Company, Rockford, Illinois, U.S.A. Ferner können $C_{2-7}$-Dialkanale, wie z.B. Glutaraldehyd (Avrameas, Immunochem. 6, 43-52 [1969]), verwendet werden.

Das Trägermaterial, mit den daran gebundenen Polypeptiden, kann mittels bekannter Methoden (z.B. Dialyse oder Säulenchromatographie) von nichtgebundenen Polypeptiden und gegebenenfalls von den überschüssigen Reagentien abgetrennt werden.

Die Peptide der vorliegenden Erfindung können mittels konventioneller Methoden der Peptidsynthese, in flüssiger oder, vorzugsweise an fester Phase, wie der Methode von Merrifield (J. Am. Chem. Soc. 85, 2149-2154 [1963]), oder mittels anderer gleichwertiger Methoden des Standes der Technik, hergestellt werden.

Die Festphasensynthese beginnt mit der C-terminalen Aminosäure des zu synthetisierenden Peptids, die in geschützter Form an ein passendes Trägermaterial gekoppelt wird. Das Ausgangsmaterial kann durch Bindung einer aminogruppengeschützten Aminosäure über eine Benzylesterbrücke an chlormethyliertes oder ein hydroxymethyliertes Trägermaterial oder durch Amidbindung an ein Benzylhydrylamin (BHA)-, ein Methylbenzylhydrylamin (MBHA)- oder ein Benzyloxybenzylalkohol-Trägermaterial hergestellt werden. Diese Trägermaterialien sind käuflich erhältlich und ihre Bereitstellung und Verwendung sind wohlbekannt.

Allgemeine Methoden zum Schützen und Entfernen der Schutzgruppen von Aminosäuren, die in dieser Erfindung verwendet werden können, sind beschrieben in "The Peptides", Vol. 2 (herausgegeben von E. Gross und J. Meienhofer, Academic Press, New York, 1-284 [1979]). Schutzgruppen umfassen z.B. die 9-Fluorenylmethyloxycarbonyl (Fmoc), die tertiäre Butyloxycarbonyl (Boc)-, die Benzyl (Bzl)-, die t-Butyl (But)-, die 2-Chlorbenzyloxycarbonyl (2Cl-Z)-, die Dichlorbenzyl (Dcb)- und die 3,4-Dimethylbenzyl- (Dmb)-gruppe.

Nach Entfernen der α-Aminoschutzgruppe der an den Träger gebundenen C-terminalen Aminosäuren werden die geschützten Aminosäuren in der gewünschten Reihenfolge schrittweise angekoppelt. Das vollständige Peptid kann so synthetisiert werden. Als Alternative dazu können kleine Peptide aufgebaut werden, die dann zum gewünschten Peptid zusammengefügt werden. Geeignete Kopplungsreagenzien gehören zum Stand der Technik, wobei Dicyclohexylcarbodiimid (DCC) besonders geeignet ist.

Jede(s) geschützte Aminosäure oder Peptid wird im Ueberschuss in das Festphasensynthesereaktionsgefäss gegeben und die Kopplungsreaktion kann in Dimethylformamid (DMF) oder Methylenchlorid ($CH_2Cl_2$) oder einer Mischung aus beiden durchgeführt werden. In Fällen von unvollständiger Kopplung wird die Kopplungsreaktion wiederholt bevor die N-terminale α-Aminoschutzgruppe zwecks Kopplung der nächsten Aminosäure entfernt wird. Die Ausbeute jedes Kopplungsschrittes kann verfolgt werden und zwar bevorzugt nach der Ninhydrinmethode. Die Kopplungsreaktionen und die Waschschritte können automatisiert durchgeführt werden.

Die Abspaltung des Peptids vom Trägermaterial kann durch in der Peptidchemie wohlbekannte Methoden erreicht werden, z.B. durch Umsetzung mit Fluorwasserstoff (HF) in Gegenwart von p-Kresol und Dimethylsulfid während 1 Stunde bei 0 °C, gefolgt möglicherweise von einer zweiten Umsetzung mit HF in Gegenwart von p-Kresol während 2 Stunden bei 0 °C. die Abspaltung der Peptide von chlormethylierten oder hydroxymethylierten Trägermaterialien ergibt Peptide mit freiem C-Terminus; die Abspaltung der Peptide von Benzylhydrylamin- oder Methylbenzylhydrylamin-Trägern ergibt Peptide mit amidiertem C-Terminus.

Andererseits können die Polypeptide der vorliegenden Erfindung auch unter Verwendung der Methoden der DNA-Rekombinationstechnik (Manniatis et al. in "Molecular Cloning - A Laboratory Manual", Cold Spring Harbor Laboratory [1982]) hergestellt werden. z.B. kann ein DNA-Fragment, das für ein solches Polypeptid codiert, mittels konventioneller chemischer Methoden synthetisiert werden, z.B. mittels der Phosphotriestermethode wie sie von Narang et al. in Meth. Enzymol. 68, 90-108 [1979] beschrieben ist oder mittels der Phosphodiestermethode (Brown et al., Meth. Enzymol. 68, 109-151 [1979]). Bei beiden Methoden werden zuerst längere Oligonukleotide synthetisiert, die in vorgegebener Art und Weise aneinander gehängt werden. Die Nukleotidsequenz des DNA-Fragmentes kann identisch sein zu derjenigen Nukleotidsequenz, die das natürliche Polypeptid im Plasmodium Parasiten codiert. Da der genetische Code degeneriert ist, besteht andererseits die Möglichkeit, dass eine teilweise oder vollständig unterschiedliche Nukleotidseqeunz das gleiche Polypeptid codiert. Gegebenenfalls können für die Nukleotidsequenz solche Codons gewählt werden, die auch vom Wirtsorganismus, das zur Expression des Polypeptids verwendet wird, bevorzugt verwendet werden (Grosjean et al., Gene 18, 199-209 [1982]). Dabei muss aber darauf geachtet werden, dass das so erhaltene DNA-Fragment keine Teilsequenzen enthält, die die Konstruktion des Expressionsvektors, z.B. durch Einführung einer unerwünschten Restriktionsenzymschnittstelle, erschweren oder die Expression des Polypeptids behindern.

Im weiteren können die Polypeptide der allgemeinen Formel A-B auch hergestellt werden, indem man ein DNA-Fragment, das für die Teilsequenz B kodiert, aus dem Genom eines Plasmodiumisolates isoliert und in einem Wirtsorganismus exprimiert. Das DNA-Fragment das für die Teilsequenz B kodiert kann erhalten werden, indem genomische DNA eines Plasmodiumstammes mit einem oder mehreren geeigneten Restriktionsendonukleasen, z.B. EcoR1, gespalten wird. Fragmente mit einer Länge von 1,5 bis 8 x $10^3$ Basenpaaren werden isoliert und in einen geeigneten Vektor, z.B. in den λ-Phagenvektor gt11 (Young et al., Proc. Natl. Acad. Sci. USA 80, 1194-1198 [1983]) erhältlich von der American Type Culture Collection, 12301 Parklawn Drive, Rochville, Maryland, USA (ATCC-Nr. 37194) eingebaut. Die rekombinierte Phagen-DNA kann in vitro in Phagen gepackt werden. Die so erhaltenen Phagen werden in geeignete Wirtszellen, z.B. in E. coli Y1088 enthaltend das Plasmid pMC9 (ATCC-Nr. 37195) eingeführt. Aus etwa 100'000 rekombinierten Phagen werden diejenigen Phagen ausgewählt, die mit einer geeigneten Probe hybridisieren. Solche geeigneten Proben sind Oligonukleotide, die einer Teilsequenz der genomischen DNA entsprechen, die für ein erfindungsgemässes Polypeptid kodiert. Die Art und Weise wie diese Proben ausgewählt und verwendet werden ist dem Fachmann bekannt. Phagen, die das gewünschte DNA-Fragment enthalten, werden vermehrt und die DNA isoliert. Das DNA-Fragment kann anschliessend in einen geeigneten replizierbaren mikrobiellen Vektor eingebaut werden, vorzugsweise in einen Expressionsvektor, der die notwendigen Expressionssignale liefert und der für die Teilsequenzen A der allgemeinen Formel A-B erfindungsgemässen Polypeptide kodiert. Ein bevorzugter Expressionsvektor ist der Vektor pDS78/RBSII, 6xHis. Der Aufbau und die Herstellung dieses Vektors ist in den Beispielen ausführlich beschrieben. Die Polypeptide der vorliegenden Erfindung können, nach entsprechender Anpassung der Nukleotidsequenz, auch in anderen geeigneten Expressionsvektoren hergestellt werden. Beispiele solcher Expressionsvektoren sind in der Europäischen Patenanmeldung, Publikations-Nr. 186 069, die am 2. Juli 1986 veröffentlicht worden ist, beschrieben. Weitere Expressionsvektoren sind dem Fachmann bekannt.

Der Expressionsvektor, der ein DNA-Fragment mit der DNA Sequenz, die für erfindungsgemässes Polypeptid kodiert, enthält, wird anschliessend in einem geeigneten Wirtorganismus eingeführt. Geeignete Wirtsorganismen sind Mikroorganismen, z.B. Hefezellen oder Bakterienzellen die fähig sind, das von Expressionsvektoren kodierte Polypeptid zu exprimieren. Bevorzugter Wirtsorganismus ist E.coli M15 (von Villarejo et al. in J. Bacteriol. 120, 466-474 [1974] als DZ291 beschrieben). Weitere geeignete Wirtsorganismen sind E.coli 294 (ATCC-Nr. 31446), E.coli RR1 (ATCC-Nr. 31343) und E.coli W3110 (ATCC-Nr. 27325).

Die Art und Weise wie die Expression der erfindungsgemässen Polypeptide erfolgt, ist abhängig vom verwendeten Expressionsvektor und vom Wirtsorganismus. Ueblicherweise werden die Wirksorganismen, die den Expressionsvektor enthalten, unter Bedingungen, die für das Wachstum der Wirtsorganismen optimal sind, vermehrt. Gegen Ende des exponentiellen Wachstums, wenn die Zunahme der Zellzahl pro Zeiteinheit abnimmt, wird die Expression des Polypeptids der vorliegenden Erfindung induziert, d.h. die das Polypeptid kodierende DNA transkribiert und die transkribrierte mRNA translatiert. Die Induktion kann durch Zugabe eines Induktors oder eines Derepressors zum Wachstumsmedium oder durch Veränderung eines physikalischen Parameters z.B. einer Temperaturänderung erfolgen. In dem in der vorliegenden Erfindung verwendeten Expressionsvektor, wird die Expression durch den lac-Repressor kontrolliert. Durch Zugabe von Isopropyl-β-D-thiogalactopyranosid (IPTG) wird die Expressionskontrollsequenz dereprimiert und dadurch die Synthese des Polypeptids induziert.

Das in den Wirtsorganismen produzierte Polypeptid kann durch spezielle Transportmechanismen aus der Zelle sekretiert, oder durch Aufbrechen der Zelle isoliert werden. Das Aufbrechen der Zelle kann durch mechanische (Charm et al., Meth. Enzymol. 22, 476-556 [1971]). (Lysozymbehandlung) oder chemische (Detergenzbehandlung, Harnstoff-oder Guanidin•HCl-Behandlung, etc.) Einwirkungen erfolgen, oder durch eine Kombination dieser Einwirkungen.

In Eukaryonten werden Polypeptide, die aus der Zelle ausgeschieden werden, in Form eines Vorläufermoleküls synthetisiert. Durch Abspaltung des sog. Signalpeptids entsteht das reife Polypeptid. Da prokaryotische Wirtsorganismen nicht fähig sind eukaryotische Signalpeptide von Vorläufermolekülen abzuspalten, müssen eukaryotische Polypeptide in prokaryotischen Wirtsorganismen direkt in ihrer reifen Form exprimiert werden. Das Translationsstartsignal AUG, dem auf dem Niveau der DNA das Codon ATG entspricht, bewirkt, dass alle Polypeptide in einem prokaryotischen Wirtsorganismus mit einem Methioninrest am N-Terminus synthetisiert werden. In gewissen Expressionssystemen wird dieser N-terminale Methioninrest abgespalten. Es hat sich aber gezeigt, dass die An- oder Abwesenheit des N-terminalen Methionins, die biologische Aktivität eines Polypeptids kaum beeinflusst (vergl. Winnacker, in "Gene und Klone", S. 255. Verlag Chemie, Weinheim, BRD [1985]). In Fällen in denen das N-terminale Methionin stört, kann es auch mittels einer für das N-terminale Methionin spezifischen Peptidase abgespalten werden. Miller et al. (Proc. Natl. Acad. Sci. U.S.A. 84, 2718-2722 [1987]) haben die Isolation einer solchen Peptidase aus Salmonella typhimurium beschrieben. Die vorliegende Erfindung betrifft deshalb Polypeptide mit oder ohne N-terminalen Methioninrest.

Die erfindungsgemässen Polypeptide können mittels bekannter Methoden gereinigt werden, wie zum Beispiel mittels Zentrifugation bei unterschiedlichen Geschwindig keiten, mittels Präzipitation mit Ammoniumsulfat, mittels Dialyse (bei Normaldruck oder bei reduziertem Druck), mittels präparativer Isoelektrofokussierung, mittels präparativer Gelelektrophorese oder mittels verschiedener chromatographischer Methoden wie Gelfiltration, Hochleistungs-Flüssigchromatographie (HPLC), Ionenaustauschchromatographie, Umkehrphasenchromatographie und Affinitätschromatographie (z.B. an Sepharose Blau CL-6B, an Phosphocellulose, an trägergebundenen, gegen das Polypeptid gerichteten monoklonalen Antikörpern oder an Metallchelatharzen wie sie in der vorliegenden Erfindung beschrieben sind).

Die bevorzugte Reinigungsmethode in der vorliegenden Erfindung ist die affinitätschromtographische Reinigung. Besonders bevorzugt ist die Reinigung der erfindungsgemässen Polypeptide an Metallchelatharzen (Sulkowsky, Trends in Biotechn. 3, 1-7 [1985]) oder an Phosphocellulose. Dabei werden die selektive Bindung von benachbarten Histidinresten an Nitrilotriessigsäure-Nickelchelaltharze (NTA-Harze) bzw. die Affinität von Aldolasen an Phosphocellulose ausgenützt. Die beiden Reinigungsmethoden können auch kombiniert werden.

Die Polypeptide der vorliegenden Erfindung können in Form von Multimeren, z.B. in Form von Dimeren, Trimeren, Tetrameren vorliegen, oder können auch Teil sein von Fusionspolypeptiden. Multimere können entstehen wenn Polypeptide in prokaryotischen Wirtsorganismen hergestellt werden, zum Beispiel durch die Bildung von Disulfidbrücken zwischen Cysteinresten. Fusionspolypeptide können hergestellt werden, indem DNA-Fragmente, die für ein Polypeptid der vorliegenden Erfindung kodieren, verknüpft werden mit einem oder mehreren DNA-Fragmenten, die für ein weiteres, beliebiges Polypeptid kodieren. Beispiele für ein solches Fusionspolypeptid sind Polypeptide der allgemeinen Formel A-B wie sie oben definiert sind. Weitere Beispiele sind Polypeptide der allgemeinen Formel B-C bzw. A-B-C worin C ein beliebiges Polypeptid ist und A und B die oben ange gebenen Bedeutungen haben. Beispiele für ein Polypeptid der allgemeinen Formel A-B-C sind Fusionspolypeptide zwischen dem Teil B in der allgemeinen Formel A-B und β-Galaktosidase, wie sie gemäss Rüther et al., EMBO J., 2, 1791-1794 [1983] hergestellt werden können. Weder das Affinitätspeptid A noch das Polypeptid C dürfen die Funktion der erfindungsgemässen Polypeptide als Antigene bzw. als Vakzine gegen Malarie beeinträchtigen.

Die vorliegende Erfindung betrifft auch immunogene Kompositionen, die ein Polypeptid gemäss der vorliegenden Erfindung, sowie ein geeignetes Adjuvants, enthalten. Geeignete Adjuvantien für die Anwendung an Mensch und Tier sind dem Fachmann bekannt (WHO Techn. Rep. Series 595, 1-40 [1976]; Jollis et al., "Chemical and Biological Basis of Adjuvants", in Molecular Biochemistry and Biophysics Vol. 13, 1-148 [1973], Springer Verlag Berlin).

Die erfindungsgemässen Polypeptide können als Lyophilisate zur Rekonstitution mit sterilem Wasser oder einer Salzlösung, vorzugsweise einer Kochsalzlösung, vorliegen. Durch Einführung der erfindungsgemässen Polypeptide und immunogenen Kompositionen in Säuger wird deren Immunsystem aktiviert, das heisst Antikörper gegen das Polypeptid werden gebildet. Die vorliegende Erfindung betrifft auch solche Antikörper. Die erfindungsgemässen Antikörper erkennen das natürliche Aequivalent des Polypeptids des Malariaparasiten und können deshalb für die passive Immunisierung oder für diagnostische Zwecke verwendet werden.

Antikörper gegen die erfindungsgemässen Polypeptide können in Affen, Kaninchen, Pferden, Ziegen, Meerschweinchen, Ratten, Mäusen, Kühen, Schafen etc., aber auch in Menschen hergestellt werden. Je nach Bedürfnis können das Antiserum oder die gereinigten Antikörper verwendet werden. Die Antikörper können auf bekannte Art und Weise, z.B. durch Fällung mit Ammoniumsulfat, gereinigt werden. Es ist auch möglich, monoklonale Antikörper, die gegen das Polypeptid der vorliegenden Erfindung gerichtet sind, gemäss der von Köhler et al. (Nature, 256, 495-497 [1975] entwickelten Methode, herzustellen. Polyklonale oder monoklonale Antikörper können auch zur affinitäts-chromatographischen Reinigung der Polypeptide der vorliegenden Erfindung oder deren natürlichen Aequivalenten verwendet werden.

Die erfindungsgemässen Polypeptide und die immunogenen Kompositionen können zur Immunisierung von Säugern gegen Malarie verwendet werden. Die Art der Verabreichung, die Dosierung, sowie die Anzahl der Injektionen können vom Fachmann auf bekannte Art und Weise optimiert werden. Typischerweise werden über einen längeren Zeitraum mehrere Injektionen verabreicht, um einen hohen Titer an Antikörpern gegen das Malariaantigen, d.h. gegen das Polypeptid der vorliegenden Erfindung, zu erhalten.

Die folgenden Figuren und das nachfolgende ausführliche Beispiel tragen zum besseren Verständnis der vorliegenden Erfindung bei. Es soll aber nicht der Eindruck erweckt werden, dass die Erfindung auf den Gegenstand des Beispiels oder der Figuren beschränkt ist.

In den Figuren treten die folgenden Abkürzungen und Symbole auf:

B, Bg, E, H, Sa, X und Xb bezeichnen Schnittstellen für die Restriktionsenzyme BamHI, BglII, EcorRI, HindIII, SalI, XhoI bzw. XbaI.

repräsentiert die Promotoren der Gene bla, lacI und neo; repräsentiert die ribosomalen Bindungsstellen der Gene bla, cat, neo und lacI; repräsentiert die Terminatoren $t_0$ und T1; repräsentiert das regulierbare Promotor/Operator Element N25OPSN25OP29; repräsentiert die ribosomale Bindungsstelle RBSII;

repräsentiert die kodierende Region unter Kontrolle dieser ribosomalen Bindungsstelle; repräsentiert eine Region, die für die sechs Histidine kodiert; repräsentiert die für die Replikation benötigte Region (repl.); repräsentiert kodierende Regionen für Dihydrofolatreduktase (dhfr), Chloramphenicolacetyltransferase (cat), lac-Repressor (lacI), ß-Lactamase (bla) und Neomycinphosphotransferase (neo).

Fig. 1 Schematische Darstellung des Plasmids pDS78/RBSII.

Fig. 2 Nukleotidsequenz des Plasmid pDS78/RBSII. In der Sequenz sind die Erkennungsstellen für die in Fig. 1 aufgeführten Restriktionsenzyme überstrichen, während die für $\beta$-Lactamase bzw. Dihydrofolatreduktase kodierenden Regionen unterstrichen sind.

Fig. 3 Schematische Darstellung des Plasmids pDMI,1

Fig. 4 Nukleotidsequenz des Plasmids pDMI,1. In der Sequenz sind die Erkennungsstellen für die in Fig. 3 aufgeführten Restriktionsenzyme überstrichen, während die für Neomycinphosphotransferase bzw. lac-Repressor kodierenden Regionen unterstrichen sind.

Fig. 5 Schematische Darstellung der Herstellung des XhoI/BamHI-Fragments mit dem regulierbaren Promotor/Operator-Element N25OPSN25OP29, der ribosomalen Bindungsstelle RBSII sowie der für sechs Histidine kodierenden Region.

Fig. 6 Schematische Darstellung der Konstruktion des Plasmids pDS78/RBSII, 6xHis unter Verwendung des Plasmids pDS78/RBSII und des XhoI/BamHI-Fragments F mit dem regulierbaren Promotor/Operator-Element N25OPSN25OP29, der ribosomalen Bindungsstelle RBSII sowie der für sechs Histidine kodierenden Region.

Fig. 7 Southern-Transfer-Analyse (Southern, J.Mol. Biol. 98, 503-517 [1975]) von genomischer DNA aus 12 verschiedenen Stämmen von P. falciparum. Alle 12 Isolate haben die typischen DraI-Fragmente A, B, C und D. Als Probe diente das P. falciparum DNA Fragment aus K1-B (siehe Beispiel).

Fig. 8 Western-Transfer-Analyse (Western-Blot, Towbin et al., Proc. Natl. Acad. Sci. USA, 76, 4350-4354 [1979]) von P. falciparum Proteinen aus 11 Isolaten mit Antikörpern gegen das Merozoitenantigen von P. falciparum. Alle 11 Isolate haben die charakteristische Bande, die einem Polypeptid mit einem Molekulargewicht von etwa 41'000 entspricht.

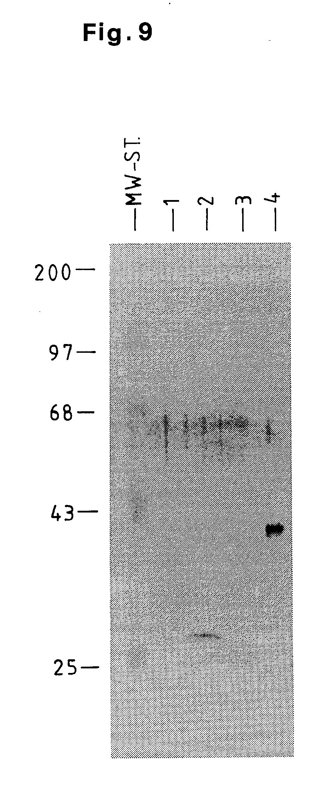

Fig. 9 Expression des rekombinaten Proteins (27 kD) in E.coli
E.coli Zell-Lysate wurden im Western-Blot mit Antikörpern gegen das Parasitenantigen getestet. Die mit MW-ST. bezeichnete Spur enthält vorgefärbte Molekulargewichtsstandards. Die Grösse ist in Kilodalton (= 1'000 Dalton) angegeben. Spur 1 enthält ein nicht-induziertes Lysat der transformierten Zellen. Spur 2 enthält eine induzierte Probe. Spur 3 enthält nicht transformierte Zellen als Kontrolle. Spur 4 enthält ein P.falciparunm K1 Lysat. Wie erwartet reagieren die Antikörper nur mit dem rekombinanten Protein (27 kD) in Spur 2 und mit dem 41'000 Dalton Parasitenprotein in Spur 4.

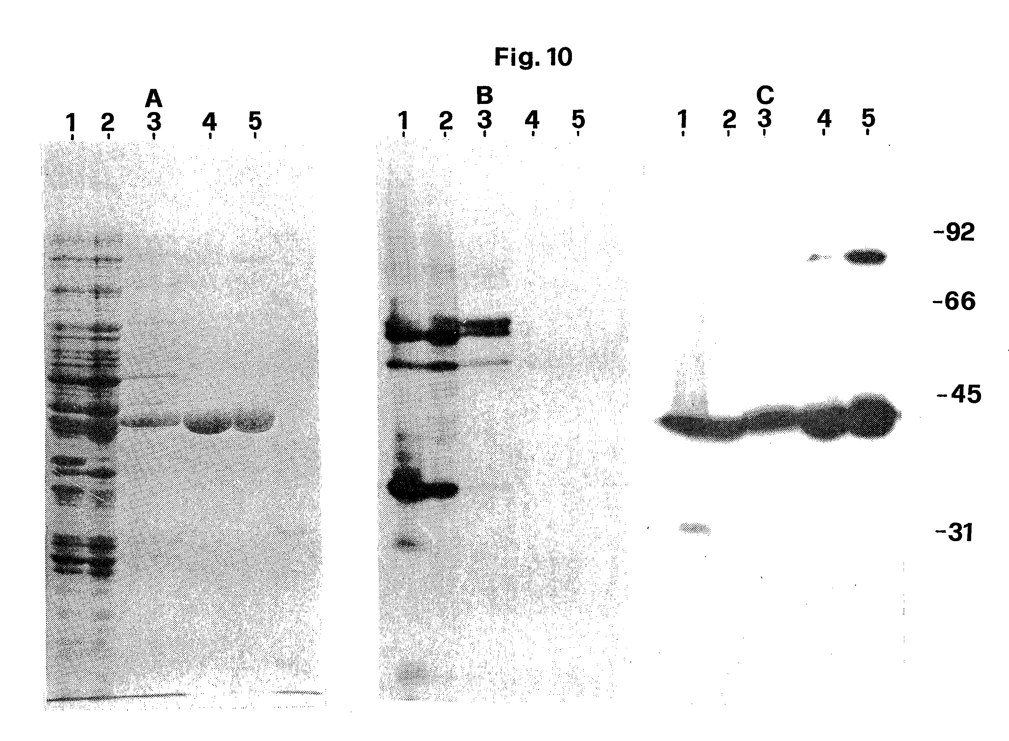

Fig. 10 Reinigung des rekombinanten Proteins (41 kD)
Analytische Polyacrylamidgelelektrophorese und Western-Blotanalyse der verschiedenen Reinigungsschritte der Reinigung des rekombinanten Proteins (41 kD).

(A) Coomassie-Blau gefärbtes Polyacrylamidgel. Spur 1: Zell-Lysat von E. coli-Zellen die mit p8/3 transformiert sind. Spur 2: Lösliche Fraktion des Zell-Lysates nach der Zentrifugation (100'000 x g). Spur 3: Eluat des spezifisch an die Phosphocellulosesäule gebundenen Materials. Spur 4: Eluat des spezifisch an das NTA-Harz gebundenen Materials. Spur 5: Endprodukt nach Ultrafiltration auf einer Sephacryl S-200 Säule. Folgende Molekulargewichtsmarkerproteine wurden verwendet:
31 = Carboanhydrase Molekulargewicht (MW) = 31'000 Dalton, 45 = Ovalbumin MW = 45'000 Dalton, 66 = Rinderserumalbumin MW = 66'000 Dalton, 92 = Phosphorylase B MW = 92'000 Dalton.

(B) Western-Blot des Polyacrylamidgels (A) mit Kaninchen-Antiserum gegen ein E. coli-Lysat.

(C) Western-Blot des Polyacrylamidgels (A) mit Antikörpern gegen Merozoitenantigene von P. falciparum (Perrin et al., J. Clin. Invest. 75, 1718-1721 [1985]).

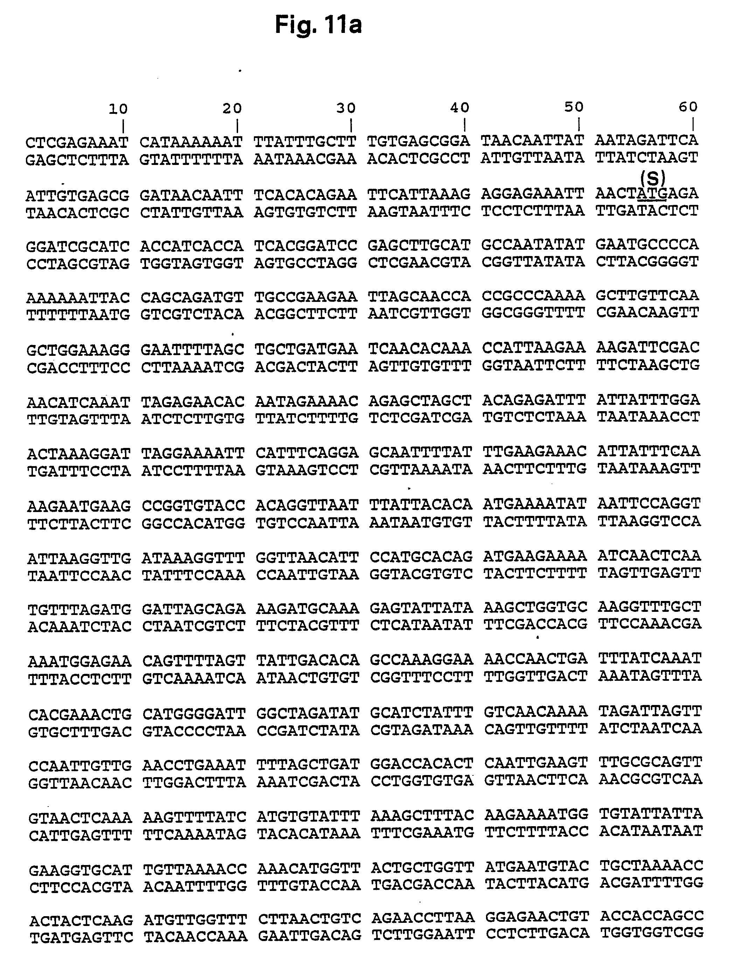

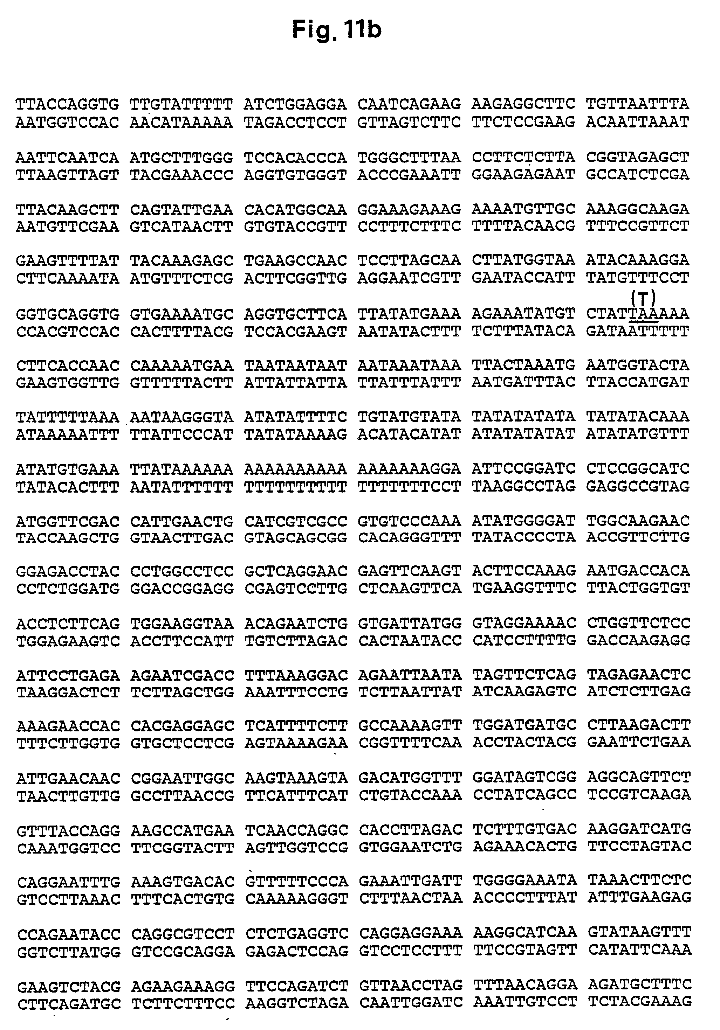

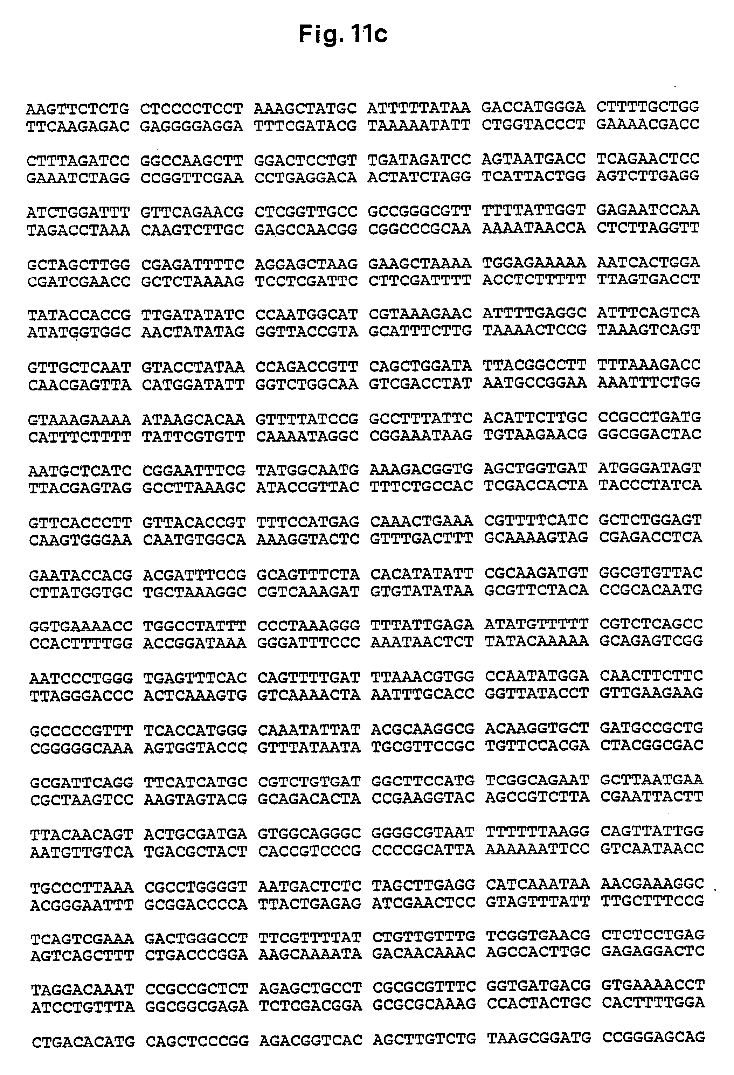

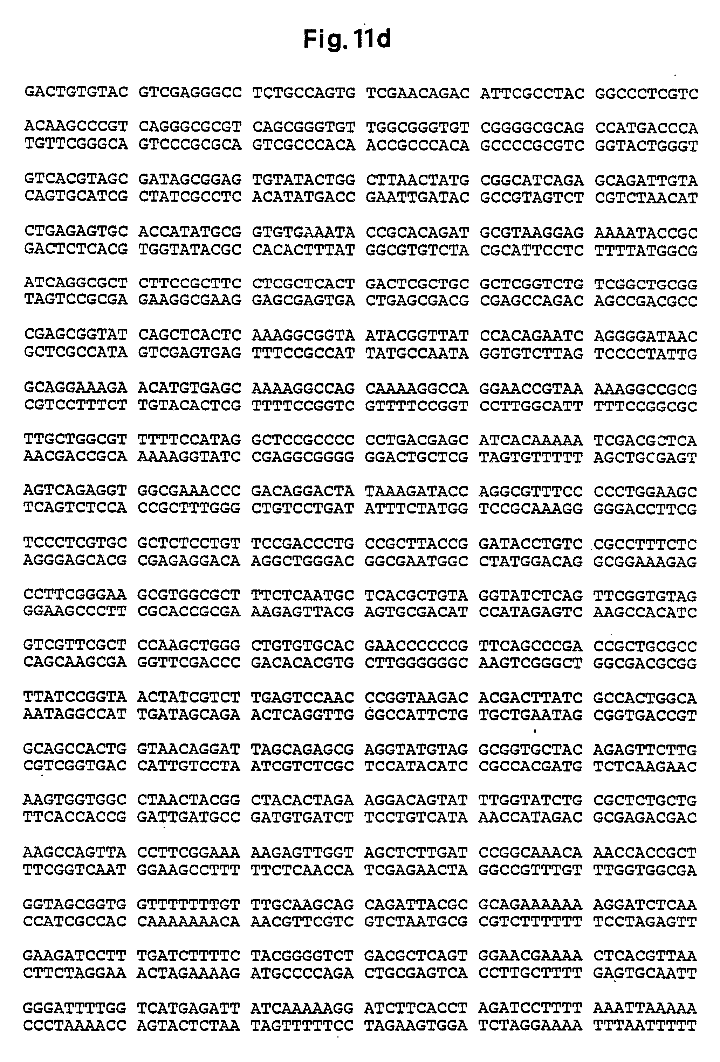

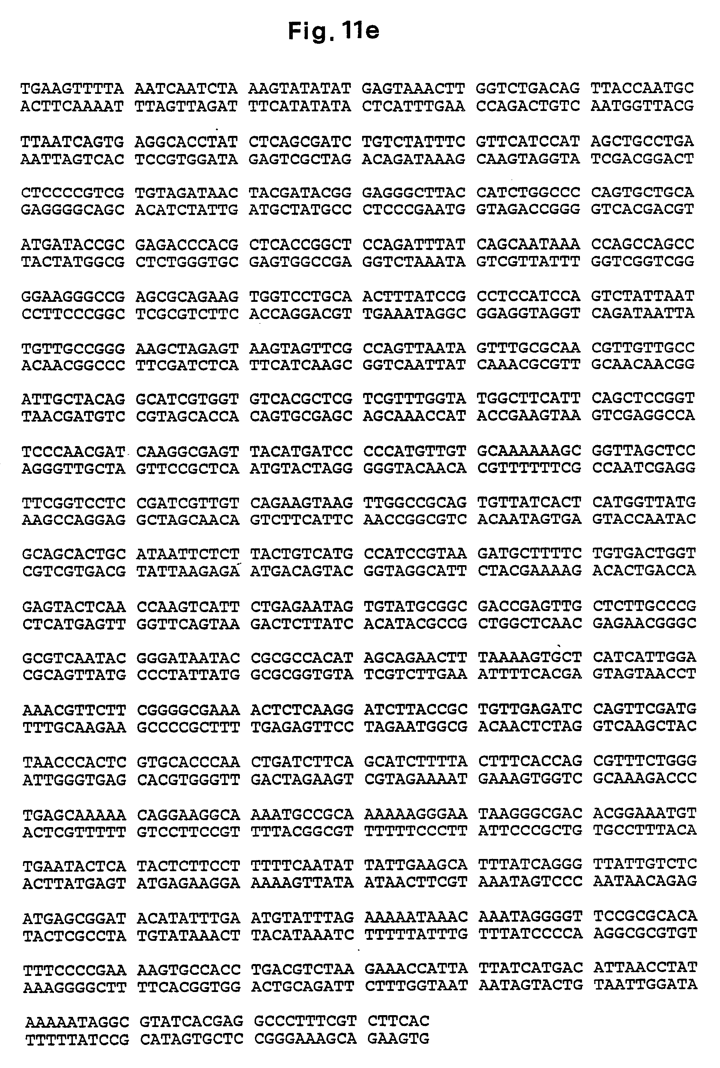

Fig. 11 Nukleotidsequenz des Plasmids p8/3
Die für das Polypeptid (41 kD) kodierende Sequenz beginnt mit dem ATG an Position 115-117 (S) und endet mit dem Stopcodon an Position 1255-1257 (T). Die für das Affinitätspeptid kodierende Sequenz beginnt mit dem erwähnten ATG und endet mit dem Tyrosin (Tyr) kodierenden Codon TAT an Position 166-168, während die für die Teilsequenz B des Polypeptids (41 kD) kodierende Sequenz mit dem für Methionin (Met) kodierenden Codon ATG an Position 169-171 beginnt und ebenfalls mit dem Stopcodon an Position 1255-1257 endet.

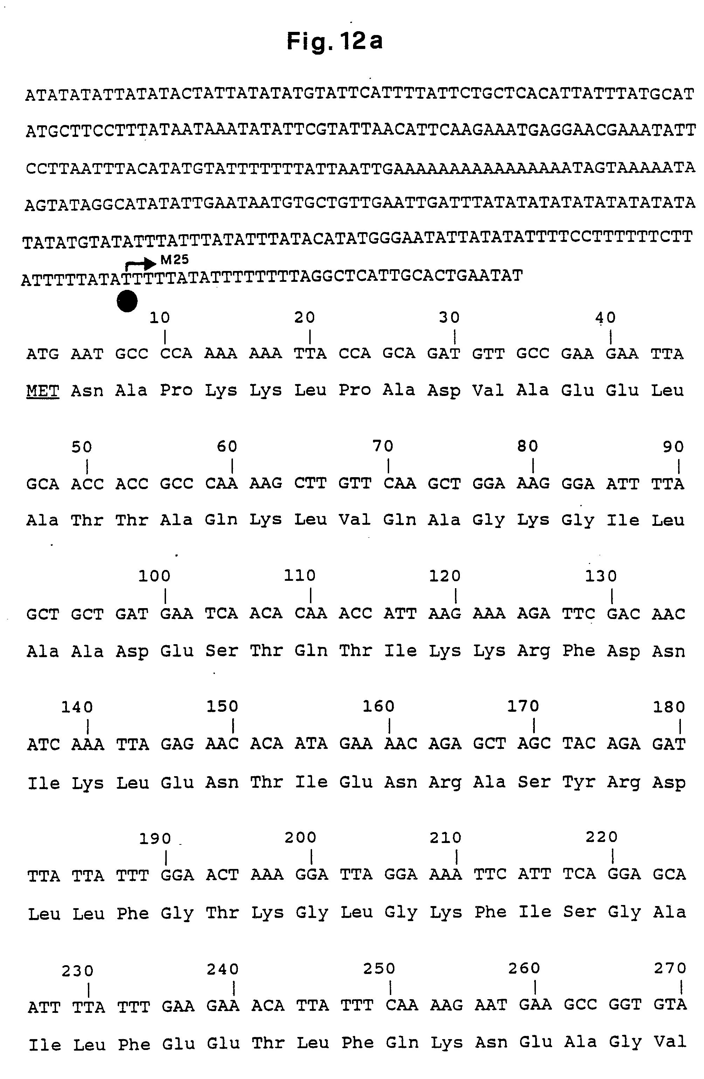

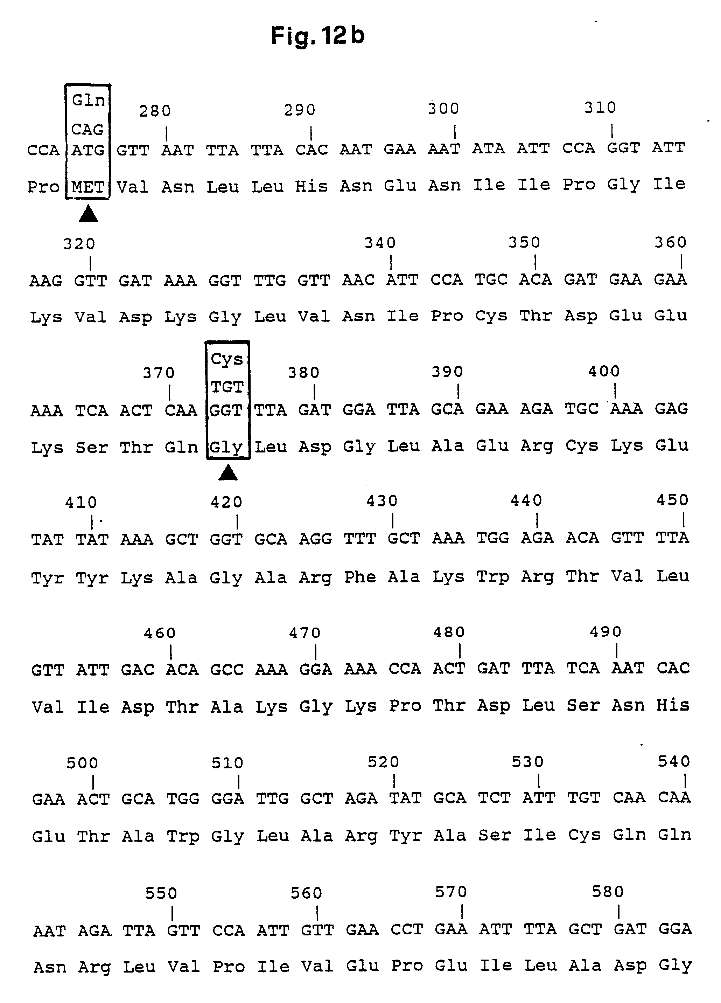

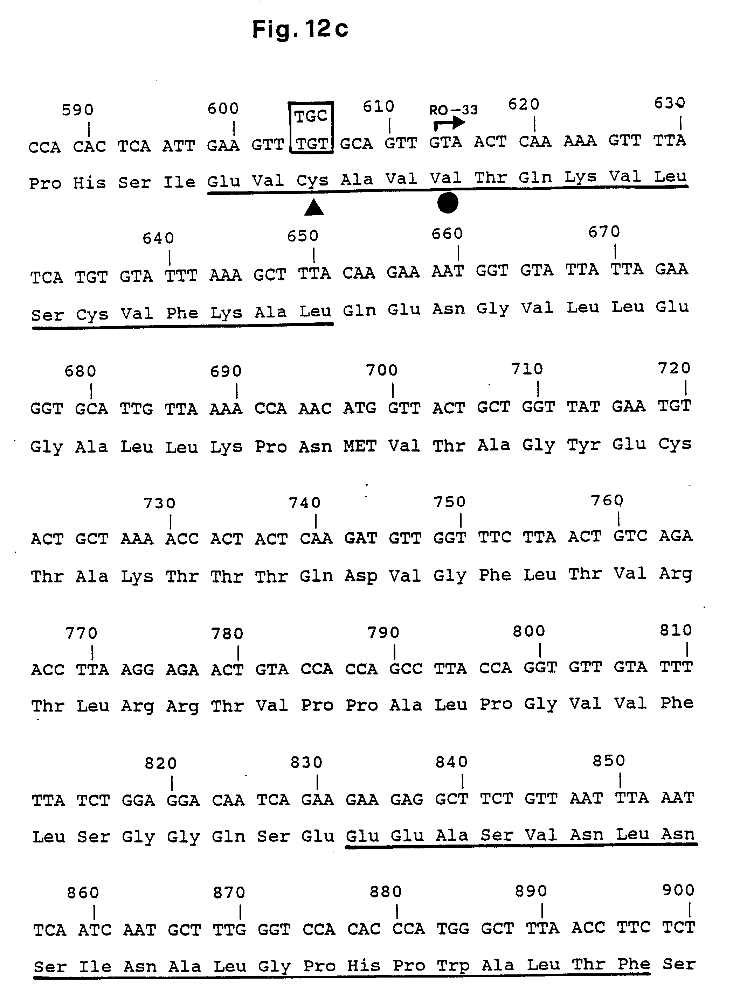

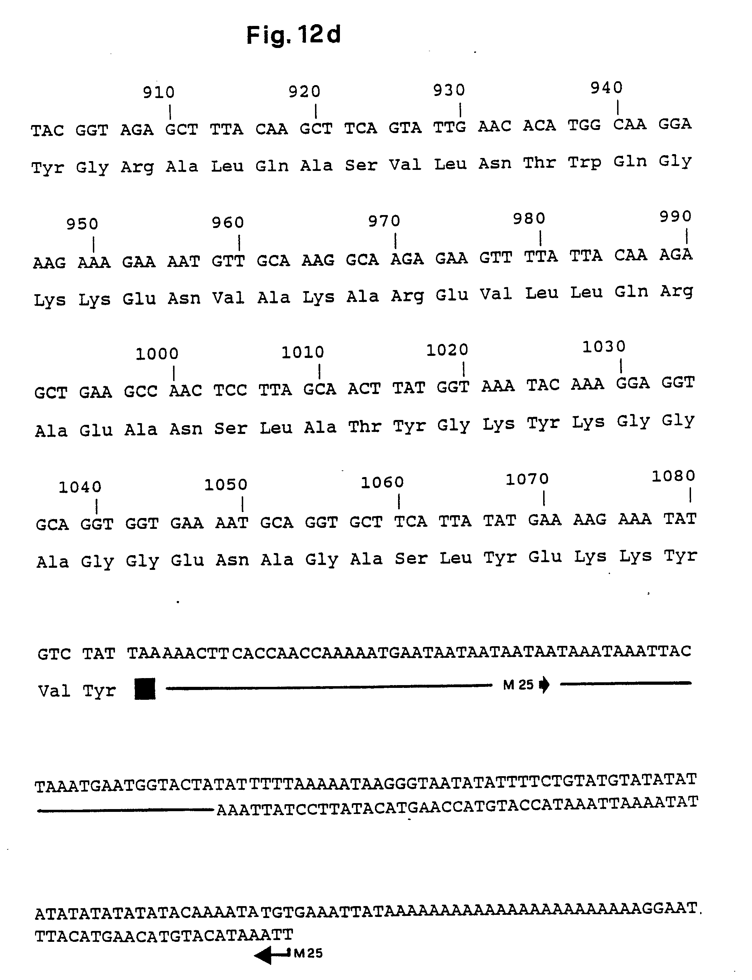

Fig. 12 Nukleotidsequenz und die davon abgeleitete Aminosäuresequenz der genomischen DNA des P.falciparum K1 Isolates, die für das 41'000 Dalton Merozoitenantigen kodiert. Das N-terminale Met ist unterstrichen. Der offene Leseraster endet mit dem Termiantionscodon TAA an Position 1087 bis 1089. Die Figur zeigt auch einen Teil der nichtkodierenden Sequenz vor und nach dem für das Merozoitenantigen kodierenden Sequenz. Die Nukleotidsequenz der genomischen DNA eines anderen Isolates (RO-33, Ghana) war weitgehend identisch mit der Nukleotidsequenz aus dem K1 Isolat. M25 bezeichnet die entsprechende Nukleotidsequenz einer cDNA des M25 Isolates von P.falciparum. Die Nukleotidsequenzen unterscheiden sich in der kodierenden Sequenz in 3 Codons, die in Figur 12 eingerahmt sind. Die Sequenzunterschiede führen zu zwei Aminosäureaustauschen, wobei ein Met bzw. ein Gly im Merozoitenantigen des K1 Isolates einem Gln bzw. einem Cys im Merozoitenantigen des M25 Isolates entspricht.

Verwendete Abkürzungen:

| | |
|---|---|
| ATP | Adenosintriphosphat |
| bp | Basenpaare |
| BSA | Rinderserumalbumin |
| cpm | Impulse pro Minute |
| dATP | Desoxyadenosintriphosphat |
| dCTP | Desoxycytidintriphosphat |
| dGTP | Desoxyguanosintriphosphat |
| dTTP | Desoxythymidintriphosphat |

DTT Dithiothreitol
EDTA Ethylendiamintetraessigsäure
IPTG Isopropyl-β-D-thiogalactopyranosid
kb 1'000 Basenpaare
kD Kilodalton
M molar
mM millimolar
ml Milliliter
nm Nanometer
PFU plaguebildende Einheiten
RPM Umdrehungen pro Minute
SDS Natriumdodecylsulfat
TEMED N,N,N′,N′-Tetramethylethylendiamin
Tris Trishydroxymethan
X-Gal 5-Brom-4-chlor-3-indonyl-β-D-galactopyranosid

Puffer und Medien

100 x Denhardt's (100 ml):

2 g Polyvinylpyrrolidon
2 g Ficoll
2 g BSA
100 mg Natriumazid

DNA-Gel Ladepuffer

1 x TBE (Zusammensetzung siehe unten)
20% Glycerin
0,1% Bromphenolblau
0,1% Xylencyanol

Formamid-Mix:

80% (w/v) Formamid
50 mM Tris/Borsäure [pH 8.3]
1 mM EDTA
0.1% Xylenxyanol
0.1% Bromphenolblau

HIN-Puffer:

10 mM Tris/HCl [pH 7.4]
10 mM Magnesiumchlorid
50 mM Natriumchlorid

Ligase-Puffer:

50 mM Tris/HCl [pH 7.8]
] 10 mM Magnesiumchlorid
20 mM DTT
10 mM dATP

LB-Medium: Pro Liter:

10 g Bactotrypton
5 g Hefeextrakt
10 g Natriumchlorid

SDS-Gel Ladepuffer:

5% SDS
5mM Tris/HCl [pH 6.8]
200 mM DTT
20% Glyzerin
0.1% Bromphenolblau

20 x SSC: Pro Liter:

175.3 g Natriumchlorid
82.2 g Natriumcitrat [pH 7.0]

SM-Puffer:

10 mM Natriumchlorid
10 mM Magnesiumchlorid
10 mM Tris/HCl [pH 7.4]

10 x T4-Polymerase Puffer:

0.33 M Tris/Acetat [pH 7,9]
0.66 Kaliumacetat
0.10 M Magnesiumacetat
5 mM DTT
1 mg/ml BSA

10 x TBE:

0.89 M Tris/Borsäure [pH 8.0]
0.89 Borsäure
20 mM EDTA

10 x TBS:

0.5 M Tris/HCl [pH 7.4]
1.5 M Natriumchlorid

100 x TE:

1 M Tris/HCl [pH 8.0]
100 mM EDTA

Folgende Methoden wurden im nachfolgenden Beispiel mehrmals verwendet und sind deshalb hier zusammengefasst.

Methode 1: DNA Fällung mit Lithiumacetat

Die DNA Lösung wird mit einem zehntel Volumen 5 M Lithiumacetat und zwei Volumen Isopropanol versetzt, gut gemischt und 10 Minuten auf Trockeneis gestellt. Die präzipitierte DNA wird 10 Minuten bei 12'000 RPM (20°C) in einer Eppendorftischzentrifuge zentrifugiert und der Ueberstand vorsichtig abgenommen. Das Sediment wird einmal mit 80% (v/v) Aethanol gewaschen und anschliessend während 5 Minuten in einer Vakuum-Zentrifuge getrocknet. Die DNA wird in Wasser gelöst und weiterverarbeitet.

Methode 2: Agarose Gelelektrophorese von DNA

Die getrocknete DNA wird in 1 x DNA Gel Ladepuffer gelöst und während 5 Minuten auf 65°C erhitzt. 100 ml 1 x TBE Puffer werden mit Agarose (800 mg für ein 0,8% Gel bzw. 1.2 g für ein 1.2 % Gel) versetzt und gekocht bis die Agarose vollständig gelöst ist. Nach dem Abkühlen werden 2 µl Ethidiumbromid-Lösung (10 mg/ml) zugesetzt und die Gelösung in eine horizontale Gelektrophorese-Apparatur gegossen (IBI, Genofit, Genf, Schweiz). Nach dem Erstarren des Gels, werden die Proben auf das Gel aufgetragen und die DNA während 2 Stunden bei 150 Volt konstanter Spannung aufgetrennt. Als Grössenmarker dienen kommerzielle Standardmischungen von DNA-Fragmenten definierter Länge (Gibco-BRL, Basel, Schweiz). Die DNA Banden werden unter 300 nm UV-Licht sichtbar gemacht.

Methode 3: Isolation von DNA aus einem Agarose-Gel

Die DNA wird auf einem Agarose-Gel aufgetrennt (Methode 2). Vor der Bande, die isoliert werden soll, wird ein Stück NA 45 Nitrocellulose Membran eingesetzt (Schleicher und Schuell, Dassel, BRD) und die DNA wird während 5 Minuten bei 200 V auf die Membran elektrophoretisiert. Die Membran wird mit einer Pinzette entnommen und unter fliessendem, destilliertem Wasser gewaschen. Die Membran wird in ein Eppendorf-Röhrchen übertragen und die DNA mit 200 µl 1.5 M Lithiumacetat, 10mM Tris/HCl [pH 8.0], 0.1 mM EDTA während 10 Minuten bei 65°C eluiert. Die Elution wird nochmals wiederholt. Die vereinigten Ueberstände werden mit 2 Volumen Isopropanol versetzt. Die ausgefällte DNA (Methode 1) wird in 50 µl Wasser gelöst.

Methode 4: Plaquereinigung von Lambda-Phagen

Eine Bakterienkultur (z.B. E. coli Y1088) auf einer Agarplatte wird mit Lambda-Phagen (z.B. gt11) infiziert. Dadurch entstehen im Bakterienrasen lytische Plaques. Mit einer umgedrehten Pasteurpipette wird ein Agarzylinder (Durchmesser 5 mm) enthaltend ein Plaque aus dem Agar ausgestochen. Der Agarzylinder wird in ein Eppendorf-Röhrchen enthaltend 50 µl SM-Puffer übertragen und das Röhrchen während 30 Minuten geschüttelt. Die Phagen-Suspension wird zentrifugiert (5 Minuten bei 12'000 RPM, 20°C) und der Ueberstand in ein frisches Röhrchen überführt. 1 µl der Phagen-Suspension wird mit 1 ml SM-Puffer verdünnt. Von dieser Lösung werden 1, 10 und 100 µl zu 50 µl einer gemäss Morrison (Methods Enzymol. 68, 326-331 [1979]) $Mg^{++}$ - behandelten Zellsuspension von E. coli Y1090 enthaltend das Plasmid pMC9 (ATCC No. 37197) gegeben. Nach 30 minütiger Inkubation bei Raumtemperatur wird die Lösung zu 3 ml 0.8% (w/v) Agar in LB-Medium gegeben und das Gemisch auf LB-Ampicillin Agarplatten (LB-Medium, 40 µg/ml Ampicillin) gegossen. Je nach Titer hat es auf einigen Platten (z.B. denjenigen mit der 1:1000 Verdünnung) einzelne Plaques die, wenn sie in der Antikörperreaktion oder der DNA-Hybrdisisierung positiv sind, isoliert werden können. Die Phagen aus den Plaques können vermehrt werden und z.B. zur Isolation von Phagen DNA verwendet werden.

Methode 5: Isolation von Lambda-Phagen DNA

Mit einem sterilen Zahnstocher wird ein einzelner Plaque von einer Agarplatte gepickt und der Zahnstocher während 30 Minuten in 500 µl SM-Puffer bei 37 °C inkubiert. Der Zahnstocher wird herausgenommen und die Phagenlösung zentrifugiert (10 Minuten 12'000 RPM, 20 °C). Der Ueberstand wird in ein frisches Gefäss überführt und mit 50 µl Chloroform versetzt. 100 µl der Phagenlösung werden entnommen und mit 50 µl einer $Mg^{++}$ - behandelten Zellsuspension von E. coli Y1090 (ATCC Nr. 37197) gemischt. Nach 30-minütiger Inkubation bei Raumtemperatur wird die Suspension mit 3 ml 0.8% (w/v) Agar in LB-Medium vermischt und auf LB-Ampicillin Agarplatten gegossen (s. Methode 4). Nach fünfstündiger Inkubation bei 37 °C werden die Petrischalen mit 5 ml SM-Puffer überschichtet und übernacht bei Raumtemperatur geschüttelt. Dadurch werden die Phagen aus dem Agar eluiert. Der SM-Puffer mit den Phagen wird abgegossen und während 10 Minuten bei 12'000 RPM (Raumtemperatur) zentrifugiert. Der Ueberstand (= Phagenstock) wird mit 100 µl Chloroform versetzt. Der Phagentiter im Phagenstock beträgt üblicherweise $10^{10}$-$10^{11}$ PFU/ml. 50 ml LB Medium (mit 40 µg/ml Ampicillin und 10 mM Magnesiumchlorid) werden mit 250 µl einer gesättigten Kultur von E. coli Y1088 enthaltend Plasmid pMC9 (ATCC Nr. 37195) und 1 ml Phagenstock angeimpft. Die Kultur wird übernacht bei 37 °C geschüttelt. Nach Zugabe von 2 ml Chloroform werden Zellbruchstücke abzentrifugiert (10 Minuten bei 12'000 RPM, 20 °C). Zum Ueberstand werden je 50 µl einer DNase I und RNase Lösung (je 10 mg/ml in Wasser) pipettiert, und das Gemisch während 30 Minuten bei 37 °C inkubiert. Zur Phagensuspension (45 ml) werden 14 ml 35% (w/v) Polyaethylenglykoll-6000 (SIGMA, St. Louis, Missouri, USA), 2.5 M Natriumchlorid pipettiert und nach gutem Mischen wird die Lösung eine Stunde auf Eis gesetzt. Die Phagen werden durch Zentrifugation (20 Minuten bei 12'000 RPM, 4 °C) sedimentiert und in 1 ml SM-Puffer gelöst. Nach Zugabe von 10 µl 25% (w/v) Lithiumdodecylsulfat-Lösung (Serva, Chemie Brunschwig AG, Basel, Schweiz) und 5 µl 0.5 M EDTA [pH 8.0] und einer Spatelspitze Proteinase K (Merck, Darmstadt, BRD) werden die Phagenpartikel während 10 Minuten bei 65 °C lysiert. Zu dem Lysat wird 1 Volumen Phenol gegeben, das zuvor mit 1 M Tris/HCl [pH 8,0] gesättigt wurde. Nach gutem Mischen werden die Phasen durch Zentrifugation getrennt (5 Minuten bei 6'000 RPM). Der Ueberstand wid abgenommen, die Extraktion wird wiederholt und die DNA wird aus dem zweiten Ueberstand nach Methode 1 gefällt.

Methode 6: Vektorvorbereitung

1 µg Plasmid- oder Phagen-DNA werden 1 Stunde bei 37 °C mit 10 Einheiten Restriktionsenzym in 100 µl T4-Polymerase Puffer verdaut. Es werden 400 µl Wasser und 5 Einheiten bakterielle Phosphatase (Gibco-BRL) zupipettiert und während einer Stunde bei 65 °C dephosphoryliert. Die Lösung wird zweimal mit Phenol extrahiert und gefällt (Methode 1). Das Vektorfragment wird über ein Agarosegel gereinigt (Methode 2), isoliert (Methode 3) und in 50 µl Wasser gelöst.

Methode 7: Transformation von E.coli

Eine 3 ml LB-Kultur wird mit E.coli Zellen angeimpft und über Nacht bei 37 °C geschüttelt. 1 ml dieser gesättigten Kultur dient zum Animpfen einer 50 ml LB-Flüssigkultur. Diese wird solange geschüttelt bis die optische Dichte bei 600 nm ($OD_{600}$) einen Wert von 0,2 erreicht hat. Die Zellen werden sedimentiert (5 Minuten bei 6'000 RPM, Raumtemperatur) und in 50 ml eiskaltem 50mM Kalziumchlorid resuspendiert. Die Lösung wird während 30 Minuten auf Eis gestellt. Die Zellen werden erneut abzentrifugiert (s. oben) und in 10 ml 50 mM Kalziumchlorid, 20% Glyzerin suspendiert. Die kompetenten Zellen werden in 500 µl Portionen bei -80 °C eingefroren. Zur Transformation wird eine Portion langsam auf Eis aufgetaut (30 Minuten). 10 µl DNA Lösung (1-10 ng), 8 µl 30% (w/v) Polyäthylenglykoll (SIGMA), 10 µl 500 mM Magnesiumchlorid/100 mM Kalziumchlorid und 72 µl Wasser werden gut gemischt und mit 100 µl kompetenten Zellen während 20 Minuten auf Eis inkubiert. Anschliessend wird die Mischung noch 10 Minuten bei Raumtemperatur inkubiert.

Bei der Verwendung von Vektoren des Typs M13 (Yanisch-Perron et al., Gene 33, 103-119 [1985] werden nun 50 µl 10%(w/v) X-Gal (Gibco-BRL) in Dimethylformamid, 10 µl 100 mM IPTG (Gibco-BRL) in Wasser und 50 µl einer gesättigten TG-1 (Amersham, Brauschweig, BRD) Kultur zugegeben. Nach gutem Mischen werden 3 ml 0.8% (w/v) Agar in LB-Medium zugesetzt und die Mischung wird auf einer LB-Agarplatte ausgegossen. Die Petrischalen werden über Nacht bei 37 °C inkubiert.

Bei der Verwendung von Plasmid DNA, die auf Antibiotika-Resistenz selektioniert werden kann (pUC, pDS78/RBSII, 6xHis etc.), wird zur Transformationsmischung 1 ml LB-Medium gegeben und eine Stunde bei 37 °C inkubiert. Die Zellen werden während 3 Minuten bei 6'000 RPM (Raumtemperatur) abzentrifugiert und in 100 µl LB-Medium resuspendiert. Diese 100 µl werden mittles einer Rotations-Scheibe (Schütt, Göttingen, BRD) gleichmässig auf einer LB Agar-Platte, die die zur Selektion benötigten Antibiotika enthält, verteilt und ebenfalls über Nacht bei 37° C inkubiert.

Methode 8: Vorbereitung der DNA zur Sequenzierung

Wenn TG-1 Bakterien mit einem Vektor des Typs M13, der ein zu sequenzierendes DNA-Fragment enthält, wie oben beschrieben transformiert wird, so entstehen weisse Plaques. Diese weissen Plaques werden mit dem Zahnstocher gepickt und in 3 ml LB Medium resuspendiert. Dazu werden noch 10 µl einer gesättigten TG-1 Kultur gegeben. Das Gemisch wird während 5 Stunden bei 37° C geschüttelt. 1.5 ml Kultur werden in ein Eppendorf Röhrchen überführt und zentr^fugiert (5 Minuten bei 12'000 RPM, 20° C). 800 µl Ueberstand werden in ein neues Röhrchen überführt und mit 200 µl 20% (w/v) Polyäthylenglykol, 2.5 M Natriumchlorid Lösung gemischt und während 20 Minuten bei Raumtemperatur inkubiert. Der Rest der Kultur wird bei 4° C aufbewahrt bzw. zur Präparation von "Mini-Lysat" DNA (Methode 10) verwendet. Die Phagen werden durch Zentrifugation (10 Minuten bei 12'000 RPM, 20° C) präzipitiert. Das Sediment wird in 100 µl 1x TE Puffer gelöst und mit 100 µl gesättigtem Phenol extrahiert. Die Phasen werden durch Zentrifugation getrennt 95 Minuten bei 12'000 RPM). 80 µl der wässrigen Phase werden in ein neues Reagenzröhrchen überführt, die DNA präzipitiert und in 12 µl Wasser gelöst (Methode 1).

Methode 9: DNA Sequenzierung nach Sanger

3 µl der gemäss Methode 8 vorbereiteten DNA werden mit 2 µl Wasser, 1 µl HIN-Puffer, 1 µl 25 µM dATP. 2 µl alpha-[$^{32}$P]-ATP (6000 Ci/mmol, Amersham) und 1 µl Sequenzierungsstarter (Pharmacia, Dübendorf, Schweiz) gemischt und während 5 Minuten bei 55° C erhitzt. Danach wird die Lösung auf Eis gesetzt. Zwischenzeitlich werden 4 Röhrchen mit je 3 µl der Stoplösungen A°, G°, T° und C° vorbereitet. Die Stoplösungen haben folgende Zusammensetzung:

A° : 3 µM ddATP, 112 µM dCTP, 112 µM dGTP, 112 µM dTTP
C° : 100 µM ddCTP, 11.5 µM dCTP, 112 µM dGTP, 112 µM dTTP
G° : 100 µM ddGTP, 112 µM dCTP, 5.6 µM dGTP, 112 µM dTTP
T° : 500 µM ddTTP, 85 µM dCTP, 85 µM dGTP, 5.6 µM dTTP.

Zu dem Röhrchen mit der DNA werden nun 5 Einheiten Klenow Polymerase (Pharmacia) pipettiert und gut gemischt. Je 3 µl dieser Lösung werden mit der Stoplösung vermischt und während 10 Minuten bei 37° C inkubiert. Zu jedem der vier Röhrchen wird 1 µl 0.25 mM dATP gegeben, gemischt und nochmals während 10 Minuten inkubiert. Schliesslich wird die Reaktion, durch Zugabe von 2 µl Formamid-Mix und 5 minütiges Erhitzen auf 96° C, gestoppt. Die DNA wird nun auf ein 0.2 mm Gel folgender Zusammensetzung aufgetragen:

6 ml 10x TBE-Puffer
28.8 g Harnstoff
3.6 Acrylamid (Bio-Rad Laboratories AG, Glattbrugg, Schweiz)
180 mg Bisacrylamid (Bio-Rad)
400 µl 10% Ammoniumpersulfat
20 µl TEMED

Die DNA wird während 1 bis 6 Stunden bei 40 Watt konstanter Leistung aufgetrennt. Die Glasplatten werden getrennt und das Gel während 5 Minuten in 10% (v/v) Essigsäure und 10% (v/v) Methanol fixiert. Das Gel wird dann zweimal 5 Minuten mit 10% (v/v) Methanol gewaschen, auf Whatman 3M Papier (Bender und Hobein, Zürich, Schweiz) aufgezogen und im Geltrockner getrocknet. Das trockene Gel wird während 2 bis 20 Stunden mit KODAK-XAR Film (Eastman Kodak Co., Rochester, New York, USA) autoradiographiert.

Methode 10: DNA Isolation in kleinem Masstab ("Mini-Lysat")

Etwa 1 bis 2 ml Bakterienkultur (z.B. E. coli TG-1 enthaltend ein Vektor des Typs M13; s. Methode 8) werden während 5 Minuten bei 12'000 RPM (20° C) zentrifugiert. Der Ueberstand wird vorsichtig abgesaugt. Die sedimentierten Zellen werden in 500 µl 50mM Tris/HCl [pH 7.6], 5 mM EDTA resuspendiert. Nach Zugabe einer kleinen Spatelspitze Lysozym (SIGMA) wird die Suspension während 5 Minuten bei Raumtemperatur inkubiert. Dann werden 15 µl 25% (w/v) Lithiumdodecylsulfat-Lösung (Serva) und 30 µl 5 M Kaliumacetat zugesetzt und die Suspension vorsichtig gemischt. Nach 15minütiger Inkubation auf Eis wird die Probe während 15 Minuten bei 12'000 RPM (4° C) zentrifugiert. Der Ueberstand wird in ein neues Röhrchen dekantiert und mit 50 µl RNase Lösung (10 mg/ml) versetzt. Nach 5 minütiger Inkubation bei 37° C wird die Probe einmal mit Phenol und einmal mit Chloroform extrahiert (jeweils gleiche Volumen). Die DNA in der wässrigen Phase wird gefällt (Methode 1) und schliesslich in 100 µl Wasser gelöst.

Methode 11: Radioaktive Markierung von DNA ("Nick-Translation")

Zu 20 µl DNA-Lösung werden folgende Reagenzien pipettiert:
5 µl HIN-Puffer, 10 µl alpha-[$^{32}$P]-ATP (6000 Ci/mMol, Amersham), 5 µl DNase I (1 ng/ ml), 5 µl 1 mM dCTP, dGTP, dTTP und 5 µl DNA Polymerase I (Boehringer Mannheim AG, Rotkreuz, Schweiz). Der Ansatz (50 µl) wird während 90 Minuten bei 14° C inkubiert und anschliessend einmal mit Phenol extrahiert (siehe Methode 5). Die wässrige Phase enthält die DNA-Probe und wird direkt für Hybridisierungsexperimente verwendet.

Methode 12: Hybridisierung von DNA

Der Filter mit DNA wird mit Vorhybridisierungs-Mix (2xSSC, 0.1% (w/v) Lithiumdodecylsulfat, 10 µg/ml denaturierte Kalbsthymus-DNA, 5 x Denhardt's, 5x TE-Puffer) während einer Stunde bei 60° C inkubiert. Die Kalbsthymus DNA wird vorher durch Kochen denaturiert. Der Vorhybridisierungs-Mix wird durch Hybridisierungs-Mix ersetzt, die der Vorhybridisierungsmix entspricht, jedoch zusätzlich etwa $10^7$ cpm an radioaktiver Probe enthält. Nach Inkubation über Nacht bei 60° C werden die Filter 3 mal 30 Minuten in 2 x SSC gewaschen. Die Filter werden getrocknet und über Nacht gegen Kodak XAR-Film exponiert.

Methode 13: Vorbereitung eines 12% SDS-Polyacrylamidgels nach Laemmli, Nature 227, 680-685 [1970]

60 ml Trenngel:15 ml 1.5 M Tris/HCl [pH 8.8], 0.4% (w/v)
SDS, 8mM EDTA.
24 ml 29% (w/v) Acrylamid (Bio-Rad),
1% (w/v) Bisacrylamid (Bio-Rad) in Wasser 25 ml Wasser.
500 µl 10% (w/v) Ammoniumpersulfat in Wasser.

Die Lösungen werden gemischt. Unmittelbar vor dem Giessen zwischen 2 Glasplatten werden 100 µl TEMED zugesetzt. Nachdem das Trenngel polymerisiert hat, wird das Sammelgel mit folgender Zusammensetzung gegossen:
20 ml Sammelgel:
5 ml 0.5 M Tris/HCl [pH 6.8], 0.4 % (w/v) SDS, 8 mM EDTA.
3 ml 29% (w/v) Acrylamid, 1% (w/v) Bisacrylamid in Wasser.
12 ml Wasser.
250 µl 10% (w/v) Ammoniumpersulfat-Lösung in Wasser.

Nach dem Mischen werden 30 µl TEMED zugegeben und ein Probenkamm wird vor dem Polymerisieren eingesetzt. Als Elektrophoresepuffer dient 190 mM Glycin, 25 mM Tris [pH 7,6], 1% (w/v) SDS. Als Grössenmarker werden kommerzielle Molekulargewichtsstandards aufgetragen (z.B. Bio-Rad).

Methode 14: Immunblot (Westernblot)

4 µl einer Proteinprobe werden auf einem 12% SDS-Poly acrylamidgel während 45 Minuten bei 25 mA konstantem Strom aufgetrennt. Das Gel wird entnommen und in Transfer-Puffer gelegt. Auf das Gel wird ein mit Wasser befeuchtetes Blatt Nitrocellulose (Schleicher & Scheull) gelegt. Gel und Nitrocellulose werden mit Whatman 3MM Papier belegt auf das dann jeweils ein Schwämmchen gelegt wird. Der so entstehende Sandwich wird in eine Elektrophorese-Apparatur eingeführt, wobei die Nitrocellulose gegen den Pluspol gerichtet wird. Der Transfer der Proteine erfolgt bei 400 mA konstantem Strom während 15 Minuten. Nach dem Uebertragen wird die Nitrocellulose während 10 Minuten in 1 x TBS Puffer geschüttelt. Anschliessend wird für 30 Minuten in 1 x TBS, 5% (w/v) Magermilchpulver vorinkubiert. Der Antikörper gegen das Parasitenantigen wird im Verhältnis 1:1000 in 1 x TBS, 5% (w/v) Magermilchpulver verdünnt und während einer Stunde mit dem Nitrocellulose-Blatt inkubiert. Danach wird das Blatt fünfmal während drei Minuten in frischem 1 x TBS gewaschen und anschliessend während einer Stunde mit Ziegen-anti-Kaninchen-Peroxidase Serum (Bio-Rad; 1:1000 verdünnt) in 1 x TBS, 5% (w/v) Magermilchpulver inkubiert. Die Nitrocellulose wird nochmals wie oben gewaschen und anschliessend in 50 ml 1 x TBS gelegt. Die Lösung wird mit 10 ml einer 4-Chlornaphtol Lösung (SIGMA, 50 mg/ml in Methanol) versetzt und gut gemischt. Durch Zugabe von 50 µl Wasserstoffperoxid wird die Farbreaktion gestartet. Nachdem die Banden sichtbar geworden sind, wird das Nitrocellulose-Blatt in Wasser aufbewahrt, um eine Ueberexposition zu vermeiden. Als Molekulargewichtsmarker dienen vorgefärbte Markerproteine, die gemäss den Angaben des Herstellers (z.B. Gibco-BRL) verwendet werden.

Methode 15: Southern-Transfer von DNA auf Nylonmembranen

Etwa 10 µg Plasmid oder Parasiten DNA pro Spur werden auf einem Agarose Gel aufgetrennt (Methode 2). Nach dem Lauf wird das Gel photographiert und zweimal während 15 Minuten in 0.2 N HCl bewegt. Anschliessend wird das Gel zweimal während 15 Minuten in 0.5 M Natronlauge bewegt. Das Gel wird zweimal während 15 Minuten in 0.5 M Tris/HCl [pH 8.0], 1.5 M Natriumchlorid neutralisiert und auf einen Schwamm gelegt, der mit 20 x SSC getränkt ist. Auf das Gel wird eine Nylonmembran (PALL, Basel, Schweiz) gelegt. Darauf kommen 3 Blatt Whatman 3 MM Papier und etwa Papierhandtücher. Der Aufbau wird oben mit 500 g Gewicht beschwert. Nach drei Stunden wird die Membran herausgenommen und zuerst bei Raumtemperatur und anschliessend während 1 Stunde bei 80°C im Vakuum getrocknet. Die Membran kann gemäss Methode 12 weiterbehandelt werden.

## Beispiel

### Konstruktion des Plasmids pDS78/RBSII, 6xHis

1. Beschreibung der Plasmide pDS78/RBSII und pDMI,1

Zur Konstruktion des Plasmids pDS78/RBSII, 6xHis wurde das Plasmid pDS78/RBSII verwendet. Mit diesem Plasmid sowie dem Plasmid pDMI,1 transformierte E.coli M15 Zellen wurden bei der Deutschen Sammlung von Mikroorganismen in Göttingen am 3. September 1987 hinterlegt [E. coli M15 (pDS78/RBSII; pDMI,1), DSM-Nr: 4232].

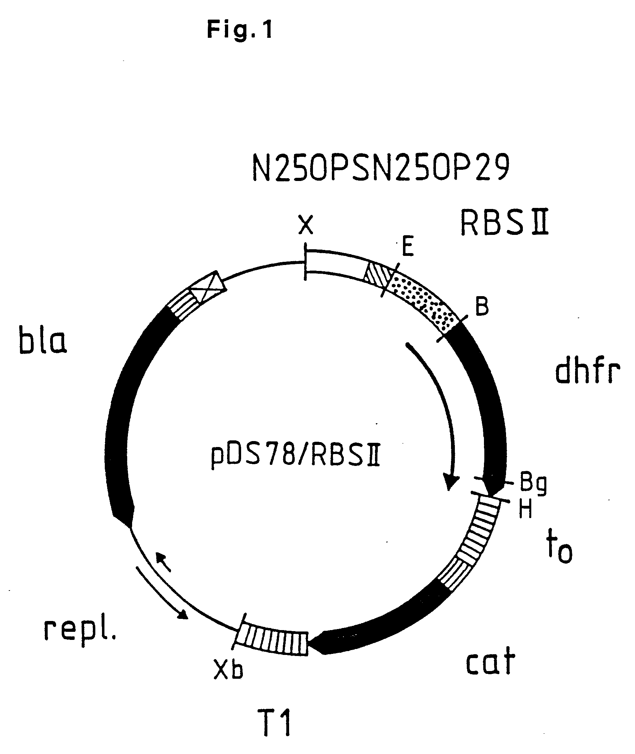

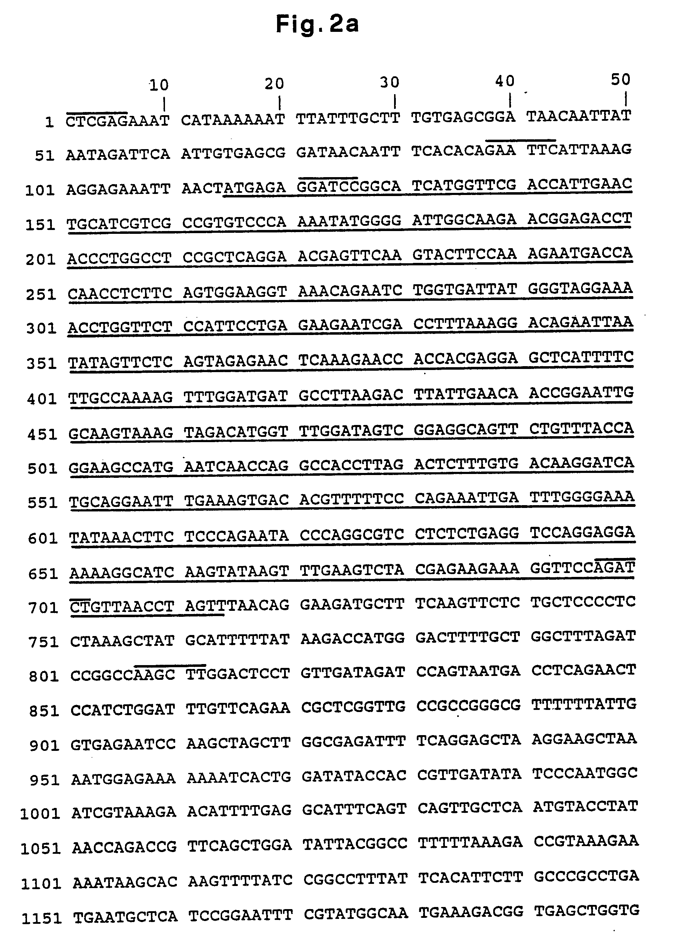

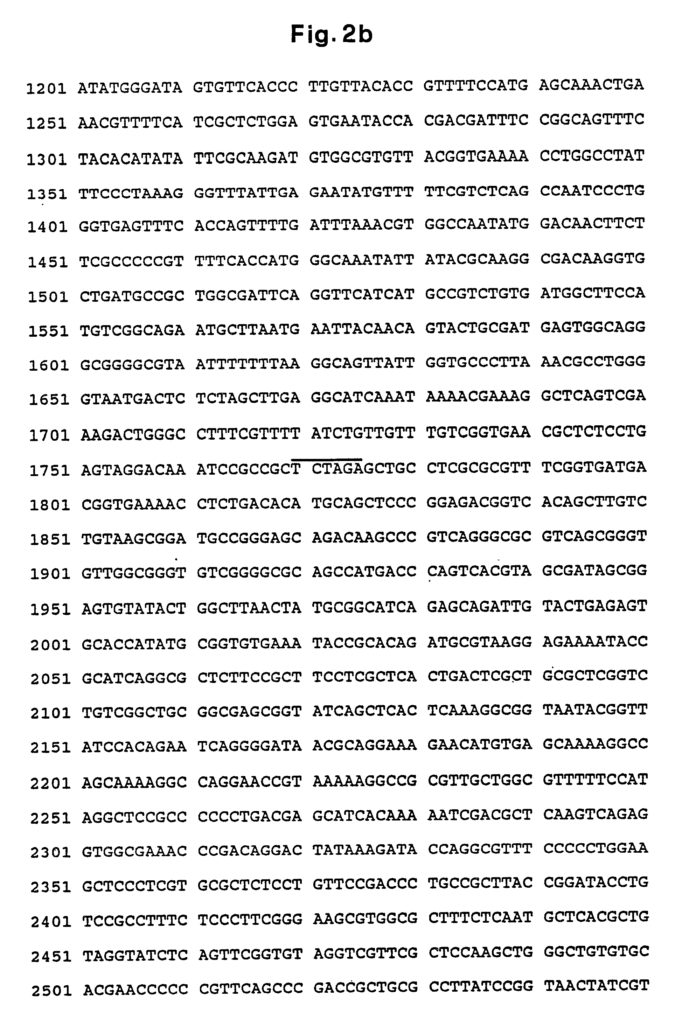

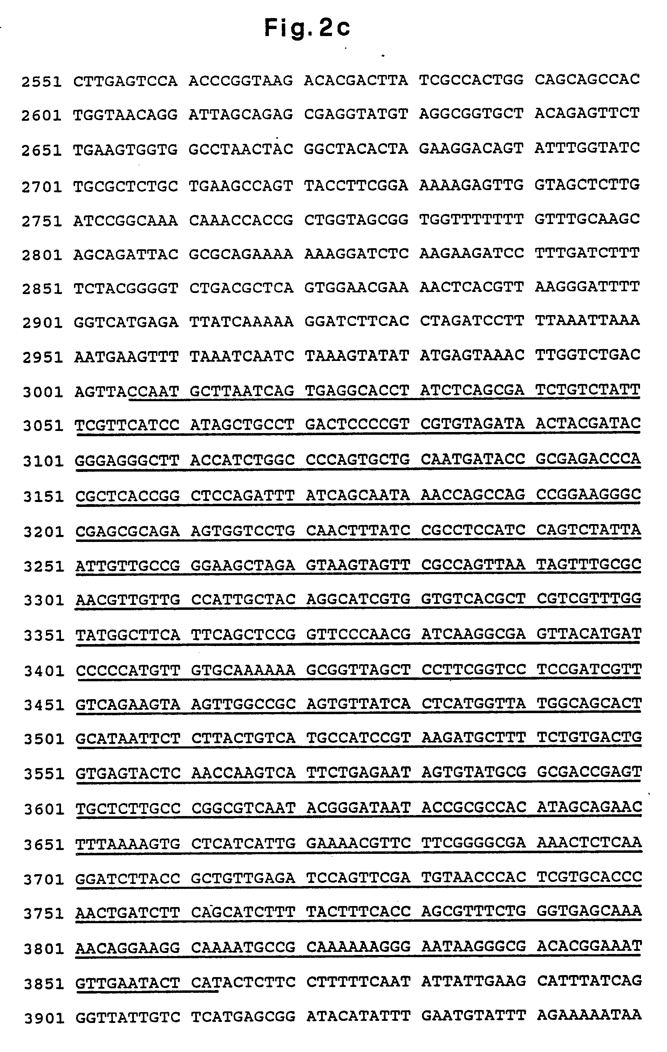

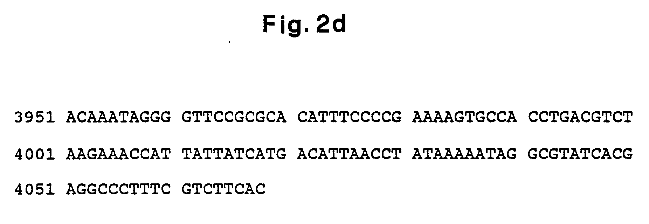

Der Anteil von pDS78/RBSII (Fig. 1 und 2), der zwischen den Restriktionsschnittstellen für XbaI und XhoI liegt und die Replikationsregion sowie das Gen für β-Lactamase, das den Zellen Ampicillinresistenz verleiht, enthält, stammt ursprünglich aus dem Plasmid pBR322 (Bolivar et al., Gene 2, 95-113 [1977]; Sutcliffe, Cold Spring Harbor Symp, Quant. Biol. 43, 77-90 [1979]). Jedoch ist das Gen für die β-Lactamase dahingegen modifiziert, dass die Schnittstellen für die Restriktionsenzyme HincII und PstI eliminiert sind. Diese Veränderungen in der DNA-Sequenz wirken sich aber nicht auf die Aminosäuresequenz der β-Lactamase aus. Der verbleibende Teil des Plasmids trägt als Expressionskontrollsequenz das regulierbare Promotor/Operator-Element N25OPSN25OP29 (R. Gentz, Dissertation, Universität Heidelberg, BRD [1984]) und die ribosomale Bindungsstelle RBSII. Diese ribosomale Bindungsstelle wurde von der ribosomalen Bindungsstelle des Promotors $P_{G25}$ des E.coli-Phagen T5 (R. Gentz, supra) abgeleitet und als EcoRI/BamHI-Fragment über DNA-Synthese erhalten. Es folgt das Gen der Dihydrofolatreduktase der Maus-Zellinie AT-3000 (Chang et al., Nature 275, 617-624 [1978]; Masters et al., Gene 21, 59-63 [1983]), das dahingehend verändert wurde, dass direkt vor dem Terminationscodon für die Translation eine Schnittstelle für das Restriktionsenzym BglII eingeführt wurde. Weiterhin enthält das Plasmid pDS78/RBSII den Terminator $t_0$ des E.coli-Phagen Lambda (Schwarz et al., Nature 272, 410-414 [1978]), das Promotor-freie Gen der Chloramphenicolacetyltransferase (Marcoli et al., FEBS Letters, 110, 11-14 [1980] und den Terminator T1 des E.coli rrnB Operons (Brosius et al., J. Mol. Biol., 148, 107-127 [1981]).

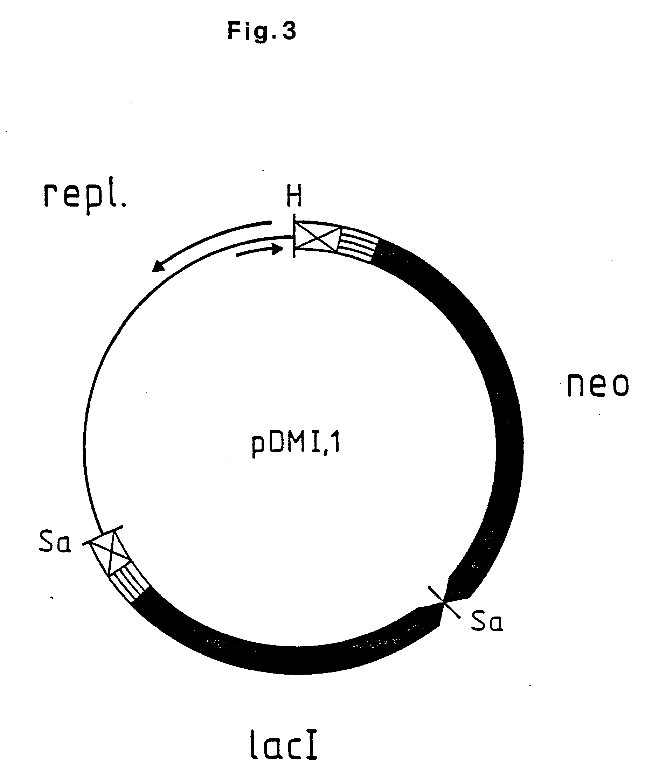

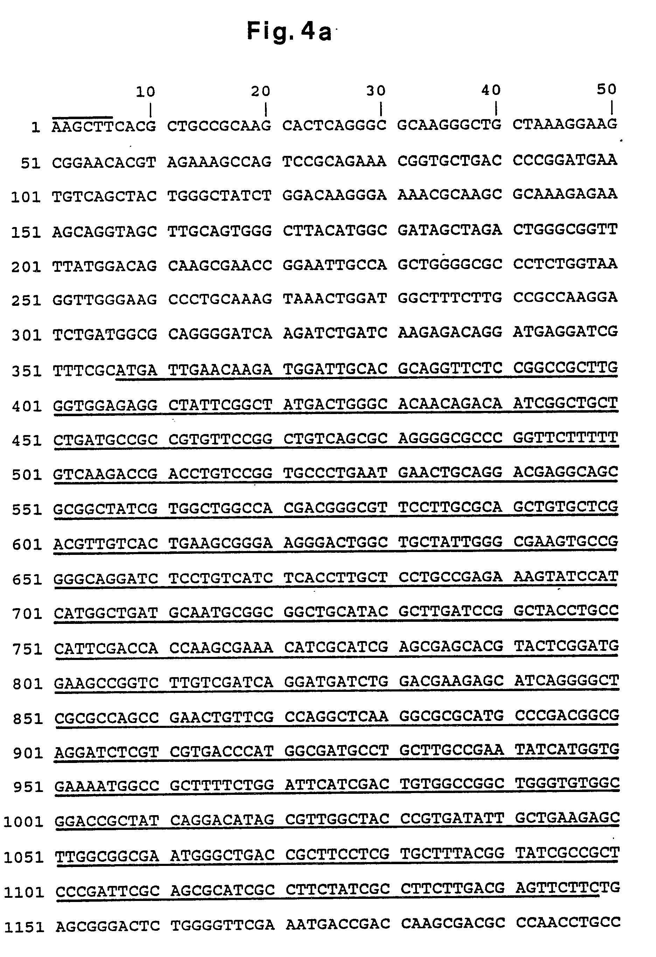

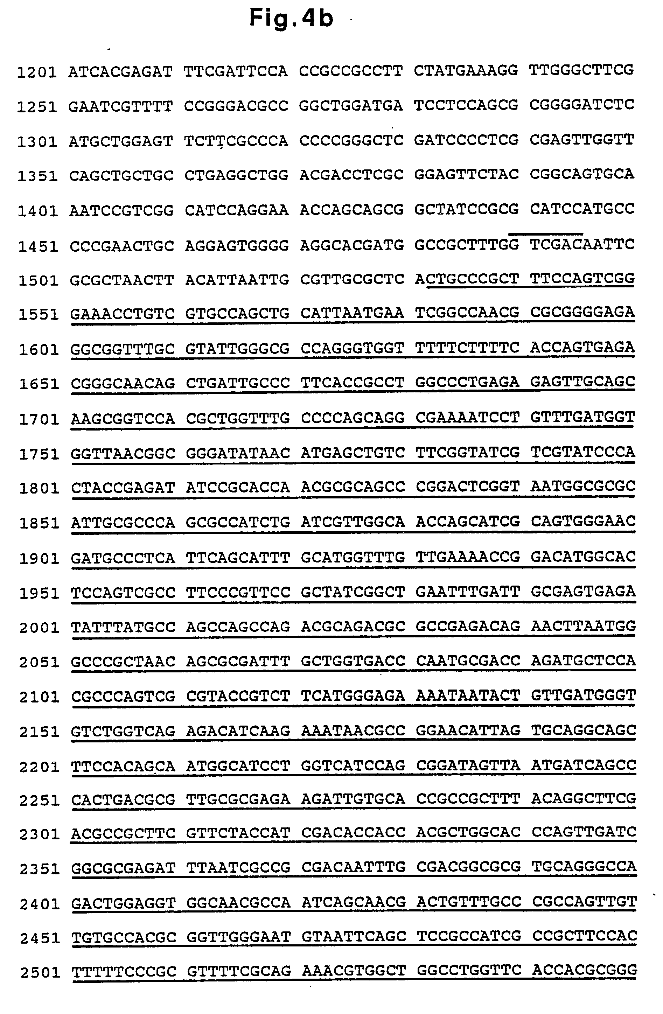

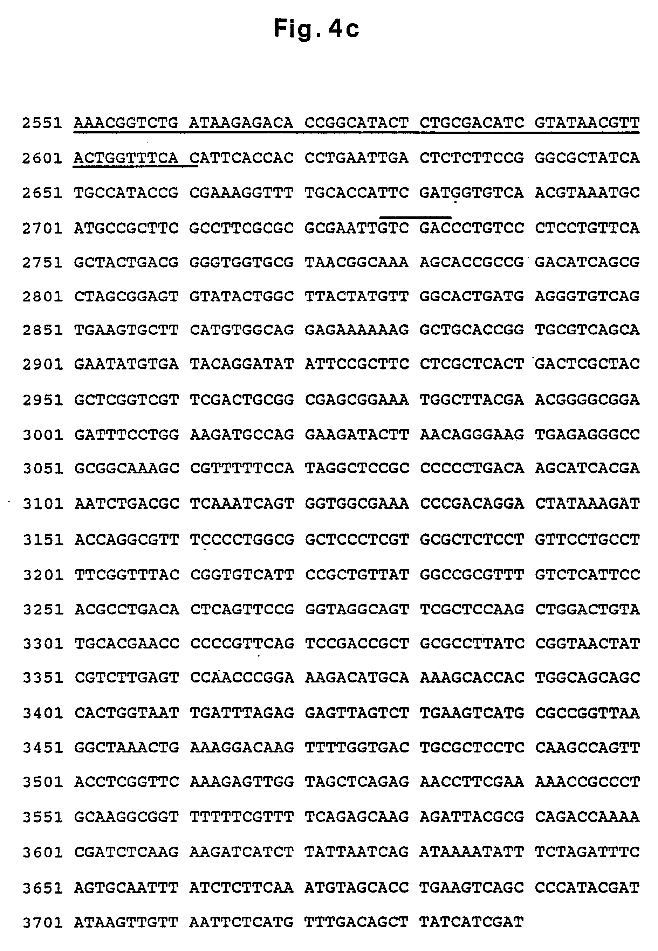

pDS78/RBSII enthält das regulierbare Promotor/Operator-Element N25OPSN25OP29 sowie die ribosomale Bindungsstelle RBSII. Auf Grund der hohen Effizienz dieser Expressionssignale können das Plasmid pDS78/RBSII und seine Derivate, wie das Plasmid pDS78/RBSII, 6xHis, nur dann stabil in E.coli-Zellen gehalten werden, wenn das Promotor/Operator Element durch das Binden eines lac-Repressors an den Operator reprimiert wird. Der lac-Repressor wid durch das lacl-Gen kodiert. N25OPSN25OOP29 kann nur dann effizient reprimiert werden, wenn eine ausreichende Zahl von Repressormolekülen in den Zellen vorhanden ist. Daher wurde das lacl$^q$-Allel benutzt, das eine Promotormutante enthält, die zu einer erhöhten Expression des Repressorgens führt. Dieses lacl$^q$-Allel ist im Plasmid pDMI,1 (Fig. 3 und 4) enthalten. Dieses Plasmid trägt zusätzlich zum lacl-Gen das neo-Gen, das den Bakterien Kanamycinresistenz verleiht. Die Kanamycinresistenz kann als Selektionsmarker benutzt werden. pDMI,1 ist mit den oben erwähnten Plasmiden kompatibel. E.coli-Zellen, die mit den oben beschriebenen Expressionsvektoren transformiert werden, müssen pDMI,1 enthalten, um sicherzustellen, dass der Expressionsvektor stabil in den Zellen gehalten wird. Eine Induktion dieses Systems wird durch Zugabe von IPTG ins Medium bei der gewünschten Zelldichte erreicht.

Das Plasmid pDMI,1 (Fig. 3 und 4) trägt das neo-Gen der Neomycinphosphotransferase aus dem Transposon Tn5 (Beck et al., Gene 19, 327-336 [1982]), das E. coli-Zellen Kanamycinresistenz verleiht sowie das lacl-Gen (Farabough, Nature 274, 765-769 [1978]) mit der Promotormutation I$^q$ (Calos, Nature 274, 762-765 [1978]), das den lac-Repressor kodiert. Ausserdem enthält das Plasmid pDMI,1 eine Region des Plasmids pACYC184 (Chang et al., J. Bacteriol. 134, 1141-1156 [1978]), die alle Informationen enthält, die für die Replikation und stabile Weitergabe an die Tochterzellen notwendig sind.

2. Konstruktion des Plasmids pDS78/RBSII, 6xHis

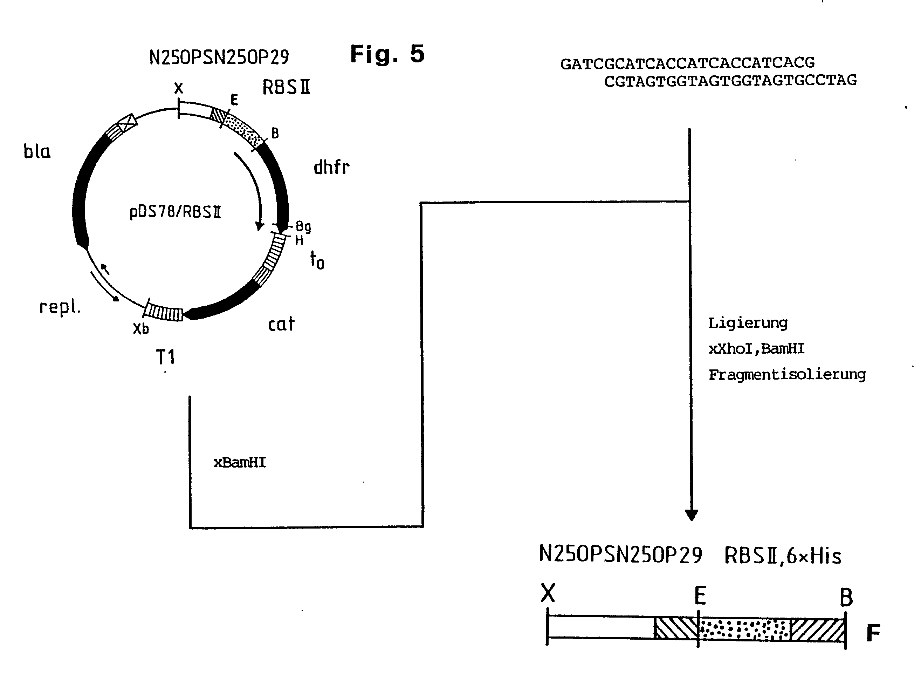

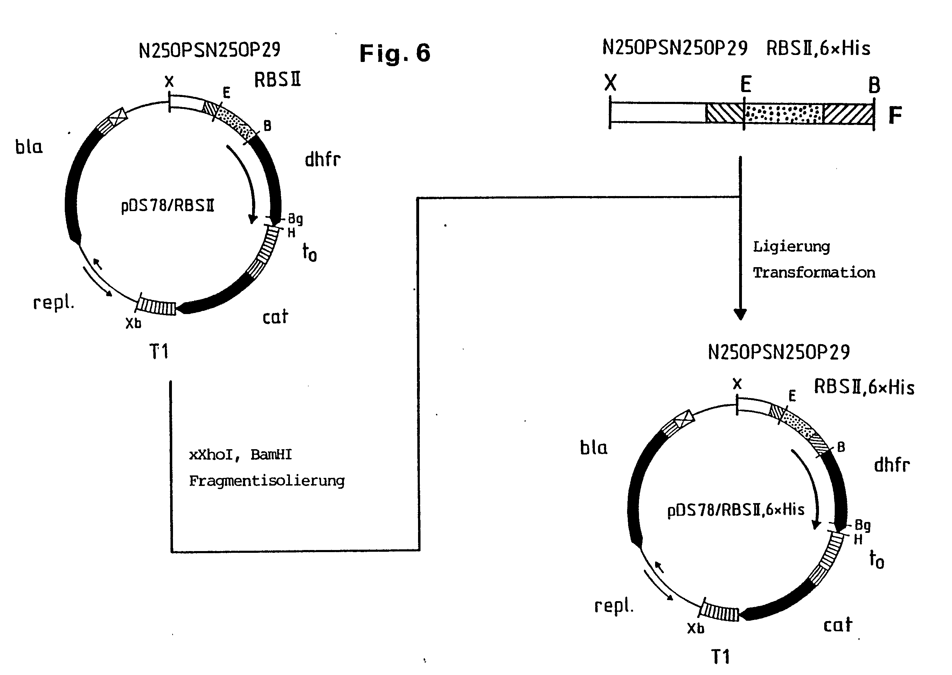

Zur Konstruktion des Plasmids pDS78/RBSII, 6xHis (Fig. 5 und 6) wurde das EcoRI/BamHI-Fragment von pDS78/RBSII mit der ribosomalen Bindungsstelle RBSII um eine Region verlängert, die für sechs Histidine kodiert.

Dazu wurden zunächst zwei komplementäre Oligonukleotide, deren Nukleotidsequenz in Fig. 5 als doppelsträngige DNA-Sequenz dargestellt ist, simultan auf einem Multisyntheseapparat (beschrieben in der Europäischen Patentanmeldung, Publikations-Nr. 181 491, veröffentlicht am 21.05.85) hergestellt, wobei Glas definierter Porengrösse (CPG) als Trägermaterial verwendet wurde (Kiefer et al., Immunol. Meth. 3, 69-83 [1985]; Sproat et al., Tetrahedr. Lett., 24 5771-5774 [1983]; Adams et al., J. Amer. Chem. Soc., 105, 661-663 [1985]). Die lyophilisierten Oligonukleotide wurden in Wasser aufgenommen und 1 Stunde bei 4° C gelöst. Die DNA-Konzentration betrug 100 nMole/ml. Zur Phosphorylierung wurden jeweils 150 pMole der beiden Oligonukleotide in 20 µl 50 mM Tris/HCl [pH 8,5] und 10 mM $MgCl_2$ mit 2 pMolen $\gamma$-[$^{32}$P]-ATP (5000 Ci/mMol, Amersham) und 1 Einheit (E) T4-Polynukleotidkinase (Gibco-BRL) während 20 Minuten bei 37° C inkubiert. Anschliessend wurden 5 nMole ATP zugegeben und nach weiteren 20 Minuten bei 37° C wurden die Reaktionen durch Erhitzen auf 65° C beendet.

Die DNA des Plasmids pDS78/RBSII wurde zur Ligierung mit den beiden phosphorylierten Oligonukleotiden vorbereitet, indem zunächst 2 pMole der Plasmid DNA mit dem Restriktionsenzym BamHI nach Angaben des Herstellers geschnitten wurden. Die DNA wurde mit Phenol extrahiert, mit Aether gewaschen und gemäss Methode 1 ausgefällt. Das Sediment wurde getrocknet und in 20 µl TE-Puffer aufgenommen. Zur Ligierung mit den phosphorylierten Oligonukleotiden wurden nun 1,5 pMole der mit BamHI geschnittenen Plasmid-DNA mit je 60 pMolen der phosphorylierten Oligonukleotide in Ligasepuffer mit 2 Einheiten T4-DNA Ligase während 2 Stunden bei 15° C inkubiert. Nach einer weiteren Inkubation bei 65° C während 5 Minuten wurde die ligierte DNA mit den Restriktionsenzymen XhoI und BamHI nach Angaben der Hersteller geschnitten, bevor das XHoI/BamHI-Fragment F mit dem regulierbaren Promotor N25OPSN25OP29, der ribosomalen Bindungsstelle RBSII sowie der für 6 Histidine kodierenden Region (Fig. 5) unter Verwendung der Methode 3 isoliert wurde.

Zur Konstruktion des Plasmids pDS78/RBSII,6xHis wurde das oben beschriebene XhoI/BamHI-Fragment F in das Plasmid pDS78/RBSII integriert, wobei das ursprüngliche XhoI/BamHI-Fragment dieses Plasmids ersetzt wurde (Fig. 6). Dazu wurden zunächst 1 pMol DNA des Plasmids pDS78/RBSII mit den Restriktionsenzymen XhoI und BamHI geschnitten und das grössere DNA-Fragment gemäss Methode 3 isoliert. 0,05 pMole dieses Fragments wurden nun mit 0,1 pMolen des isolierten XhoI/BamHI-Fragments F in Ligierungspuffer mit 2 Einheiten T4-DNA Ligase während 2 Stunden bei 15° C inkubiert. E.coli M15 Zellen transformiert mit Plasmid pDMI,1 wurden nach der Methode von Morrison (Methods Enzymol. 68, 326-331 [1979]) für die Transformation mit pDS78/RBSII,6xHis vorbereitet. Die Ligierungsmischung wurde nach 7-minütigem Erhitzen auf 65° C zu 200 µl dieser kompetenten Zellen hinzugefügt. Die Probe wurde 30 Minuten in Eis gehalten, dann 2 Minuten bei 42° C inkubiert, und nach der Zugabe von 0,5 ml LB-Medium während 90 Minuten bei 37° C inkubiert. Die Zellen wurden dann auf LB-Agarplatten, die 100 µg/ml Ampicillin und 25 µl/ml Kanamycin enthielten, ausplattiert und über Nacht im Brutschrank bei 37° C inkubiert. Einzelne Kolonien wurden mit einem sterilen Zahnstocher gepickt und in 10 ml LB-Medium enthaltend 100 µl/ml Ampicillin und 25 µg/ml Kanamycin während 12 Stunden im Schüttelinkubator inkubiert. Danach wurden die Zellen sedimentiert und die Plasmid-DNA nach der Methode von Birnboim et al. (Nucleic Acids Res. 7, 1515-1523 [1979]) isoliert.

Je 0,2 µg der isolierten Plasmide wurden mit den Restriktionsenzymen XhoI und BamHI geschnitten, um nachzuprüfen, ob das XhoI/BamHI-Fragment F in diesen Plasmiden enthalten ist. Plasmide mit einem solchen Fragment erhielten die Bezeichnung pDS78/RBSII,6xHis (Fig. 6).

Um nachzuweisen, dass die richtige Sequenz in pDS78/RBSII,6xHis enthalten ist, wurde die doppelsträngige zirkuläre Plasmid-DNA sequenziert, wobei eine mit $\gamma$-[$^{32}$P]-ATP markierte Startersequenz ("Primer") benutzt wurde. Dieses Startersequenz enthält die Nukleotide von Position 89-108 des Plasmids pDS78/RBSII. 0,3 pMole der isolierten Plasmid-DNA wurden mit Alkohol gefällt, das Sediment einmal mit 80% (v/v) Aethanol gewaschen, getrocknet und schliesslich in 8 µl 1/4 TE-Puffer gelöst. Die Probe wurde während 5 Minuten bei 95° C inkubiert, auf 0° C abgekühlt und zentrifugiert (2 Minuten, 12'000 RPM). 1,5 pMole der Startersequenz in einem Volumen von 2 µl wurden zugegeben bevor die Probe zunächst für 2 Minuten bei 95° C und dann für 5 Minuten bei 42° C inkubiert wurde. Dann wurde die DNA nach der Methode von Sanger et al. (Proc. Natl. Acad. Sci. USA 74, 5463-6567 [1977] sequenziert. Da ein radioaktiver markierter "Primer" verwendet wurde, wurden alle Reaktionen mit unmarkierten Desoxyribonukleotidtriphosphaten durchgeführt. Die DNA-Sequenzanalyse ergab, dass pDS78/RBSII,6xHis die in Fig. 5 angegebene Sequenz enthält.

## Isolation eines P.falciparum Gens mit Antikörpern aus einer genomischen Expressions-Genbank

### Konstruktion der Expressionsgenbank von P.falciparum

P.falciparum-Zellen (K1-Isolat) wurden mittels üblicher Methoden (Trager et. al., Science 193, 673-675 [1976]) in 10 Kulturschalen gezüchtet und anschliessend in Kulturmedium enthaltend 0,1% Saponin gewaschen. Die gewaschenen Parasiten wurden in 2 ml 10 mM EDTA, pH 8,0, 0,5% (w/v) SDS resuspendiert. Nach der Zugabe von 50 mg Proteinase K (Merck) wurde das Gemisch während 10 Minuten bei 65° C inkubiert und anschliessend mit 2 ml Phenol (gesättigt mit 1 M Tris/HCl [pH 8,0]) versetzt. Die Phasen wurden durch Schütteln vermischt und durch Zentrifugation (10 Minuten bei 6'000 RPM, 20° C) wieder getrennt. Die Phenolextraktion wurde zweimal wiederholt (es sollte keine Interphase mehr sichtbar sein). Die DNA gemäss Methode 1 gefällt, mit Aethanol gewaschen und getrocknet. Die DNA wurde in 2 ml Wasser gelöst und mechanisch geschert, d.h. 80-mal durch eine Spritze mit einer 0,5 x 16 mm Nadel gedrückt. Danach wurden 0,2 Volumen 5 x EcoRI Methylasepuffer (50 mM Tris/HCl [pH 7,5], 0,25 M NaCl, 50 mM EDTA, 25 mM β-Mercaptoäthanol, 0,4 mM S-Adebisylmethionin) zugegeben. 10 µg DNA wurden mit 50 Einheiten EcoR1 Methylase (New England Biolabs Beverly, Massachusetts, USA) während 30 Minuten bei 37° C methyliert. Die DNA wurde wie oben beschrieben einmal mit Phenol extrahiert und gemäss Methode 1 gefällt. Die DNA wurde in 200 µl T4-Polymerasepuffer gelöst und nach Zugabe von 5 µl 5 mM dATP, dCTP, dGTP und dTTP, sowie 10 Einheiten T4 Polymerase (Gibco-BRL) während 30 Minuten bei 37° C inkubiert. Die DNA wurde erneut mit Phenol extrahiert und gemäss Methode 1 gefällt. Die DNA wurde in 50 µl T4-DNA Ligasepuffer gelöst und nach Zugabe von 0,01 $OD_{260}$-Einheiten phosphorylierte EcoR1-Oligonukleotidadaptoren (New England Biolabs) und 2 µl T4-DNA-Ligase (12 Weiss-Einheiten, New England Biolabs) über Nacht bei 14° C ligiert. Die DNA wurde gemäss Methode 1 gefällt, in 20 µl 1 x DNA-Gelladepuffer gelöst und auf einem 0,8% Agarosegel aufgetrennt (Methode 2). DNA Fragmente mit einer Länge von 2 bis 6 kb (1 kb = 1'000 Nukleotide) wurden gemäss Methode 3 isoliert. Die erhaltene DNA wurde in 50 µl Wasser gelöst und nach Zugabe von 6 µl 10 x Ligase Puffer, 2 µl dephosphorylierten Lambda-Armen (Promega Biotech.) und 6 Weiss-Einheiten T4-DNA-Ligase übernacht bei 14° C ligiert. Die DNA wurde gefällt (Methode 1) und in 5 µl Wasser gelöst. Nach Zugabe von 20 µl "Packaging Extract" (Genofit S.A., Genf, Schweiz) wurde die DNA während 2 Stunden bei 20° C nach Vorschrift des Lieferanten in Phagenpartikel gepackt. Nach der Zugabe von 500 µl SM-Puffer sowie 50 Ml Chloroform war die Genbank bereit für den Antikörpertest.

### Antikörpertest der Genbank

Antikörper gegen Oberflächenproteine des Merozoitenstadiums von P. falciparum wurden, wie von Perrin et al. (J. Clin. Invest. 75, 1718-1721 [1984]) beschrieben, in Kaninchen hergestellt. Ein Antiserum das spezifisch war für ein P.falciparum K1 Merozoitenantigen mit einem Molekulargewicht von 41'000 wurde für den Antikörpertest der Genbank ausgewählt.

E. coli Y1090 wurde in 3 ml LB-Medium enthaltend 40 µl/ml Ampicillin übernacht bei 37° C im Schüttelbad inkubiert. Am anderen Morgen wurden die Zellen sedimentiert (10 Minuten bei 7'000 x g, 20° C) und in 1 ml SM-Puffer resuspendiert. Zu dieser Zellsuspension wurden $10^6$ infektiöse Phagenpartikel der Genbank zugegeben und während 30 Minuten bei Raumtemperatur inkubiert. Dann wurden 60 ml auf 42° C temperierte 0,8%ige Agarlösung in LB-Medium zugegeben und gut vermischt. Der Weichagar mit den infizierten Zellen wurde auf 6 LB-Agarplatten (Durchmesser 135 mm) enthaltend 40 µg/ml Ampicillin verteilt und während 5 Stunden bei 42° C inkubiert. Auf jede Schale wurde ein, in 100 mM IPTG-Lösung getauchter und getrockneter, Nitrocellulosefilter (Schleicher und Schuell) gelegt und übernacht bei 37° C inkubiert. Am nächsten Tag wurde die Lage des Filters bezüglich der Schale markiert und der markierte Filter in 1 x TBS aufbewahrt. Ein neuer, in 100 mM IPTG-Lösung behandelter, Nitrocellulose Filter wurde auf die Platte gelegt, markiert und während 4 Stunden bei 37° C auf den Platten inkubiert. Beide Filtersätze wurden während 10 Minuten in 1 x TBS geschüttelt, dann 20 Minuten in 1 x TBS, 20% FCS (fötales Kälberserum) inkubiert. Das Kaninchenantiserum wurde 1:1000 mit 1 x TBS/20% FCS verdünnt und beide Filtersätze während einer Stunde bei Raumtemperatur in einem Schüttelbad inkubiert. Die Filter wurden nun dreimal während je drei Minuten in 1 x TBS, 0,1% Triton®-X-100 (Bio-Rad) in einem Schüttelbad gewaschen, gefolgt von einer einstündigen Inkubation mit 5 µCi [$^{125}$J]-Protein A (Amersham, Katalog Nr. 1M.144) in 1 x TBS, 0,1% proteasefreiem Rinderserum Albumin (SIGMA). Die Filter wurden nochmals wie oben gewaschen und die Filter bei Raumtemperatur getrocknet. Die Filter wurden gegen Kodak XAR übernacht exponiert. Plaques die auf beiden Platten vorhanden waren wurden mit Hilfe der Markierungen identifiziert und anhand der Markierung auf den Petrischalen gepickt. Die einzelnen Proben wurden nochmals in verschiedenen Verdünnungen gemäss Methode 4 in Softagar ausplattiert und positive Plaques nochmals wie oben beschrieben identifiziert. Ein einzelner, positiver Plaque (K1-A) wurde gepickt, die Lambdaphagen gemäss Methode 5 vermehrt und die DNA isoliert.

10 µg K1-A DNA wurden mit 490 µl T4-Polymerasepuffer gelöst und mit 50 Einheiten HindIII während einer Stunde bei 37° C verdaut. Die DNA wurde gefällt (Methode 1) und auf einem 0.8% Agarose Gel analysiert (Methode 2). Als Kontrolle wurden 10 µg gt11 DNA mit HindIII verdaut und analysiert. Ein HindIII Fragment (270 Basenpaare), war nur in der Spur mit der K1-A DNA vorhanden. Das Fragment wurde isoliert (Methode 3), in 50 µl Wasser gelöst und bei 4° C aufbewahrt. Zur Sequenzierung wurden 50 ng HindIII geschnittene, dephosphorylierte M13 mp18 DNA (Pharmacia; Methode 6) mit 10 µl des gelösten HindIII Fragmentes aus K1-A gemischt, 2 µl 10 x Ligase Puffer, 6 µl Wasser und 6 Weiss-Einheiten T4-DNA Ligase (New England Biolabs) zugegeben und die DNA's während einer Stunde bei Raumtemperatur ligiert. Kompetente TG-1 E. coli Zellen wurden mit der ligierten DNA transformiert (Methode 7). Ein weisses Plaque wurde isoliert, amplifiziert und genügend DNA für die Bestimmung der Sequenz isoliert (Methode 8). Die DNA-Sequenz wurde gemäss der Methode 9 bestimmt. Der verwendete M13 mp18 Klon, mit dem HindIII Fragment wurde mit K1-A-M bezeichnet.

Das HindIII-Fragment aus der K1-A-M DNA wurde nun zur Isolation eines längeren DNA Stückes, das für das Merozoiten Antigen kodiert, verwendet. Dazu wurde die doppelsträngige DNA des M13 Klones K1-A-M isoliert (Methode 10). Nach Zugabe von 20 Einheiten HindIII wurden 5 µg DNA während einer Stunde bei 37° C verdaut. Die Lösung wurde wiederum gefällt (Methode 1). Die DNA wurde auf einem 1.2% Agarose Gel aufgetrennt (Methode 2). Das 270 bp HindIII-Fragment wurde isoliert (Methode 3) und die gereinigte DNA in 20 µl Wasser gelöst. Die DNA wurde mittels "Nick Translation" markiert (Methode 1). Die P.falciparum Lambda Genbank wurde nun wie oben beschrieben nochmals plattiert (2 x $10^5$ Phagenpar tikel auf zwei Petrischalen von 135 mm Durchmesser). Nach fünf Stunden, als Plaques sichtbar wurden, wurden die Petrischalen aus dem 37° C Inkubator genommen und über Nacht im Kühlschrank aufbewahrt. Auf die kalten Schalen werden nun PALL-Nylonfilter (PALL, Basel, Schweiz) gelegt und die relative Position der Filter auf den Petrischalen wurde mit Tinte markiert. Nach 5 Minuten wurden die Filter vorsichtig von der Platte abgehoben und mit der Seite mit den Plaques nach oben auf Whatmann 3MM Papier gelegt, das zuvor mit alkalischer Lösung (0.5 N Natriumhydroxid und 0.5 M Tris) getränkt worden war. Nach einigen Minuten wurden die Filter auf ein neues, mit der alkalischen Lösung getränktes Whatmann 3MM Papier gelegt. Danach wurden die Filter kurz auf einem 3MM Filterpapier getrocknet und dann zweimal während fünf Minuten auf Whatman 3MM Papier gelegt, das zuvor mit 1.5 M NaCl, 0.5 M Tris/HCl [pH 8.0] getränkt worden war. Die Filter wurden dann an der Luft getrocknet und während 90 Minuten bei 80° C im Vakuum gebacken. Die Hybridisierung der Filter mit dem 270 bp HindIII-Fragment (1 x $10^7$ cpm) als Probe erfolgte nach Methode 12.

Positive Plaques wurden wie oben beschrieben gepickt und die Spezifität der Hybridisierung mit der radioaktiven Probe nach Methode 4 und 12 nachgeprüft. Ein einzelner Plaque, K1-B, wurde nach Methode 5 vermehrt, die DNA wie oben beschrieben mit HindIII verdaut und auf Agarosegelen aufgetrennt (Methode 2). Die HindIII Fragmente, die nicht auch in der Vektor DNA vorhanden waren, wurden isoliert, in M13 mp18 kloniert und sequenziert (Methoden 6, 7, 8 und 9).

Restriktionsanalyse der P.falciparum DNA in der K1-B DNA

1 µg K1-B Lambda DNA in 50 µl T4-Polymerase Puffer wurde mit 5 Einheiten EcoRI während einer Stunde verdaut. Die DNA wurde analysiert (Methode 2) und ein 1.3 kb Fragment isoliert (Methode 3). Die isolierte Fragment DNA wurde in 20 µl Wasser gelöst. 1 µg pUC18 DNA (Pharmacia) wurde mit 5 Einheiten EcoRI verdaut und nach Methode 6 weiterverarbeitet. Nach der Isolation aus dem Gel (Methode 3) wurde der linearisierte Vektor in 50 µl Wasser gelöst. 1 µl Vektor wurden mit 5 µl 1,3 kb Fragment, 1 µl 10 x Ligase Puffer und 6 Weiss-Einheiten T4-DNA Ligase während 1 Stunde bei Raumtemperatur inkubiert. E. coli Zellen wurden mit der DNA gemäss Methode 7 transformiert und die Plasmid-DNA aus den Transformanten isoliert (Methode 10). Das erhaltene Plasmid wurde mit pK1-B bezeichnet. Je 0.5 µg pKl-B DNA wurden wie oben beschrieben mit den Restriktionsenzymen SphI, XmnI, HpaI bzw. in Doppelverdauungen zusätzlich noch mit HindIII verdaut und auf einem Agarosegel gemäss Methode 2 analysiert. Mit Hilfe dieser Restriktionsanalyse konnte die Gesamtsequenz der P. falciparum DNA in pK1-B bestimmt werden.

Expression des HpaI/SphI Fragmentes in E.coli

Ein für P.falciparum spezifisches HpaI/SphI Fragment wurde aus dem Klon pK1-B gemäss den Methoden 1 bis 3 isoliert. 6 µg pK1-B DNA wurden mit 15 Einheiten HpaI und 15 Einheiten SphI in 100 µl 1x T4-Polymerasepuffer während einer Stunde bei 37° C verdaut. Die DNA wurde gefällt (Methode 1), auf einem 0.8% Agarose Gel aufgetrennt (Methode 2) und ein 700 bp Fragment isoliert (Methode 3). Das Fragment wurde in 20 µl Wasser resuspendiert und nach Zugabe von 10 pMolen eines phosphorylierten BamHI Oligonukleotidadaptors (12-mer: CCCGGATCCGGG; New England Biolabs), 2.5 µl 10 x Ligase Puffer und 6 Weiss-Einheiten T4 DNA-Ligase übernacht bei 14° C ligiert. Die DNA wurde gefällt (Methode 1), in 50 µl 1x T4-Polymerase Puffer gelöst und nach Zugabe von 40 Einheiten BamHI während 1 Stunde bei 37° C verdaut. Die DNA wurde gefällt (Methode 1) und auf einem 1.0 prozentigen Agarose Gel aufgetrennt (Methode 2). Ein 700 bp Fragment wurde isoliert (Methode 3) und in 10 µl Wasser gelöst. Zur Vorbereitung des Vektors (siehe Methode 6) wurde 1 µg pDS78/RBSII,6xHis Vektor DNA mit 10 Einheiten BamHI während einer Stunde in T4-Polymerase Puffer bei 37° C verdaut. Die Vektor DNA wurde dephosphoryliert (Methode 4), einmal mit Phenol extrahiert (s. oben), auf einem 0.8 % Agarose Gel gereinigt (Methode 2) und anschliessend gemäss Methode 3 isoliert. Die isolierte DNA wurde in 50 µl Wasser gelöst.

5 µl der linearisierten pDS78/RBSII,6xHis Vektor DNA die mit BamHI verdaut und dephosphoryliert worden war (Methode 6), wurden mit 5 µl des 700 bp Fragments, 1.2 µl 10x Ligase Puffer und 6 Weiss Einheiten T4-DNA Ligase während einer Stunde bei Raumtemperatur inkubiert. 10 µl DNA wurden dann in kompetente M15 (pDMI,1) Zellen transformiert (Methode 7) und auf LB-Platten mit 100 µg/ml Ampicillin und 25 µg/ml Kanamycin plattiert. Einzelne Kolonien wurden mit einem Zahnstocher gepickt und in 3 ml LB-Medium enthaltend 100 µg/ml Ampicillin und 25 µg/ml Kanamycin überführt. Die Kulturen wurden bei 37° C im Schüttelbad inkubiert bis die optische Dichte bei 600 nm ($OD_{600}$) gegenüber reinem Medium 0,6 betrug. Ein Aliquot von 500 µl der Kultur wurde als nicht-induzierte Kontrolle entnommen. Zum Rest der Kultur wurde IPTG ( 1mM Endkonzentration) gegeben und die induzierte Kultur weitere 3 Stunden inkubiert. Danach wurden 500 µl der induzierten Kultur entnommen und zusammen mit der nicht-induzierten Probe zentrifugiert (3 Minuten bei 12'000 RPM, 20° C). Der Ueberstand wurde abgesaugt und das Zellsediment in 100 µl SDS-Probenpuffer resuspendiert. Die Proben werden während 7 Minuten gekocht und die Proteine auf einem 12% SDS-Gel (Methode 13) mittels Elektrophorese (drei Stunden bei 50 mA konstantem Strom) aufgetrennt. Das Gel wurde während 30 Minuten mit 0.1% (w/v) Coomassie Blau in 30% (v/v) Essigsäure und 10% (v/v) Methanol auf dem Schüttler gefärbt. Entfärbt wurde das Gel während 2 Stunden bei 65° C in 10% (v/v) Methanol und 10% (v/v) Essigsäure. Klone die gegenüber der uninduzierten Probe eine zusätzliche Bande mit dem zu erwartenden Molekulargewicht von 27 kD ( = 27'000 Dalton) zeigten, wurden gemäss Methode 14 analysiert. Das neue Protein wurde mit Protein (27 kD) bezeichnet. Die Aminosäuresequenz des exprimierten Proteins entspricht der Aminosäuresequenz (II).

Analyse von 11 verschiedenen Parasitenisolaten mit Antikörpern gegen das Antigen

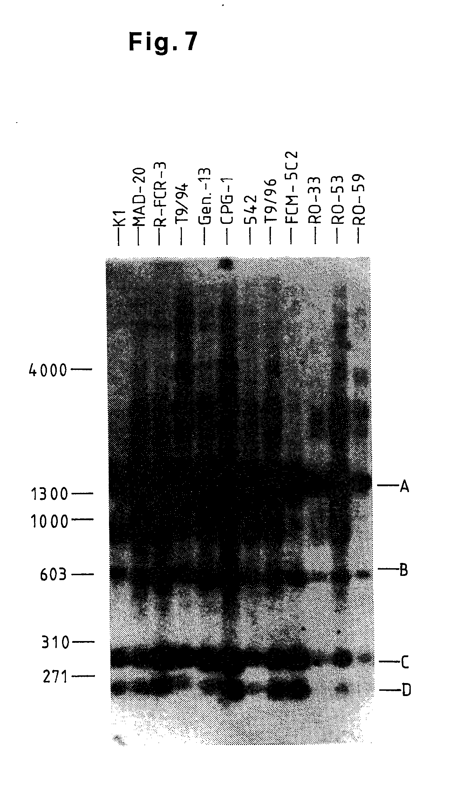

Folgende 11 Isolate von P.falciparum wurden getested: RO-33, Ghana; RO-56, Aethiopien; Geneva Nr. 13, Senegal; H-B3, Honduras; RO-53, Kamerun; R-FCR 3, Gambia; MAD-20, Papua Neu Guinea; 542, Brasilien; RO-58, Ost Afrika; FCH-5-C2, Tansania; K1, Thailand. Die Parasiten wurden aus Malariapatienten isoliert und nach Standardmethoden kultiviert. Es können aber auch Malariaparasiten aus andern Isolaten von P.falciparum verwendet werden. Parasiten von zwei Kulturschalen wurden abzentrifugiert (10 Minuten bei 1'500 RPM, 4° C) und in 200 µl SDS-Gelladepuffer gelöst. Nach siebenminütigem Kochen wurden die Proben auf einem SDS-Gel aufgetrennt (Methode 13). Die Proben wurden auf Nitrocellulose übertragen und mit Antikörpern gegen das Antigen getested (Methode 14). Das Ergebnis (Fig. 7) zeigt, dass das Antigen in allen Isolaten vorkommt und in allen Isolaten ein ähnliches Molekulargewicht hat.

Analyse von Parasitenisolaten mit Genproben aus K1

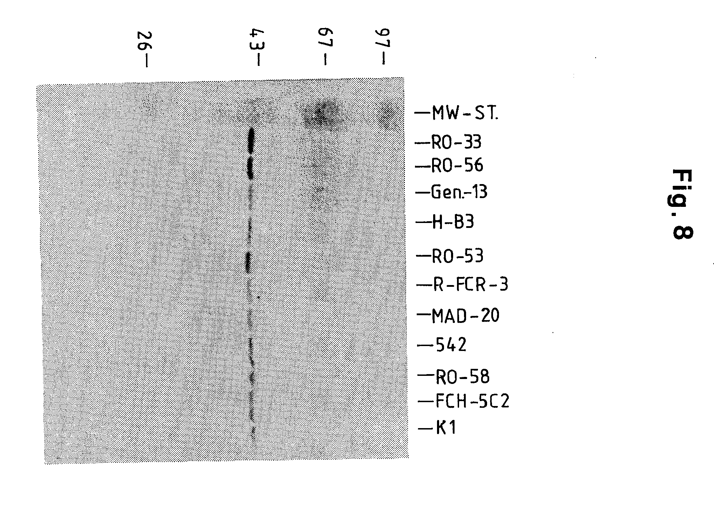

Das EcoRI Fragment aus pK1-B wurde gemäss den Methoden 2 und 3 isoliert und mittels "Nick-Translation" markiert (Methode 11). Die markierte Probe wurde als Hybridisierungsprobe eingesetzt ($10^6$ cpm). Aus verschiedenen P.falciparum Isolaten wurden je 10 µg DNA isoliert (s. oben), mit 50 Einheiten DraI in T4-Polymerase Puffer verdaut und auf einem 1.2% Agarose Gel aufgetrennt (Methode 2). Die DNA wurde auf einen Nylonfilter übertragen (Methode 15) und anschliessend hybridisiert (Methode 12). Als Ergebnis (Fig. 8) zeigt sich ein einheitliches Bandenmuster bei allen getesteten Isolaten und beweist zusammen mit Sequenzdaten von DNA aus verschiedenen Isolaten auf der Ebene der DNA, dass das Antigen, das den erfindungsgemässen Polypeptiden entspricht, konserviert ist.

Expression und Reinigung des Proteins (41 kD)

1. Konstruktion des Expressionsvektors

1 µl pK1-B DNA (Konzentration 0,5 µg/µl) wurde mit 100 µl 1 x T4-Polymerase Puffer gemischt. Nach Zugabe von 5 Einheiten EcoRI wurde 1 Stunde bei 37° C inkubiert. Die Probe wurde mit Isopropanol gefällt (Methode 1) und auf einem 0,8% Agarose Gel aufgetrennt (Methode 2). Das 1,3 kb EcoRI Fragment wurde gemäss Methode 3 isoliert. 0,5 µg M13 mp 18 DNA (Pharmacia) wurden mit EcoRI und Phosphatase inkubiert (Methode 6). 5 µl Vektor-DNA wurden mit 5 µl EcoRI Fragment aus pK1-B, 2 µl 10 x Ligase Puffer, 7 µl Wasser und 1 µl T4-DNA Ligase (6 Weiss-Einheiten, Pharmacia) gemischt und über Nacht bei 14° C ligiert. Die erhaltene DNA wurde nach Methode 7 in E. coli TG-1 transformiert. Ein weisses Plaque wurde gepickt (Methode 8) und zum Animpfen von 20 ml LB-Medium benutzt, das mit 200 µl einer gesättigten TG-1 Kultur versetzt worden war. Die Kultur wurde 5 Stunden bei 37° C geschüttelt und die Zellen anschliessend während 5 Minuten bei 12'000 RPM und 4° C zentrifugiert. Die Zellen wurden einmal in Wasser gewaschen und erneut zentrifugiert. Die M13 DNA (MK1-B) wurde nach Methode 10 isoliert. 50 µl DNA wurde mit je 5 Einheiten PstI und BamHI während 1 Stunde bei 37° C verdaut und nach Methode 1 gefällt. Das DNA Sediment wurde in 100 µl Exonuklease III Puffer (66 mM Tris/HCl [pH 8,0], 6.6 mM $MgCl_2$) gelöst. Nach Zugabe von 10 µl Exonuklease III (Gibco-BRL, 5000 Einheiten/77 µl) wurde das Gemisch während 30 Sekunden bei Raumtemperatur inkubiert. Die Probe wurde nach Zugabe von 10 µl 0,5 M EDTA während 10 Minuten bei 65° C inaktiviert und nach Methode 1 gefällt. Das Sedi ment wurde in 50 µ1 SI-Puffer (2 mM Kaliumacetat, 1 mM Zinksulfat, 5% (w/v) Glycerin) gelöst und nach Zugabe von 10 Einheiten S1-Nuklease (Gibco-BRL) während 30 Minuten bei Raumtemperatur inkubiert. Die Probe wurde zweimal mit Phenol extrahiert (Methode 6) und die DNA gefällt (Methode 1). Die DNA wurde in 12 µl HIN-Puffer gelöst. Nach Zugabe von 1 µl Klenow Polymerase (5 Einheiten, Pharmacia) wurde das Gemisch während 2 Minuten bei Raumtemperatur inkubiert und nach Zugabe von 1 µl 2 mM dATP, dCTP, dGTP, dTTP nochmals während 2 Minuten bei 37° C inkubiert. Es wurden 30 µl Wasser, 5 µl 10 x Ligase Puffer und 1 µl T4-DNA Ligase (6 Weiss-Einheiten, Pharmacia) zugegeben und der Ansatz übernacht bei 14° C ligiert. Die Mischung wurde in E. coli TG-1 transformiert (Methode 7). 4 weisse Plaques wurden gepickt, die DNA vorbereitet (Methode 8) und durch Sequenzierung analysiert (Methode 9). Die zur Expression (s. unten) verwendete DNA wurde M2/13 genannt. 50 µl M2/13 DNA wurden mit 20 Einheiten EcoRI vollständig verdaut. Die DNA wurde präzipitiert und in 50 µl T4-Polymerase Puffer gelöst. Die DNA wurde für 2 Minuten bei 37° C partiell mit 1 Einheit HindIII verdaut, gefällt (Methode 1) und auf einem 0,8% Agarose Gel getrennt (Methode 2). Das 1,3 kb DNA EcoRI-HindIII Fragment wurde isoliert (Methode 3). 50 µl DNA Lösung, 6 µl 10 x HIN-Puffer, 1 µl Klenow-Polymerase (5 Einheiten, Pharmacia) und 2 µl 5 mM dATP, dCTP, dGTP, dTTP wurden gemischt und während 30 Minuten bei 37° C inkubiert. Die DNA wurde gefällt (Methode 1), in 10 µl Wasser resuspendiert und nach Zugabe von 10 pMolen eines phosphorylierten BamHI Oligonukleotidadaptors (8-mer: CGGATCCG; New England Biolabs) sowie 2,5 µl 10 x Ligase Puffer und 6 Weiss-Einheiten T4-DNA-Ligase wurde übernacht bei 14° C ligiert. Die DNA wurde gefällt (Methode 1) und auf einem 1,9% Agarosegel aufgetrennt (Methode 2). Das 1,3 kb DNA Fragment wurde isoliert und wie oben beschrieben mit 0,1 µg pDS78/RBSII,6xHis Vektor ligiert. Das Neu entstandene Plasmid wurde mit p8/3 bezeichnet. Die Nukleotidsequenz dieses Plasmids ist in Fig. 11 gezeigt. Die weitere Analyse der, das Plasmid p8/3 enthaltenden, Klone erfolgte wie oben beschrieben über SDS-Protein Gele (Methode 13) und Immunblots (Methode 14). Das vom Plasmid p8/3 exprimierte Polypeptid ist ein Protein mit einem Molekulargewicht von etwa 41'000 Dalton. Das Protein, das im folgenden mit (41 kD) bezeichnet wird, wurde wie folgt gereinigt.

2. Reinigung des Proteins (41 kD) aus E. coli

60 g rekombinante E. coli Zellen enthaltend p8/3 wurden in zwei Portionen à 30 g mit je 70 g Glasmahlkörpern (Durchmesser 0,1 mm) und je 10 ml Puffer A (50 mM Tris/HCl [pH 7,0], 50 mM KCl) in einem Zellhomogenisator (Modell MSK, Braun, Melsungen, BRD) während dreimal 1 Minute aufgeschlossen. Der Zellaufschluss wurde mit 150 ml Puffer A verdünnt und zentrifugiert (10'000 x g, 30 Minuten, 4° C). Das gesuchte Protein (41 kD) verbleibt in gelöster Form im Ueberstand (= Rohextrakt).

20 g Cellex P (Bio-Rad) wurden in Puffer A gequollen und in eine Säule gepackt (Durchmesser = 5 cm, Länge = 7 cm). Nach dem Aequilibrieren der Säule mit Puffer A wurde der Rohextrakt mit einer Pumpe (Durchflussrate = 170 ml/Std.) auf die Säule aufgetragen. Durch Erhöhung der Phosphatkonzentration (Gradient mit 1M Kaliumphosphat [pH 7,0]) wurde das adsorbierte Protein eluiert (= Cellex P-Eluat).

Danach wurde das Cellex P-Eluat an ein Nickel-Nitrilotriessigsäureharz, das gemäss Hochuli et al., J. Chroatogr. 411, 177-184 [1987] hergestellt wurde, adsorbiert. Dazu wurde die NTA-Harz-Säule (Durchmesser = 1,6 cm, Länge = 9 cm, Durchflussrate = 170 ml/Std.) mit 0,1 M Tris/HCl [pH 7,5], 0,5 M NaCl äquilibriert und das adsorbierte Protein mittels eines Gradienten von 0 bis 0,5 M Imidazol eluiert (= NTA-Eluat).

Das NTA-Eluat wurde mittels Ultrafiltration an einer YM10-Membran (Amicon, Div. W.R. Grace + Co., Danvers, Massachusetts, USA) konzentriert und auf einer Sephacryl S-200 Säule (Pharmacia, Durchmesser = 2,6 cm, Länge = 83 cm, Durchflussrate = 14,6 ml/Std.) in $PBS^{--}$ Puffer (80 g NaCl, 2 g KCl, 2 g $KH_2PO_4$, 29 g $Na_2HPO_4 \cdot 12\ H_2O$ in 10 Liter pyrogenfreiem Wasser) chromatographiert. Die Ausbeute an gereinigtem Protein betrug 19 mg (bestimmt nach Lowry, J. Biol. Chem. 193, 265-275 [1951], mit BSA als Standard).

3. Immunologische und biochemische Analyse des Proteins

Das wie oben beschrieben gereinigte Protein (41 kD) wurde mittels Polyacrylamidgelelektrophorese und Western-Blot (Towbin et al., supra) analysiert (Fig. 10). Spur 3 (Fig. 10A) zeigt, dass das (41 kD) Protein im Vergleich zu den E. coli Proteinen stark angereichert ist. Im Endprodukt (Spur 5) sind keine E. coli Proteine mehr sichtbar (Fig. 10B). Figure 10C zeigt, dass das gereinigte Protein (41 kD) teilweise als Homodimer vorliegt. Dieses Homodimer bildet sich spontan. Es lässt sich nur teilweise durch Behandlung mit Mercaptoäthanol in Monomere auftrennen.

Die Aminosäuresequenz des vom Plasmid p8/3 exprimierten Proteins (41 kD) entspricht der Aminosäuresequenz (III‴).

Mittels eines Vergleichs der Aminosäuresequenz des Proteins (41 kD) mit der Aminosäuresequenz bekannter Proteine wurde festgestellt, dass das Protein (41 kD) starke Homologie zu Aldolasen aufweist. Es wurde daher untersucht, ob das gereinigte Protein Aldolaseaktivität aufweist. Dazu wurde ein Aldolasefarbtest gemäss den Vorschriften des Herstellers (SIGMA) verwendet. Das gereinigte Protein (41 kD) zeigte eine spezifische Aktivität von 13 µMol Fructose-1,6-diphosphat pro mg Protein und pro Minute bei 37° C. Der Endotoxingehalt des gereinigten Proteins (41 kD) wurde mittels eines LAL-Testes (Pyroquant Diagnostik GmbH, Walldorf, BRD) gemäss den Angaben des Herstellers bestimmt. Es wurde ein Endotoxingehalt von weniger als 3,1 EE/mg Protein gemessen (EE = Endotoxineinheiten).

## Ansprüche

1. Polypeptide die in mindestens einem spezifischen Epitop mit dem Plasmodium falciparum Merozoitenantigen mit der Aminosäuresequenz

MetAsnAlaProLysLysLeuProAlaAspValAlaGluGluLeuAlaThrThrAla-W-
LysLeuValGlnAlaGlyLysGlyIleLeuAlaAlaAspGluSerThrGlnThrIleLys
LysArgPheAspAsnIleLysLeuGluAsnThrIleGluAsnArgAlaSerTyrArgAsp
LeuLeuPheGlyThrLysGlyLeuGlyLysPheIleSerGlyAlaIleLeuPheGluGlu
ThrLeuPheGlnLysAsnGluAlaGlyValPro-X-ValAsnLeuLeuHisAsnGluAsn
IleIleProGlyIleLysValAspLys-Y-LeuValAsnIleProCysThrAspGluGlu
LysSerThrGln-Z-LeuAspGlyLeuAlaGluArgCysLysGluTyrTyrLysAlaGly
AlaArgPheAlaLysTrpArgThrValLeuValIleAspThrAlaLysGlyLysProThr
AspLeuSerAsnHisGluThrAlaTrpGlyLeuAlaArgTyrAlaSerIleCysGlnGln
AsnArgLeuValProIleValGluProGluIleLeuAlaAspGlyProHisSerIleGlu
ValCysAlaValValThrGlnLysValLeuSerCysValPheLysAlaLeuGlnGluAsn
GlyValLeuLeuGluGlyAlaLeuLeuLysProAsnMetValThrAlaGlyTyrGluCys
ThrAlaLysThrThrThrGlnAspValGlyPheLeuThrValArgThrLeuArgArgThr
ValProProAlaLeuProGlyValValPheLeuSerGlyGlyGlnSerGluGluGluAla
SerValAsnLeuAsnSerIleAsnAlaLeuGlyProHisProTrpAlaLeuThrPheSer
TyrGlyArgAlaLeuGlnAlaSerValLeuAsnThrTrpGlnGlyLysLysGluAsnVal
AlaLysAlaArgGluValLeuLeuGlnArgAlaGluAlaAsnSerLeuAlaThrTyrGly
LysTyrLysGlyGlyAlaGlyGlyGluAsnAlaGlyAlaSerLeuTyrGluLysLysTyr
ValTyr,

worin
-W- Gln ist oder fehlen kann;
-X- Met oder Gln ist;
-Y- Gly oder Cys ist und
-Z- Gly oder Cys ist,
übereinstimmen.

2. Polypeptide gemäss Anspruch 1, die in mindestens einem spezifischen Epitop mit dem Plasmodium falciparum Merozoitenantigen mit der Aminosäuresequenz

MetAsnAlaProLysLysLeuProAlaAspValAlaGluGluLeuAlaThrThrAlaLys
LeuValGlnAlaGlyLysGlyIleLeuAlaAlaAspGluSerThrGlnThrIleLysLys
ArgPheAspAsnIleLysLeuGluAsnThrIleGluAsnArgAlaSerTyrArgAspLeu
LeuPheGlyThrLysGlyLeuGlyLysPheIleSerGlyAlaIleLeuPheGluGluThr
LeuPheGlnLysAsnGluAlaGlyValProGlnValAsnLeuLeuHisAsnGluAsnIle
IleProGlyIleLysValAspLysCysLeuValAsnIleProCysThrAspGluGluLys
SerThrGlnGlyLeuAspGlyLeuAlaGluArgCysLysGluTyrTyrLysAlaGlyAla
ArgPheAlaLysTrpArgThrValLeuValIleAspThrAlaLysGlyLysProThrAsp
LeuSerAsnHisGluThrAlaTrpGlyLeuAlaArgTyrAlaSerIleCysGlnGlnAsn
ArgLeuValProIleValGluProGluIleLeuAlaAspGlyProHisSerIleGluVal
CysAlaValValThrGlnLysValLeuSerCysValPheLysAlaLeuGlnGluAsnGly
ValLeuLeuGluGlyAlaLeuLeuLysProAsnMetValThrAlaGlyTyrGluCysThr
AlaLysThrThrThrGlnAspValGlyPheLeuThrValArgThrLeuArgArgThrVal
ProProAlaLeuProGlyValValPheLeuSerGlyGlyGlnSerGluGluGluAlaSer
ValAsnLeuAsnSerIleAsnAlaLeuGlyProHisProTrpAlaLeuThrPheSerTyr
GlyArgAlaLeuGlnAlaSerValLeuAsnThrTrpGlnGlyLysLysGluAsnValAla
LysAlaArgGluValLeuLeuGlnArgAlaGluAlaAsnSerLeuAlaThrTyrGlyLys
TyrLysGlyGlyAlaGlyGlyGluAsnAlaGlyAlaSerLeuTyrGluLysLysTyrVal
Tyr

übereinstimmen.

3. Polypeptide gemäss Anspruch 1 oder 2, kovalent verknüpft mit einem Affinitätspeptid.

4. Polypeptide gemäss einem der Ansprüche 1 bis 3, mit der Aminosäuresequenz

MetArgGlySerHisHisHisHisHisHisGlySerGlyAsnIleProCysThrAspGlu
GluLysSerThrGlnGlyLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu

AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr.

5. Polypeptide gemäss einem der Ansprüche 1 bis 3, mit der Aminosäuresequenz

MetArgGlySerHisHisHisHisHisHisGlySerGlyAsnIleProCysThrAspGlu
GluLysSerThrGlnCysLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu
AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr.

6. Polypeptide gemäss einem der Ansprüche 1 bis 3, mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaLysLeuVal
GlnAlaGlyLysGlyIleLeuAlaAlaAspGluSerThrGlnThrIleLysLysArgPhe
AspAsnIleLysLeuGluAsnThrIleGluAsnArgAlaSerTyrArgAspLeuLeuPhe
GlyThrLysGlyLeuGlyLysPheIleSerGlyAlaIleLeuPheGluGluThrLeuPhe
GlnLysAsnGluAlaGlyValProGlnValAsnLeuLeuHisAsnGluAsnIleIlePro
GlyIleLysValAspLysCysLeuValAsnIleProCysThrAspGluGluLysSerThr
GlnGlyLeuAspGlyLeuAlaGluArgCysLysGluTyrTyrLysAlaGlyAlaArgPhe
AlaLysTrpArgThrValLeuValIleAspThrAlaLysGlyLysProThrAspLeuSer
AsnHisGluThrAlaTrpGlyLeuAlaArgTyrAlaSerIleCysGlnGlnAsnArgLeu

ValProIleValGluProGluIleLeuAlaAspGlyProHisSerIleGluValCysAla
ValValThrGlnLysValLeuSerCysValPheLysAlaLeuGlnGluAsnGlyValLeu
LeuGluGlyAlaLeuLeuLysProAsnMetValThrAlaGlyTyrGluCysThrAlaLys
ThrThrThrGlnAspValGlyPheLeuThrValArgThrLeuArgArgThrValProPro
AlaLeuProGlyValValPheLeuSerGlyGlyGlnSerGluGluGluAlaSerValAsn
LeuAsnSerIleAsnAlaLeuGlyProHisProTrpAlaLeuThrPheSerTyrGlyArg
AlaLeuGlnAlaSerValLeuAsnThrTrpGlnGlyLysLysGluAsnValAlaLysAla
ArgGluValLeuLeuGlnArgAlaGluAlaAsnSerLeuAlaThrTyrGlyLysTyrLys
GlyGlyAlaGlyGlyGluAsnAlaGlyAlaSerLeuTyrGluLysLysTyrValTyr.

7. Polypeptide gemäss einem der Ansprüche 1 bis 3, mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProGlnValAsnLeuLeuHisAsnGluAsnIleIle
ProGlyIleLysValAspLysGlyLeuValAsnIleProCysThrAspGluGluLysSer
ThrGlnGlyLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr.

8. Polypeptide gemäss einem der Ansprüche 1 bis 3, mit der Aminosäuresequenz

MetArgGlySerHisHisHisHisHisHisGlySerGluLeuAlaCysGlnTyrMetAsn AlaProLysLysLeuProAlaAspValAlaGluGluLeuAlaThrThrAlaGlnLysLeu ValGlnAlaGlyLysGlyIleLeuAlaAlaAspGluSerThrGlnThrIleLysLysArg PheAspAsnIleLysLeuGluAsnThrIleGluAsnArgAlaSerTyrArgAspLeuLeu PheGlyThrLysGlyLeuGlyLysPheIleSerGlyAlaIleLeuPheGluGluThrLeu PheGlnLysAsnGluAlaGlyValProMetValAsnLeuLeuHisAsnGluAsnIleIle ProGlyIleLysValAspLysGlyLeuValAsnIleProCysThrAspGluGluLysSer ThrGlnGlyLeuAspGlyLeuAlaGluArgCysLysGluTyrTyrLysAlaGlyAlaArg PheAlaLysTrpArgThrValLeuValIleAspThrAlaLysGlyLysProThrAspLeu SerAsnHisGluThrAlaTrpGlyLeuAlaArgTyrAlaSerIleCysGlnGlnAsnArg LeuValProIleValGluProGluIleLeuAlaAspGlyProHisSerIleGluValCys AlaValValThrGlnLysValLeuSerCysValPheLysAlaLeuGlnGluAsnGlyVal LeuLeuGluGlyAlaLeuLeuLysProAsnMetValThrAlaGlyTyrGluCysThrAla LysThrThrThrGlnAspValGlyPheLeuThrValArgThrLeuArgArgThrValPro ProAlaLeuProGlyValValPheLeuSerGlyGlyGlnSerGluGluAlaSerVal AsnLeuAsnSerIleAsnAlaLeuGlyProHisProTrpAlaLeuThrPheSerTyrGly ArgAlaLeuGlnAlaSerValLeuAsnThrTrpGlnGlyLysLysGluAsnValAlaLys AlaArgGluValLeuLeuGlnArgAlaGluAlaAsnSerLeuAlaThrTyrGlyLysTyr LysGlyGlyAlaGlyGlyGluAsnAlaGlyAlaSerLeuTyrGluLysLysTyrValTyr.

9. Polypeptide gemäss einem der Ansprüche 1 bis 3, mit der Aminosäuresequenz

MetArgGlySerHisHisHisHisHisHisGlySerGluLeuAlaCysGlnTyrMetAsn AlaProLysLysLeuProAlaAspValAlaGluGluLeuAlaThrThrAlaGlnLysLeu ValGlnAlaGlyLysGlyIleLeuAlaAlaAspGluSerThrGlnThrIleLysLysArg PheAspAsnIleLysLeuGluAsnThrIleGluAsnArgAlaSerTyrArgAspLeuLeu PheGlyThrLysGlyLeuGlyLysPheIleSerGlyAlaIleLeuPheGluGluThrLeu PheGlnLysAsnGluAlaGlyValProGlnValAsnLeuLeuHisAsnGluAsnIleIle ProGlyIleLysValAspLysGlyLeuValAsnIleProCysThrAspGluGluLysSer ThrGlnCysLeuAspGlyLeuAlaGluArgCysLysGluTyrTyrLysAlaGlyAlaArg PheAlaLysTrpArgThrValLeuValIleAspThrAlaLysGlyLysProThrAspLeu SerAsnHisGluThrAlaTrpGlyLeuAlaArgTyrAlaSerIleCysGlnGlnAsnArg LeuValProIleValGluProGluIleLeuAlaAspGlyProHisSerIleGluValCys AlaValValThrGlnLysValLeuSerCysValPheLysAlaLeuGlnGluAsnGlyVal LeuLeuGluGlyAlaLeuLeuLysProAsnMetValThrAlaGlyTyrGluCysThrAla LysThrThrThrGlnAspValGlyPheLeuThrValArgThrLeuArgArgThrValPro

ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr.

10. Polypeptide gemäss einem der Ansprüche 1 bis 3, mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProMetValAsnLeuLeuHisAsnGluAsnIleIle
ProGlyIleLysValAspLysGlyLeuValAsnIleProCysThrAspGluGluLysSer
ThrGlnCysLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr.

11. Polypeptide gemäss einem der Ansprüche 1 bis 10, die an ein Trägermaterial adsorbiert oder kovalent daran gebunden sind.

12. Eine DNA Sequenz, die für ein Polypeptid gemäss einem der Ansprüche 1 bis 10 kodiert.

13. Eine DNA Sequenz gemäss Anspruch 12, die die Nukleotidsequenz von Position 1 bis Position 1086 in Figur 12 oder Fragmente davon enthält.

14. Eine DNA Sequenz gemäss Anspruch 12, die die Nukleotidsequenz von Position 337 bis Position 1086 in Figur 12 oder Fragmente davon enthält.

15. Ein replizierbarer mikrobieller Vektor, der eine DNA-Sequenz gemäss einem der Ansprüche 12 bis 14 enthält.

16. Ein replizierbarer mikrobieller Vektor gemäss Anspruch 15, worin die DNA-Sequenz so an eine Expressionskontrollsequenz gebunden ist, dass das von der DNA-Sequenz kodierte Polypeptid exprimiert werden kann.

17. Ein Mikroorganismus der einen replizierbaren mikrobiellen Vektor gemäss einem der Ansprüche 15 oder 16 enthält.

18. Antikörper die gegen ein Polypeptid gemäss einem der Ansprüche 1 bis 11 gerichtet sind.

19. Polypeptide gemäss einem der Ansprüche 1 bis 11 für die Immunisierung von Säugern gegen Malaria.

20. Ein Verfahren zur Herstellung von Polypeptiden gemäss einem der Ansprüche 1 bis 10, dadurch gekennzeichnet, dass ein Mikroorganismus, der mit einem replizierbaren mikrobiellen Vektor gemäss Anspruch 16 transformiert worden ist, unter Bedingungen kultiviert wird, die die Expression des auf dem Vektor codierten Polypeptids erlauben.

21. Ein Verfahren zur Herstellung eines Mikroorganismus gemäss Anspruch 17, dadurch gekennzeichnet, dass ein Mikroorganismus mit einem replizierbaren mikrobiellen Vektor gemäss einem der Ansprüche 15 oder 16 transformiert wird.

22. Ein Verfahren zur Herstellung von Antikörpern gemäss Anspruch 18, dadurch gekennzeichnet, dass ein Polypeptid gemäss einem der Ansprüche 1 bis 11 in einen geeigneten Wirtsorganismus, der zu einer Immunreaktion gegen das Polypeptid fähig ist, injiziert wird und die sich bildenden Antikörper auf bekannte Art und Weise isoliert werden.

23. Immunogene Kompositionen die ein Polypeptid gemäss einem der Ansprüche 1 bis 11 und ein geeignetes Adjuvans enthalten.

24. Immunogene Kompositionen gemäss Anspruch 23, als ein Vakzin.

25. Verwendung eines Polypeptids gemäss einem der Ansprüche 1 bis 11 oder einer immunogenen Komposition gemäss einem der Ansprüche 23 oder 24 zur Immunisierung von Säugern gegen Malaria.

26. Eine Methode zur Immunisierung von Säugern gegen Malaria, dadurch charakterisiert, dass des Immunsystem dieser Säuger mit einer immunisierenden Menge eines Polypeptids gemäss einem der Ansprüche 1 bis 11 oder einer immunogenen Zusammensetzung gemäss einem der Ansprüche 23 oder 24 stimuliert wird.

Patentansprüche für folgenden Vertragsstaat: ES

1. Ein Verfahren zur Herstellung eines Polypeptids, das in mindestens einem spezifischen Epitop mit dem Plasmodium falciparum Merozoitenantigen mit der Aminosäuresequenz

MetAsnAlaProLysLysLeuProAlaAspValAlaGluGluLeuAlaThrThrAla-W-
LysLeuValGlnAlaGlyLysGlyIleLeuAlaAlaAspGluSerThrGlnThrIleLys
LysArgPheAspAsnIleLysLeuGluAsnThrIleGluAsnArgAlaSerTyrArgAsp
LeuLeuPheGlyThrLysGlyLeuGlyLysPheIleSerGlyAlaIleLeuPheGluGlu
ThrLeuPheGlnLysAsnGluAlaGlyValPro-X-ValAsnLeuLeuHisAsnGluAsn
IleIleProGlyIleLysValAspLys-Y-LeuValAsnIleProCysThrAspGluGlu
LysSerThrGln-Z-LeuAspGlyLeuAlaGluArgCysLysGluTyrTyrLysAlaGly
AlaArgPheAlaLysTrpArgThrValLeuValIleAspThrAlaLysGlyLysProThr
AspLeuSerAsnHisGluThrAlaTrpGlyLeuAlaArgTyrAlaSerIleCysGlnGln
AsnArgLeuValProIleValGluProGluIleLeuAlaAspGlyProHisSerIleGlu
ValCysAlaValValThrGlnLysValLeuSerCysValPheLysAlaLeuGlnGluAsn
GlyValLeuLeuGluGlyAlaLeuLeuLysProAsnMetValThrAlaGlyTyrGluCys
ThrAlaLysThrThrThrGlnAspValGlyPheLeuThrValArgThrLeuArgArgThr
ValProProAlaLeuProGlyValValPheLeuSerGlyGlyGlnSerGluGluGluAla
SerValAsnLeuAsnSerIleAsnAlaLeuGlyProHisProTrpAlaLeuThrPheSer
TyrGlyArgAlaLeuGlnAlaSerValLeuAsnThrTrpGlnGlyLysLysGluAsnVal
AlaLysAlaArgGluValLeuLeuGlnArgAlaGluAlaAsnSerLeuAlaThrTyrGly
LysTyrLysGlyGlyAlaGlyGlyGluAsnAlaGlyAlaSerLeuTyrGluLysLysTyr
ValTyr,

worin
-W- Gln ist oder fehlen kann;
-X- Met oder Gln ist;
-Y- Gly oder Cys ist und
-Z- Gly oder Cys ist,
übereinstimmt, dadurch gekennzeichnet, dass eine Kultur eines Mikroorganismus, der mit einem replizierbaren, mikrobiellen Vektor transformiert und dadurch fähig ist, das oben genannte Polypeptid zu exprimieren, wachsen gelassen wird und das oben genannte Polypeptid exprimiert und gewonnen wird.

2. Ein Verfahren zur Herstellung eines Polypeptids gemäss Anspruch 1, worin das Polypeptid in mindestens einem spezifischen Epitop mit dem Plasmodium falciparum Merozoitenantigen mit der Aminosäuresequenz

MetAsnAlaProLysLysLeuProAlaAspValAlaGluGluLeuAlaThrThrAlaLys
LeuValGlnAlaGlyLysGlyIleLeuAlaAlaAspGluSerThrGlnThrIleLysLys
ArgPheAspAsnIleLysLeuGluAsnThrIleGluAsnArgAlaSerTyrArgAspLeu
LeuPheGlyThrLysGlyLeuGlyLysPheIleSerGlyAlaIleLeuPheGluGluThr
LeuPheGlnLysAsnGluAlaGlyValProGlnValAsnLeuLeuHisAsnGluAsnIle
IleProGlyIleLysValAspLysCysLeuValAsnIleProCysThrAspGluGluLys
SerThrGlnGlyLeuAspGlyLeuAlaGluArgCysLysGluTyrTyrLysAlaGlyAla
ArgPheAlaLysTrpArgThrValLeuValIleAspThrAlaLysGlyLysProThrAsp
LeuSerAsnHisGluThrAlaTrpGlyLeuAlaArgTyrAlaSerIleCysGlnGlnAsn
ArgLeuValProIleValGluProGluIleLeuAlaAspGlyProHisSerIleGluVal
CysAlaValValThrGlnLysValLeuSerCysValPheLysAlaLeuGlnGluAsnGly
ValLeuLeuGluGlyAlaLeuLeuLysProAsnMetValThrAlaGlyTyrGluCysThr
AlaLysThrThrThrGlnAspValGlyPheLeuThrValArgThrLeuArgArgThrVal
ProProAlaLeuProGlyValValPheLeuSerGlyGlyGlnSerGluGluAlaSer
ValAsnLeuAsnSerIleAsnAlaLeuGlyProHisProTrpAlaLeuThrPheSerTyr
GlyArgAlaLeuGlnAlaSerValLeuAsnThrTrpGlnGlyLysLysGluAsnValAla
LysAlaArgGluValLeuLeuGlnArgAlaGluAlaAsnSerLeuAlaThrTyrGlyLys
TyrLysGlyGlyAlaGlyGlyGluAsnAlaGlyAlaSerLeuTyrGluLysLysTyrVal
Tyr

übereinstimmt, dadurch gekennzeichnet, dass eine Kultur eines Mikroorganismus, der mit einem replizierbaren, mikrobiellen Vektor transformiert und dadurch fähig ist, das oben genannte Polypeptid zu exprimieren, wachsen gelassen wird und das oben genannte Polypeptid exprimiert und gewonnen wird.

3. Ein Verfahren gemäss Anspruch 1 oder 2, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid, das kovalent mit einem Affinitätspeptid verknüpft ist, zu exprimieren, verwendet wird.

4. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGlyAsnIleProCysThrAspGlu
GluLysSerThrGlnGlyLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu
AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr

zu exprimieren, verwendet wird.

5. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGlyAsnIleProCysThrAspGlu
GluLysSerThrGlnCysLeuAspGlyLeuAlaGluArgCysLysGluTyrTyrLysAla
GlyAlaArgPheAlaLysTrpArgThrValLeuValIleAspThrAlaLysGlyLysPro
ThrAspLeuSerAsnHisGluThrAlaTrpGlyLeuAlaArgTyrAlaSerIleCysGln
GlnAsnArgLeuValProIleValGluProGluIleLeuAlaAspGlyProHisSerIle
GluValCysAlaValValThrGlnLysValLeuSerCysValPheLysAlaLeuGlnGlu
AsnGlyValLeuLeuGluGlyAlaLeuLeuLysProAsnMetValThrAlaGlyTyrGlu
CysThrAlaLysThrThrThrGlnAspValGlyPheLeuThrValArgThrLeuArgArg
ThrValProProAlaLeuProGlyValValPheLeuSerGlyGlyGlnSerGluGluGlu
AlaSerValAsnLeuAsnSerIleAsnAlaLeuGlyProHisProTrpAlaLeuThrPhe
SerTyrGlyArgAlaLeuGlnAlaSerValLeuAsnThrTrpGlnGlyLysLysGluAsn
ValAlaLysAlaArgGluValLeuLeuGlnArgAlaGluAlaAsnSerLeuAlaThrTyr
GlyLysTyrLysGlyGlyAlaGlyGlyGluAsnAlaGlyAlaSerLeuTyrGluLysLys
TyrValTyr

zu exprimieren, verwendet wird.

6. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaLysLeuVal
GlnAlaGlyLysGlyIleLeuAlaAlaAspGluSerThrGlnThrIleLysLysArgPhe
AspAsnIleLysLeuGluAsnThrIleGluAsnArgAlaSerTyrArgAspLeuLeuPhe
GlyThrLysGlyLeuGlyLysPheIleSerGlyAlaIleLeuPheGluGluThrLeuPhe
GlnLysAsnGluAlaGlyValProGlnValAsnLeuLeuHisAsnGluAsnIleIlePro
GlyIleLysValAspLysCysLeuValAsnIleProCysThrAspGluGluLysSerThr
GlnGlyLeuAspGlyLeuAlaGluArgCysLysGluTyrTyrLysAlaGlyAlaArgPhe
AlaLysTrpArgThrValLeuValIleAspThrAlaLysGlyLysProThrAspLeuSer
AsnHisGluThrAlaTrpGlyLeuAlaArgTyrAlaSerIleCysGlnGlnAsnArgLeu
ValProIleValGluProGluIleLeuAlaAspGlyProHisSerIleGluValCysAla
ValValThrGlnLysValLeuSerCysValPheLysAlaLeuGlnGluAsnGlyValLeu
LeuGluGlyAlaLeuLeuLysProAsnMetValThrAlaGlyTyrGluCysThrAlaLys
ThrThrThrGlnAspValGlyPheLeuThrValArgThrLeuArgArgThrValProPro
AlaLeuProGlyValValPheLeuSerGlyGlyGlnSerGluGluAlaSerValAsn
LeuAsnSerIleAsnAlaLeuGlyProHisProTrpAlaLeuThrPheSerTyrGlyArg
AlaLeuGlnAlaSerValLeuAsnThrTrpGlnGlyLysLysGluAsnValAlaLysAla
ArgGluValLeuLeuGlnArgAlaGluAlaAsnSerLeuAlaThrTyrGlyLysTyrLys
GlyGlyAlaGlyGlyGluAsnAlaGlyAlaSerLeuTyrGluLysLysTyrValTyr

zu exprimieren, verwendet wird.

7. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProGlnValAsnLeuLeuHisAsnGluAsnIleIle
ProGlyIleLysValAspLysGlyLeuValAsnIleProCysThrAspGluGluLysSer
ThrGlnGlyLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr

zu exprimieren, verwendet wird.

8. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProMetValAsnLeuLeuHisAsnGluAsnIleIle
ProGlyIleLysValAspLysGlyLeuValAsnIleProCysThrAspGluGluLysSer
ThrGlnGlyLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr

zu exprimieren, verwendet wird.

9. Ein Verfahren gemäss einem der Ansprüche 1 bis 3, dadurch gekennzeichnet, dass eine Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProGlnValAsnLeuLeuHisAsnGluAsnIleIle
ProGlyIleLysValAspLysGlyLeuValAsnIleProCysThrAspGluGluLysSer

ThrGlnCysLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr

zu exprimieren, verwendet wird.

10. Ein Verfahren gemäss einem der Ansprüche 1 bis 3, dadurch gekennzeichnet, dass ein Kultur eines Mikroorganismus, der mit einem replizierbaren, mikrobiellen Vektor transformiert und dadurch fähig ist, ein in Anspruch 1 definiertes Polypeptid mit der Aminosäuresequenz

MetArgGlySerHisHisHisHisHisHisGlySerGluLeuAlaCysGlnTyrMetAsn
AlaProLysLysLeuProAlaAspValAlaGluGluLeuAlaThrThrAlaGlnLysLeu
ValGlnAlaGlyLysGlyIleLeuAlaAlaAspGluSerThrGlnThrIleLysLysArg
PheAspAsnIleLysLeuGluAsnThrIleGluAsnArgAlaSerTyrArgAspLeuLeu
PheGlyThrLysGlyLeuGlyLysPheIleSerGlyAlaIleLeuPheGluGluThrLeu
PheGlnLysAsnGluAlaGlyValProMetValAsnLeuLeuHisAsnGluAsnIleIle
ProGlyIleLysValAspLysGlyLeuValAsnIleProCysThrAspGluGluLysSer
ThrGlnCysLeuAspGlyLeuAlaGluArgCysLysGluTyrTyrLysAlaGlyAlaArg
PheAlaLysTrpArgThrValLeuValIleAspThrAlaLysGlyLysProThrAspLeu
SerAsnHisGluThrAlaTrpGlyLeuAlaArgTyrAlaSerIleCysGlnGlnAsnArg
LeuValProIleValGluProGluIleLeuAlaAspGlyProHisSerIleGluValCys
AlaValValThrGlnLysValLeuSerCysValPheLysAlaLeuGlnGluAsnGlyVal
LeuLeuGluGlyAlaLeuLeuLysProAsnMetValThrAlaGlyTyrGluCysThrAla
LysThrThrThrGlnAspValGlyPheLeuThrValArgThrLeuArgArgThrValPro
ProAlaLeuProGlyValValPheLeuSerGlyGlyGlnSerGluGluGluAlaSerVal
AsnLeuAsnSerIleAsnAlaLeuGlyProHisProTrpAlaLeuThrPheSerTyrGly
ArgAlaLeuGlnAlaSerValLeuAsnThrTrpGlnGlyLysLysGluAsnValAlaLys
AlaArgGluValLeuLeuGlnArgAlaGluAlaAsnSerLeuAlaThrTyrGlyLysTyr
LysGlyGlyAlaGlyGlyGluAsnAlaGlyAlaSerLeuTyrGluLysLysTyrValTyr

zu exprimieren, verwendet wird.

11. Ein Verfahren zu Herstellung eines Mikroorganismus, der fähig ist ein Polypeptid, wie es in einem der Ansprüche 1 bis 10 definiert ist, zu exprimieren, dadurch gekennzeichnet, dass der Mikroorganismus auf bekannte Art und Weise mit einem replizierbaren, mikrobiellen Vektor transformiert wird, worin die das Polypeptid kodierende Nukleotidsequenz operativ an eine Expressionskontrollsequenz gebunden ist.

12. Ein Verfahren zur Herstellung von Antikörpern, die gegen ein Polypeptid, wie es in einem der Ansprüche 1 bis 10 definiert ist, gerichtet sind, dadurch gekennzeichnet, dass das obengenannte Polypeptid in einen geeigneten Wirt, der zu einer Immunantwort gegen das Polypeptid befähigt ist, auf bekannte Art und Weise injiziert wird und die gebildeten Antikörper, mittels bekannter Methoden, isoliert werden.

13. Verwendung eines Polypeptids, wie es in einem der Ansprüche 1 bis 10 definiert ist, für die Herstellung eines Vakzins.

Fig. 1

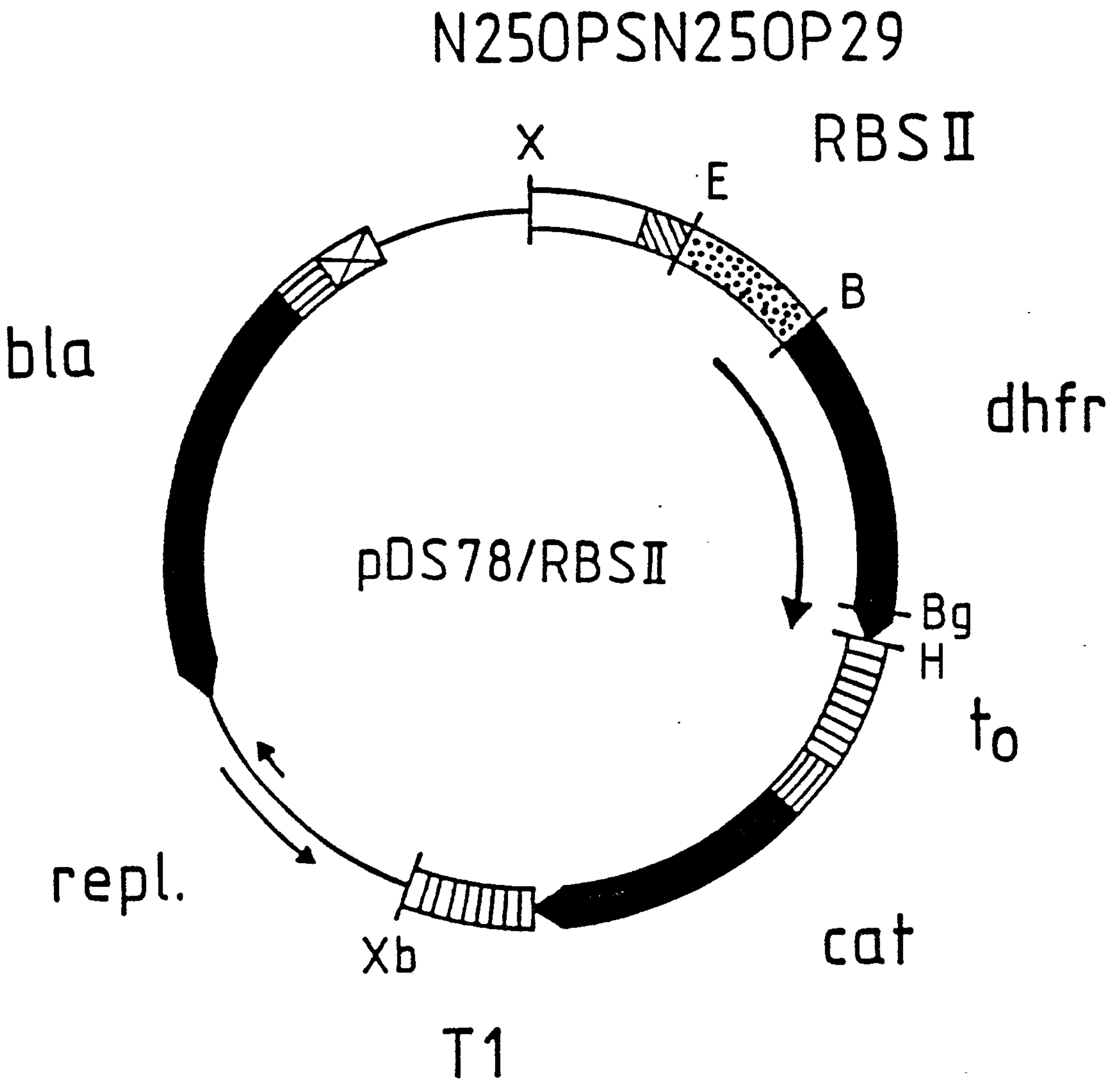
N25OPSN25OP29
X
RBSII
E
B
bla
dhfr
pDS78/RBSII
Bg
H
to
repl.
Xb
cat
T1

# Fig. 2a

```
              10         20         30         40         50
               |          |          |          |          |
   1 CTCGAGAAAT CATAAAAAAT TTATTTGCTT TGTGAGCGGA TAACAATTAT
  51 AATAGATTCA ATTGTGAGCG GATAACAATT TCACACAGAA TTCATTAAAG
 101 AGGAGAAATT AACTATGAGA GGATCCGGCA TCATGGTTCG ACCATTGAAC
 151 TGCATCGTCG CCGTGTCCCA AAATATGGGG ATTGGCAAGA ACGGAGACCT
 201 ACCCTGGCCT CCGCTCAGGA ACGAGTTCAA GTACTTCCAA AGAATGACCA
 251 CAACCTCTTC AGTGGAAGGT AAACAGAATC TGGTGATTAT GGGTAGGAAA
 301 ACCTGGTTCT CCATTCCTGA GAAGAATCGA CCTTTAAAGG ACAGAATTAA
 351 TATAGTTCTC AGTAGAGAAC TCAAAGAACC ACCACGAGGA GCTCATTTTC
 401 TTGCCAAAAG TTTGGATGAT GCCTTAAGAC TTATTGAACA ACCGGAATTG
 451 GCAAGTAAAG TAGACATGGT TTGGATAGTC GGAGGCAGTT CTGTTTACCA
 501 GGAAGCCATG AATCAACCAG GCCACCTTAG ACTCTTTGTG ACAAGGATCA
 551 TGCAGGAATT TGAAAGTGAC ACGTTTTTCC CAGAAATTGA TTTGGGGAAA
 601 TATAAACTTC TCCCAGAATA CCCAGGCGTC CTCTCTGAGG TCCAGGAGGA
 651 AAAAGGCATC AAGTATAAGT TTGAAGTCTA CGAGAAGAAA GGTTCCAGAT
 701 CTGTTAACCT AGTTTAACAG GAAGATGCTT TCAAGTTCTC TGCTCCCCTC
 751 CTAAAGCTAT GCATTTTTAT AAGACCATGG GACTTTTGCT GGCTTTAGAT
 801 CCGGCCAAGC TTGGACTCCT GTTGATAGAT CCAGTAATGA CCTCAGAACT
 851 CCATCTGGAT TTGTTCAGAA CGCTCGGTTG CCGCCGGGCG TTTTTTATTG
 901 GTGAGAATCC AAGCTAGCTT GGCGAGATTT TCAGGAGCTA AGGAAGCTAA
 951 AATGGAGAAA AAAATCACTG GATATACCAC CGTTGATATA TCCCAATGGC
1001 ATCGTAAAGA ACATTTTGAG GCATTTCAGT CAGTTGCTCA ATGTACCTAT
1051 AACCAGACCG TTCAGCTGGA TATTACGGCC TTTTTAAAGA CCGTAAAGAA
1101 AAATAAGCAC AAGTTTTATC CGGCCTTTAT TCACATTCTT GCCCGCCTGA
1151 TGAATGCTCA TCCGGAATTT CGTATGGCAA TGAAAGACGG TGAGCTGGTG
```

# Fig. 2b

1201 ATATGGGATA GTGTTCACCC TTGTTACACC GTTTTCCATG AGCAAACTGA
1251 AACGTTTTCA TCGCTCTGGA GTGAATACCA CGACGATTTC CGGCAGTTTC
1301 TACACATATA TTCGCAAGAT GTGGCGTGTT ACGGTGAAAA CCTGGCCTAT
1351 TTCCCTAAAG GGTTTATTGA GAATATGTTT TTCGTCTCAG CCAATCCCTG
1401 GGTGAGTTTC ACCAGTTTTG ATTTAAACGT GGCCAATATG GACAACTTCT
1451 TCGCCCCCGT TTTCACCATG GGCAAATATT ATACGCAAGG CGACAAGGTG
1501 CTGATGCCGC TGGCGATTCA GGTTCATCAT GCCGTCTGTG ATGGCTTCCA
1551 TGTCGGCAGA ATGCTTAATG AATTACAACA GTACTGCGAT GAGTGGCAGG
1601 GCGGGGCGTA ATTTTTTTAA GGCAGTTATT GGTGCCCTTA AACGCCTGGG
1651 GTAATGACTC TCTAGCTTGA GGCATCAAAT AAAACGAAAG GCTCAGTCGA
1701 AAGACTGGGC CTTTCGTTTT ATCTGTTGTT TGTCGGTGAA CGCTCTCCTG
1751 AGTAGGACAA ATCCGCCGCT CTAGAGCTGC CTCGCGCGTT TCGGTGATGA
1801 CGGTGAAAAC CTCTGACACA TGCAGCTCCC GGAGACGGTC ACAGCTTGTC
1851 TGTAAGCGGA TGCCGGGAGC AGACAAGCCC GTCAGGGCGC GTCAGCGGGT
1901 GTTGGCGGGT GTCGGGGCGC AGCCATGACC CAGTCACGTA GCGATAGCGG
1951 AGTGTATACT GGCTTAACTA TGCGGCATCA GAGCAGATTG TACTGAGAGT
2001 GCACCATATG CGGTGTGAAA TACCGCACAG ATGCGTAAGG AGAAAATACC
2051 GCATCAGGCG CTCTTCCGCT TCCTCGCTCA CTGACTCGCT GCGCTCGGTC
2101 TGTCGGCTGC GGCGAGCGGT ATCAGCTCAC TCAAAGGCGG TAATACGGTT
2151 ATCCACAGAA TCAGGGGATA ACGCAGGAAA GAACATGTGA GCAAAAGGCC
2201 AGCAAAAGGC CAGGAACCGT AAAAAGGCCG CGTTGCTGGC GTTTTTCCAT
2251 AGGCTCCGCC CCCCTGACGA GCATCACAAA AATCGACGCT CAAGTCAGAG
2301 GTGGCGAAAC CCGACAGGAC TATAAAGATA CCAGGCGTTT CCCCCTGGAA
2351 GCTCCCTCGT GCGCTCTCCT GTTCCGACCC TGCCGCTTAC CGGATACCTG
2401 TCCGCCTTTC TCCCTTCGGG AAGCGTGGCG CTTTCTCAAT GCTCACGCTG
2451 TAGGTATCTC AGTTCGGTGT AGGTCGTTCG CTCCAAGCTG GGCTGTGTGC
2501 ACGAACCCCC CGTTCAGCCC GACCGCTGCG CCTTATCCGG TAACTATCGT

# Fig. 2c

2551 CTTGAGTCCA ACCCGGTAAG ACACGACTTA TCGCCACTGG CAGCAGCCAC
2601 TGGTAACAGG ATTAGCAGAG CGAGGTATGT AGGCGGTGCT ACAGAGTTCT
2651 TGAAGTGGTG GCCTAACTAC GGCTACACTA GAAGGACAGT ATTTGGTATC
2701 TGCGCTCTGC TGAAGCCAGT TACCTTCGGA AAAAGAGTTG GTAGCTCTTG
2751 ATCCGGCAAA CAAACCACCG CTGGTAGCGG TGGTTTTTTT GTTTGCAAGC
2801 AGCAGATTAC GCGCAGAAAA AAAGGATCTC AAGAAGATCC TTTGATCTTT
2851 TCTACGGGGT CTGACGCTCA GTGGAACGAA AACTCACGTT AAGGGATTTT
2901 GGTCATGAGA TTATCAAAAA GGATCTTCAC CTAGATCCTT TTAAATTAAA
2951 AATGAAGTTT TAAATCAATC TAAAGTATAT ATGAGTAAAC TTGGTCTGAC
3001 AGTTACCAAT GCTTAATCAG TGAGGCACCT ATCTCAGCGA TCTGTCTATT
3051 TCGTTCATCC ATAGCTGCCT GACTCCCCGT CGTGTAGATA ACTACGATAC
3101 GGGAGGGCTT ACCATCTGGC CCCAGTGCTG CAATGATACC GCGAGACCCA
3151 CGCTCACCGG CTCCAGATTT ATCAGCAATA AACCAGCCAG CCGGAAGGGC
3201 CGAGCGCAGA AGTGGTCCTG CAACTTTATC CGCCTCCATC CAGTCTATTA
3251 ATTGTTGCCG GGAAGCTAGA GTAAGTAGTT CGCCAGTTAA TAGTTTGCGC
3301 AACGTTGTTG CCATTGCTAC AGGCATCGTG GTGTCACGCT CGTCGTTTGG
3351 TATGGCTTCA TTCAGCTCCG GTTCCCAACG ATCAAGGCGA GTTACATGAT
3401 CCCCCATGTT GTGCAAAAAA GCGGTTAGCT CCTTCGGTCC TCCGATCGTT
3451 GTCAGAAGTA AGTTGGCCGC AGTGTTATCA CTCATGGTTA TGGCAGCACT
3501 GCATAATTCT CTTACTGTCA TGCCATCCGT AAGATGCTTT TCTGTGACTG
3551 GTGAGTACTC AACCAAGTCA TTCTGAGAAT AGTGTATGCG GCGACCGAGT
3601 TGCTCTTGCC CGGCGTCAAT ACGGGATAAT ACCGCGCCAC ATAGCAGAAC
3651 TTTAAAAGTG CTCATCATTG GAAAACGTTC TTCGGGGCGA AAACTCTCAA
3701 GGATCTTACC GCTGTTGAGA TCCAGTTCGA TGTAACCCAC TCGTGCACCC
3751 AACTGATCTT CAGCATCTTT TACTTTCACC AGCGTTTCTG GGTGAGCAAA
3801 AACAGGAAGG CAAAATGCCG CAAAAAAGGG AATAAGGGCG ACACGGAAAT
3851 GTTGAATACT CATACTCTTC CTTTTTCAAT ATTATTGAAG CATTTATCAG
3901 GGTTATTGTC TCATGAGCGG ATACATATTT GAATGTATTT AGAAAAATAA

# Fig. 2d

3951 ACAAATAGGG GTTCCGCGCA CATTTCCCCG AAAAGTGCCA CCTGACGTCT

4001 AAGAAACCAT TATTATCATG ACATTAACCT ATAAAAATAG GCGTATCACG

4051 AGGCCCTTTC GTCTTCAC

Fig. 3

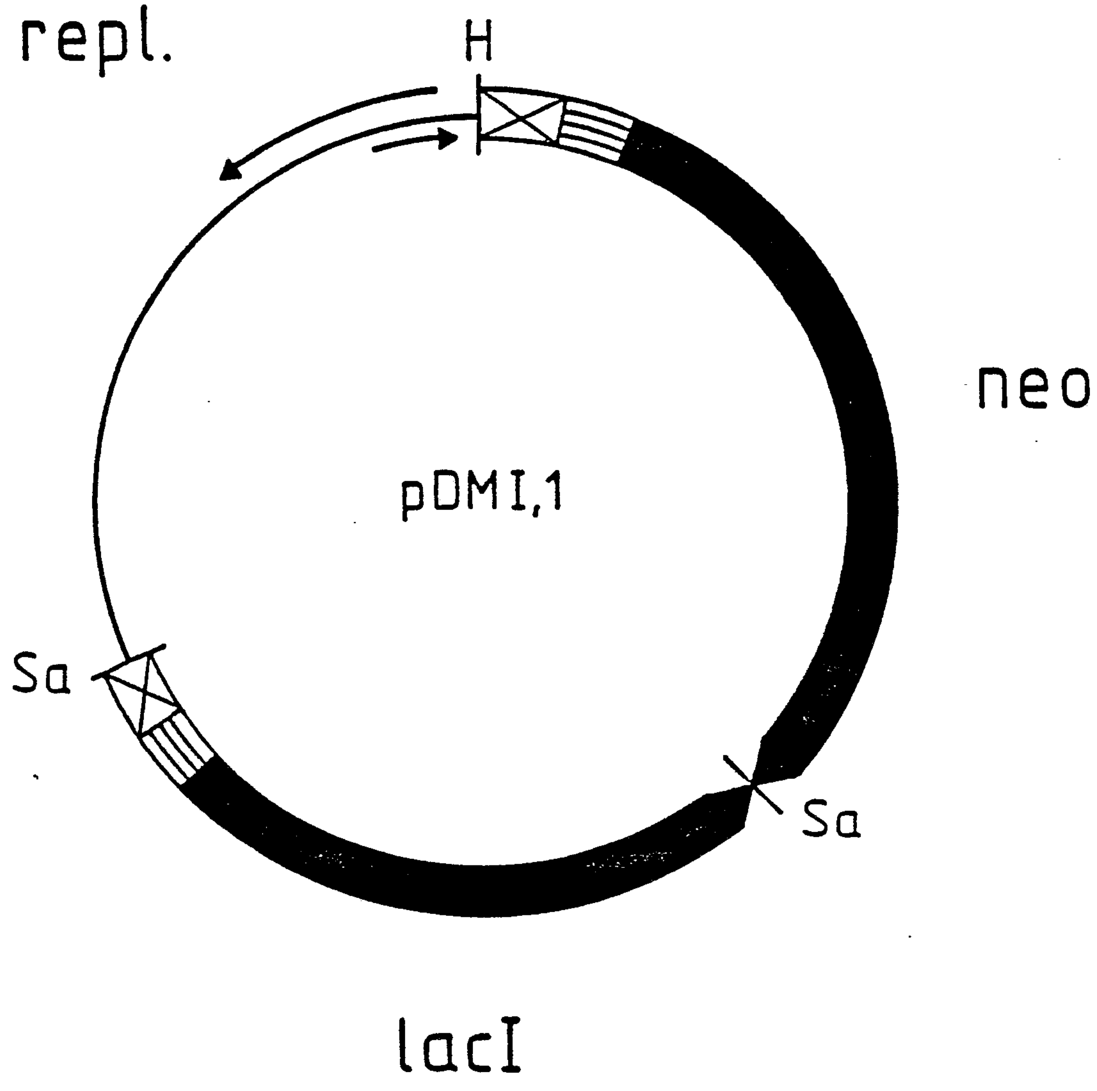

# Fig. 4a

```
                 10         20         30         40         50
                  |          |          |          |          |
   1 AAGCTTCACG CTGCCGCAAG CACTCAGGGC GCAAGGGCTG CTAAAGGAAG
  51 CGGAACACGT AGAAAGCCAG TCCGCAGAAA CGGTGCTGAC CCCGGATGAA
 101 TGTCAGCTAC TGGGCTATCT GGACAAGGGA AAACGCAAGC GCAAAGAGAA
 151 AGCAGGTAGC TTGCAGTGGG CTTACATGGC GATAGCTAGA CTGGGCGGTT
 201 TTATGGACAG CAAGCGAACC GGAATTGCCA GCTGGGGCGC CCTCTGGTAA
 251 GGTTGGGAAG CCCTGCAAAG TAAACTGGAT GGCTTTCTTG CCGCCAAGGA
 301 TCTGATGGCG CAGGGGATCA AGATCTGATC AAGAGACAGG ATGAGGATCG
 351 TTTCGCATGA TTGAACAAGA TGGATTGCAC GCAGGTTCTC CGGCCGCTTG
 401 GGTGGAGAGG CTATTCGGCT ATGACTGGGC ACAACAGACA ATCGGCTGCT
 451 CTGATGCCGC CGTGTTCCGG CTGTCAGCGC AGGGGCGCCC GGTTCTTTTT
 501 GTCAAGACCG ACCTGTCCGG TGCCCTGAAT GAACTGCAGG ACGAGGCAGC
 551 GCGGCTATCG TGGCTGGCCA CGACGGGCGT TCCTTGCGCA GCTGTGCTCG
 601 ACGTTGTCAC TGAAGCGGGA AGGGACTGGC TGCTATTGGG CGAAGTGCCG
 651 GGGCAGGATC TCCTGTCATC TCACCTTGCT CCTGCCGAGA AAGTATCCAT
 701 CATGGCTGAT GCAATGCGGC GGCTGCATAC GCTTGATCCG GCTACCTGCC
 751 CATTCGACCA CCAAGCGAAA CATCGCATCG AGCGAGCACG TACTCGGATG
 801 GAAGCCGGTC TTGTCGATCA GGATGATCTG GACGAAGAGC ATCAGGGGCT
 851 CGCGCCAGCC GAACTGTTCG CCAGGCTCAA GGCGCGCATG CCCGACGGCG
 901 AGGATCTCGT CGTGACCCAT GGCGATGCCT GCTTGCCGAA TATCATGGTG
 951 GAAAATGGCC GCTTTTCTGG ATTCATCGAC TGTGGCCGGC TGGGTGTGGC
1001 GGACCGCTAT CAGGACATAG CGTTGGCTAC CCGTGATATT GCTGAAGAGC
1051 TTGGCGGCGA ATGGGCTGAC CGCTTCCTCG TGCTTTACGG TATCGCCGCT
1101 CCCGATTCGC AGCGCATCGC CTTCTATCGC CTTCTTGACG AGTTCTTCTG
1151 AGCGGGACTC TGGGGTTCGA AATGACCGAC CAAGCGACGC CCAACCTGCC
```

# Fig.4b

1201 ATCACGAGAT TTCGATTCCA CCGCCGCCTT CTATGAAAGG TTGGGCTTCG
1251 GAATCGTTTT CCGGGACGCC GGCTGGATGA TCCTCCAGCG CGGGGATCTC
1301 ATGCTGGAGT TCTTCGCCCA CCCCGGGCTC GATCCCCTCG CGAGTTGGTT
1351 CAGCTGCTGC CTGAGGCTGG ACGACCTCGC GGAGTTCTAC CGGCAGTGCA
1401 AATCCGTCGG CATCCAGGAA ACCAGCAGCG GCTATCCGCG CATCCATGCC
1451 CCCGAACTGC AGGAGTGGGG AGGCACGATG GCCGCTTTGG TCGACAATTC
1501 GCGCTAACTT ACATTAATTG CGTTGCGCTC ACTGCCCGCT TTCCAGTCGG
1551 GAAACCTGTC GTGCCAGCTG CATTAATGAA TCGGCCAACG CGCGGGGAGA
1601 GGCGGTTTGC GTATTGGGCG CCAGGGTGGT TTTTCTTTTC ACCAGTGAGA
1651 CGGGCAACAG CTGATTGCCC TTCACCGCCT GGCCCTGAGA GAGTTGCAGC
1701 AAGCGGTCCA CGCTGGTTTG CCCCAGCAGG CGAAAATCCT GTTTGATGGT
1751 GGTTAACGGC GGGATATAAC ATGAGCTGTC TTCGGTATCG TCGTATCCCA
1801 CTACCGAGAT ATCCGCACCA ACGCGCAGCC CGGACTCGGT AATGGCGCGC
1851 ATTGCGCCCA GCGCCATCTG ATCGTTGGCA ACCAGCATCG CAGTGGGAAC
1901 GATGCCCTCA TTCAGCATTT GCATGGTTTG TTGAAAACCG GACATGGCAC
1951 TCCAGTCGCC TTCCCGTTCC GCTATCGGCT GAATTTGATT GCGAGTGAGA
2001 TATTTATGCC AGCCAGCCAG ACGCAGACGC GCCGAGACAG AACTTAATGG
2051 GCCCGCTAAC AGCGCGATTT GCTGGTGACC CAATGCGACC AGATGCTCCA
2101 CGCCCAGTCG CGTACCGTCT TCATGGGAGA AAATAATACT GTTGATGGGT
2151 GTCTGGTCAG AGACATCAAG AAATAACGCC GGAACATTAG TGCAGGCAGC
2201 TTCCACAGCA ATGGCATCCT GGTCATCCAG CGGATAGTTA ATGATCAGCC
2251 CACTGACGCG TTGCGCGAGA AGATTGTGCA CCGCCGCTTT ACAGGCTTCG
2301 ACGCCGCTTC GTTCTACCAT CGACACCACC ACGCTGGCAC CCAGTTGATC
2351 GGCGCGAGAT TTAATCGCCG CGACAATTTG CGACGGCGCG TGCAGGGCCA
2401 GACTGGAGGT GGCAACGCCA ATCAGCAACG ACTGTTTGCC CGCCAGTTGT
2451 TGTGCCACGC GGTTGGGAAT GTAATTCAGC TCCGCCATCG CCGCTTCCAC
2501 TTTTTCCCGC GTTTTCGCAG AAACGTGGCT GGCCTGGTTC ACCACGCGGG

# Fig. 4c

```
2551 AAACGGTCTG ATAAGAGACA CCGGCATACT CTGCGACATC GTATAACGTT
2601 ACTGGTTTCA CATTCACCAC CCTGAATTGA CTCTCTTCCG GGCGCTATCA
2651 TGCCATACCG CGAAAGGTTT TGCACCATTC GATGGTGTCA ACGTAAATGC
2701 ATGCCGCTTC GCCTTCGCGC GCGAATTGTC GACCCTGTCC CTCCTGTTCA
2751 GCTACTGACG GGGTGGTGCG TAACGGCAAA AGCACCGCCG GACATCAGCG
2801 CTAGCGGAGT GTATACTGGC TTACTATGTT GGCACTGATG AGGGTGTCAG
2851 TGAAGTGCTT CATGTGGCAG GAGAAAAAAG GCTGCACCGG TGCGTCAGCA
2901 GAATATGTGA TACAGGATAT ATTCCGCTTC CTCGCTCACT GACTCGCTAC
2951 GCTCGGTCGT TCGACTGCGG CGAGCGGAAA TGGCTTACGA ACGGGGCGGA
3001 GATTTCCTGG AAGATGCCAG GAAGATACTT AACAGGGAAG TGAGAGGGCC
3051 GCGGCAAAGC CGTTTTTCCA TAGGCTCCGC CCCCCTGACA AGCATCACGA
3101 AATCTGACGC TCAAATCAGT GGTGGCGAAA CCCGACAGGA CTATAAAGAT
3151 ACCAGGCGTT TCCCCTGGCG GCTCCCTCGT GCGCTCTCCT GTTCCTGCCT
3201 TTCGGTTTAC CGGTGTCATT CCGCTGTTAT GGCCGCGTTT GTCTCATTCC
3251 ACGCCTGACA CTCAGTTCCG GGTAGGCAGT TCGCTCCAAG CTGGACTGTA
3301 TGCACGAACC CCCCGTTCAG TCCGACCGCT GCGCCTTATC CGGTAACTAT
3351 CGTCTTGAGT CCAACCCGGA AAGACATGCA AAAGCACCAC TGGCAGCAGC
3401 CACTGGTAAT TGATTTAGAG GAGTTAGTCT TGAAGTCATG CGCCGGTTAA
3451 GGCTAAACTG AAAGGACAAG TTTTGGTGAC TGCGCTCCTC CAAGCCAGTT
3501 ACCTCGGTTC AAAGAGTTGG TAGCTCAGAG AACCTTCGAA AAACCGCCCT
3551 GCAAGGCGGT TTTTTCGTTT TCAGAGCAAG AGATTACGCG CAGACCAAAA
3601 CGATCTCAAG AAGATCATCT TATTAATCAG ATAAAATATT TCTAGATTTC
3651 AGTGCAATTT ATCTCTTCAA ATGTAGCACC TGAAGTCAGC CCCATACGAT
3701 ATAAGTTGTT AATTCTCATG TTTGACAGCT TATCATCGAT
```

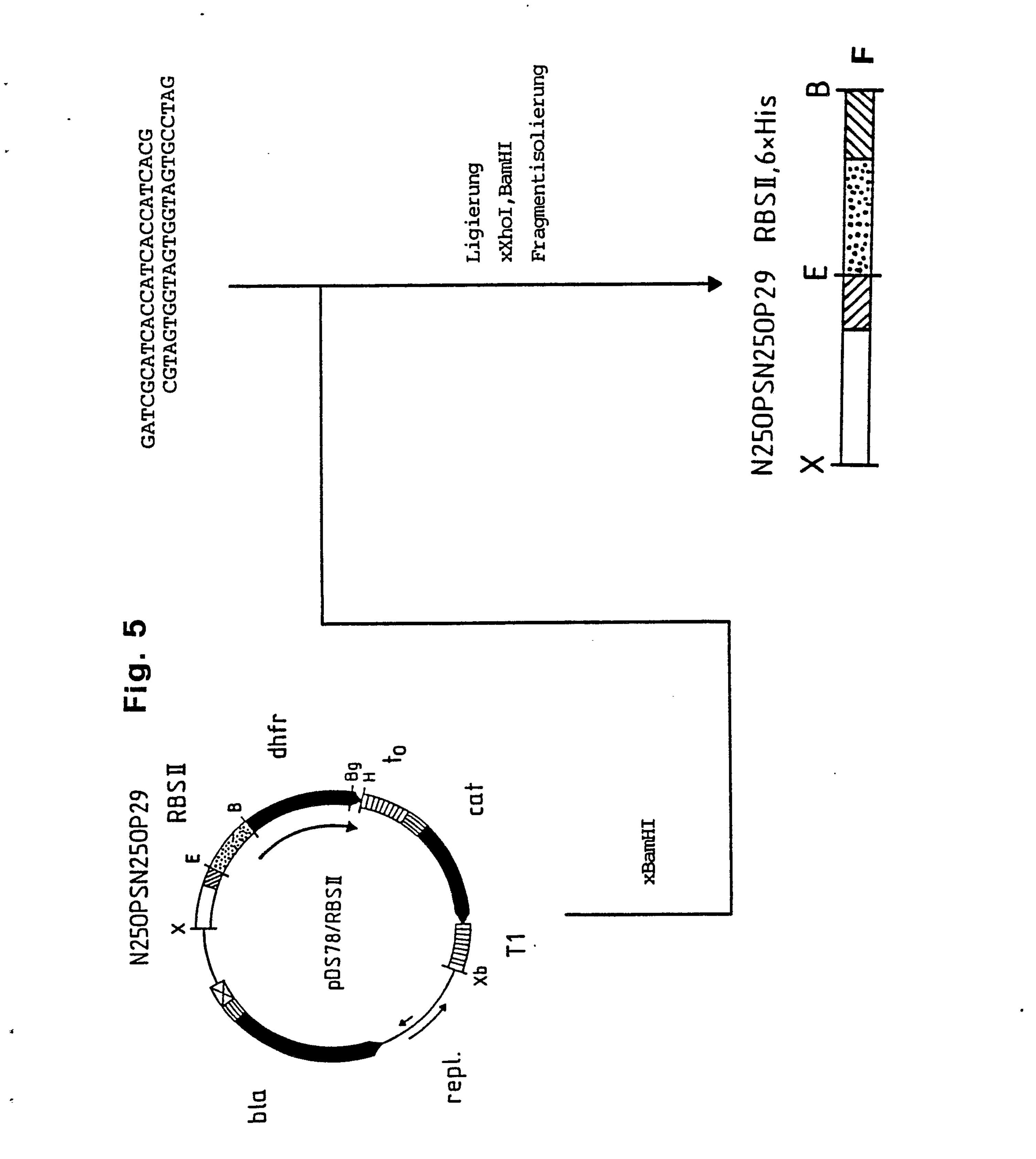
GATCGCATCACCATCACCATCACG
CGTAGTGGTAGTGGTAGTGCCTAG
Ligierung
xXhoI,BamHI
Fragmentisolierung
N250PSN250P29 RBSII,6×His
X
E
B
F
N250PSN250P29
RBSII
dhfr
X
E
B
Bg
H
to
cat
pDS78/RBSII
T1
Xb
repl.
bla
xBamHI

Fig. 5

Fig. 6

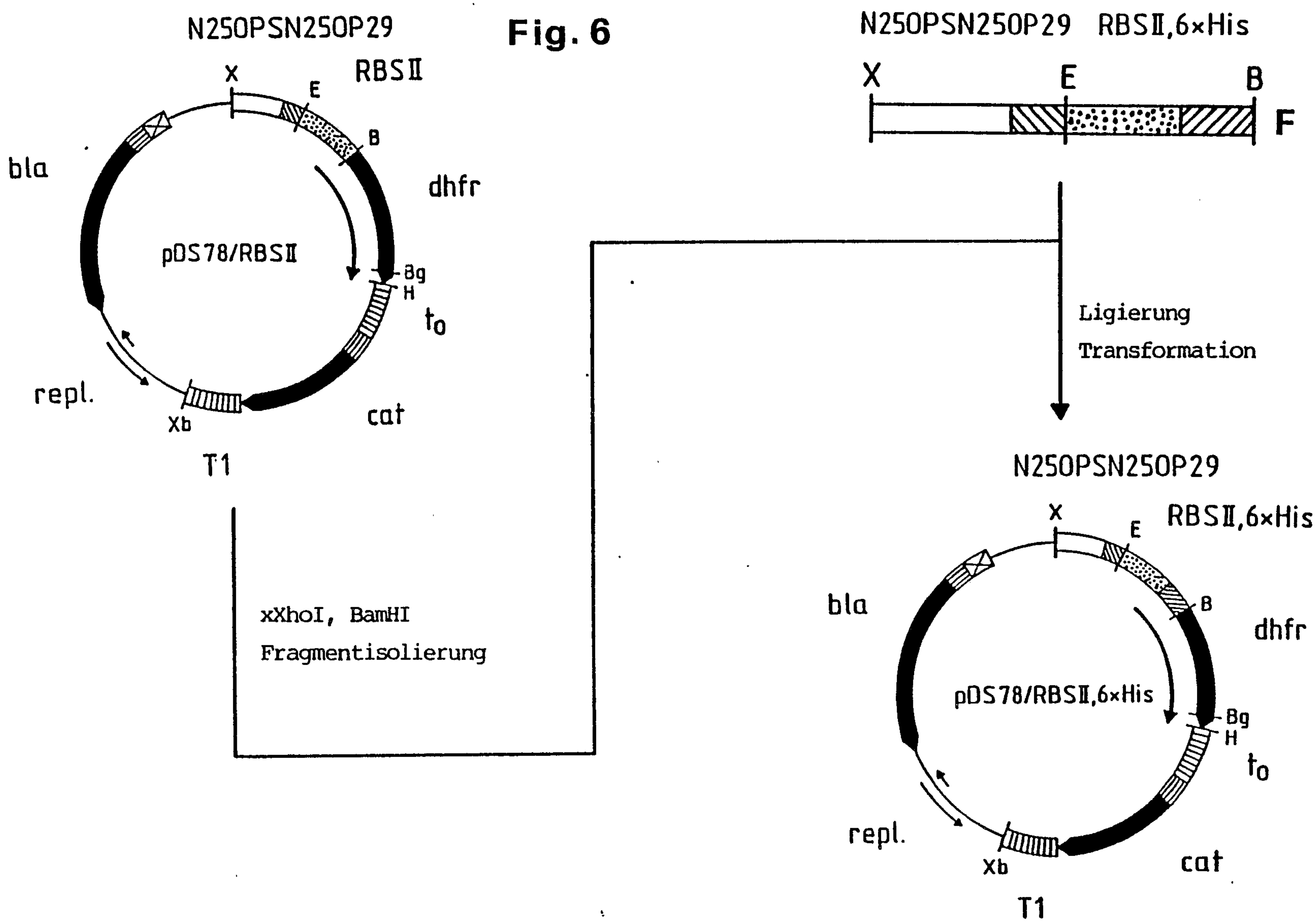
N250PSN250P29
RBSII
X
E
B
bla
dhfr
pDS78/RBSII
Bg
H
$t_0$
repl.
Xb
cat
T1
xXhoI, BamHI
Fragmentisolierung
N250PSN250P29 RBSII,6×His
X
E
B
F
Ligierung
Transformation
N250PSN250P29
X
E
RBSII,6×His
B
bla
dhfr
pDS78/RBSII,6×His
Bg
H
$t_0$
repl.
Xb
cat
T1

Fig. 7

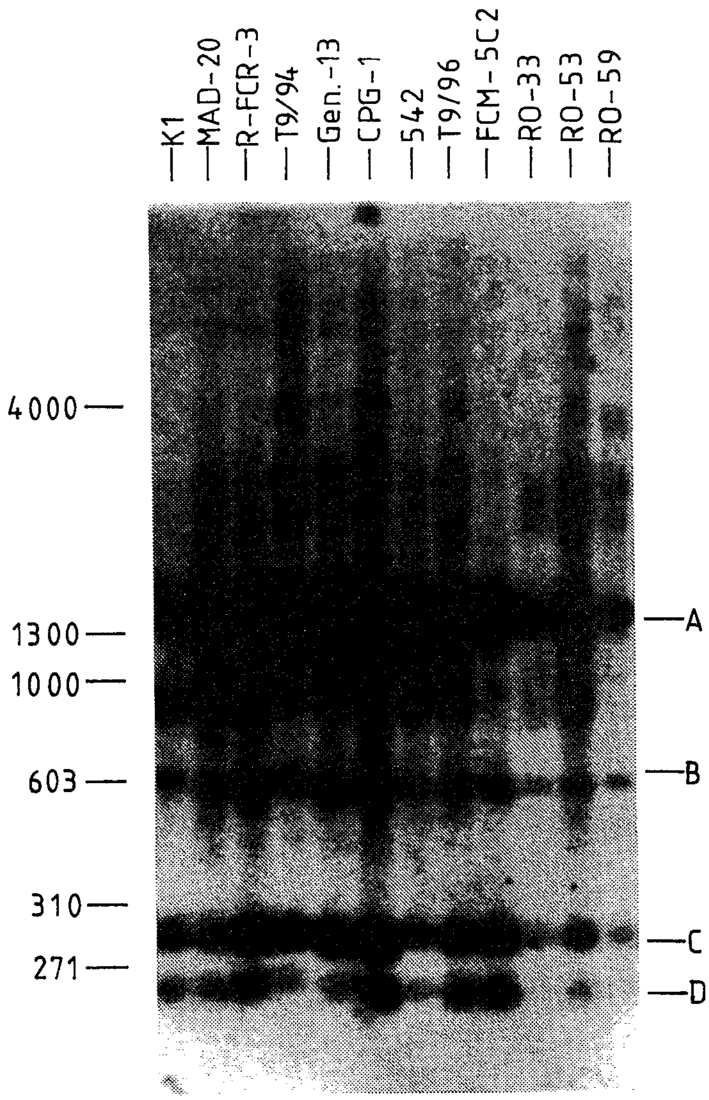
K1
MAD-20
R-FCR-3
T9/94
Gen.-13
CPG-1
542
T9/96
FCM-5C2
RO-33
RO-53
RO-59
4000
1300
1000
603
310
271
A
B
C
D

# Fig. 8

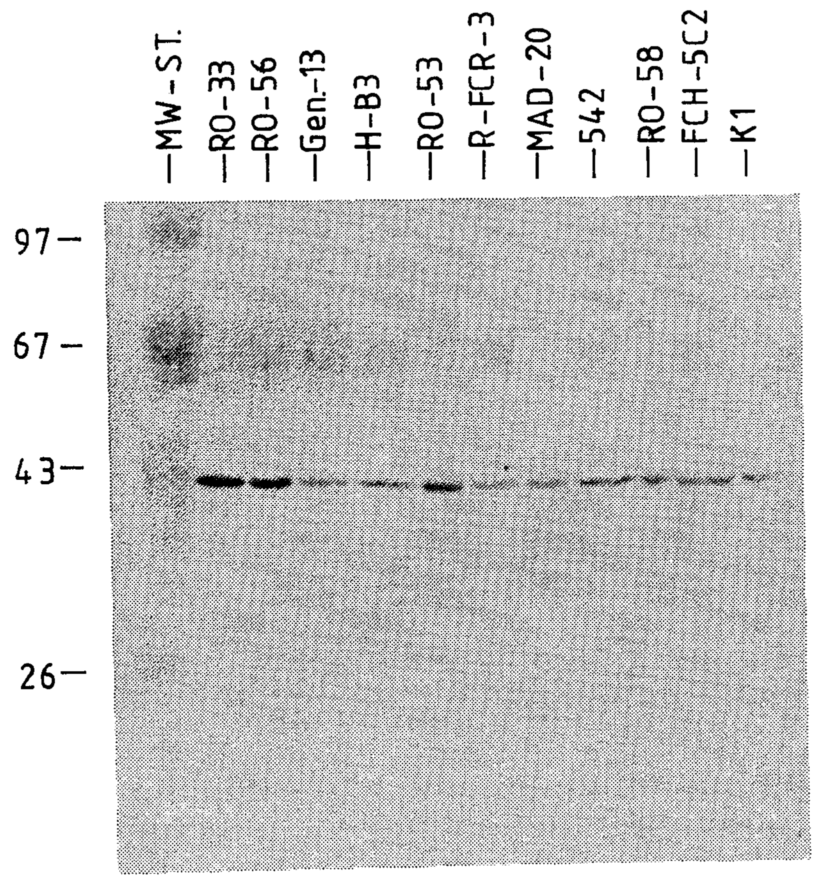
MW-ST.
RO-33
RO-56
Gen.-13
H-B3
RO-53
R-FCR-3
MAD-20
542
RO-58
FCH-5C2
K1
97
67
43
26

Fig. 9

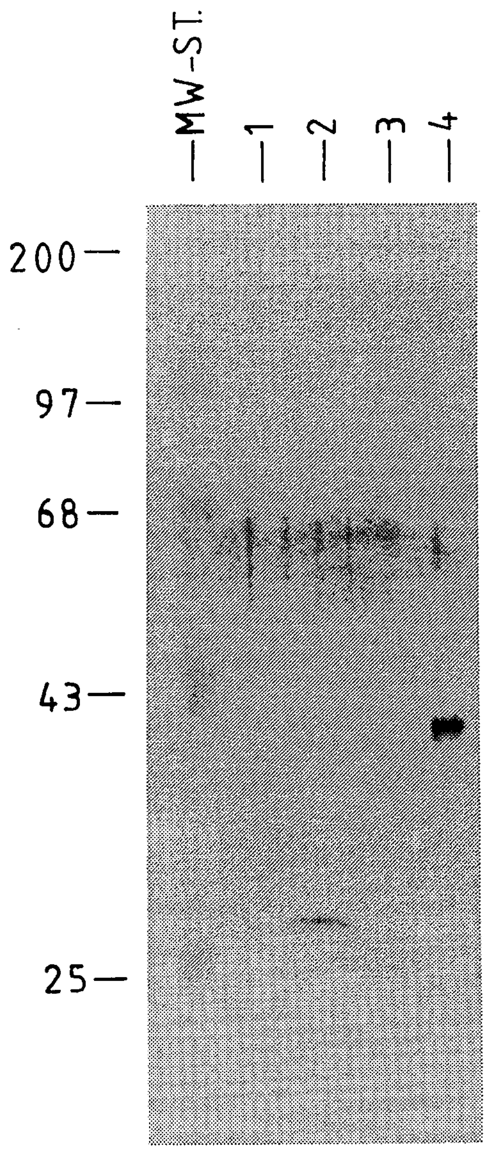

Fig. 10

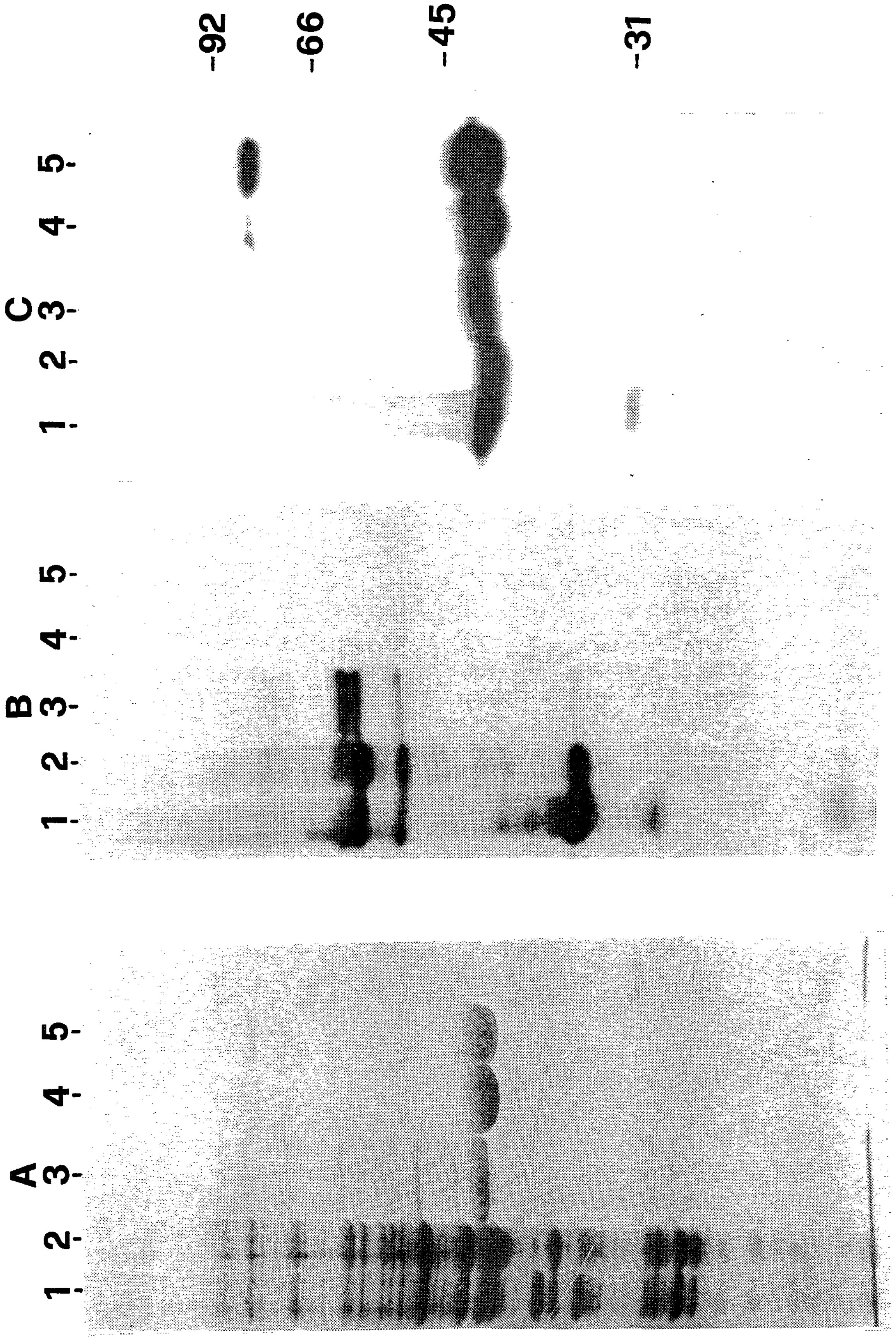

# Fig. 11a

```
        10         20         30         40         50         60
         |          |          |          |          |          |
CTCGAGAAAT CATAAAAAAT TTATTTGCTT TGTGAGCGGA TAACAATTAT AATAGATTCA
GAGCTCTTTA GTATTTTTTA AATAAACGAA ACACTCGCCT ATTGTTAATA TTATCTAAGT
                                                          (S)
ATTGTGAGCG GATAACAATT TCACACAGAA TTCATTAAAG AGGAGAAATT AACTATGAGA
TAACACTCGC CTATTGTTAA AGTGTGTCTT AAGTAATTTC TCCTCTTTAA TTGATACTCT

GGATCGCATC ACCATCACCA TCACGGATCC GAGCTTGCAT GCCAATATAT GAATGCCCCA
CCTAGCGTAG TGGTAGTGGT AGTGCCTAGG CTCGAACGTA CGGTTATATA CTTACGGGGT

AAAAAATTAC CAGCAGATGT TGCCGAAGAA TTAGCAACCA CCGCCCAAAA GCTTGTTCAA
TTTTTTAATG GTCGTCTACA ACGGCTTCTT AATCGTTGGT GGCGGGTTTT CGAACAAGTT

GCTGGAAAGG GAATTTTAGC TGCTGATGAA TCAACACAAA CCATTAAGAA AAGATTCGAC
CGACCTTTCC CTTAAAATCG ACGACTACTT AGTTGTGTTT GGTAATTCTT TTCTAAGCTG

AACATCAAAT TAGAGAACAC AATAGAAAAC AGAGCTAGCT ACAGAGATTT ATTATTTGGA
TTGTAGTTTA ATCTCTTGTG TTATCTTTTG TCTCGATCGA TGTCTCTAAA TAATAAACCT

ACTAAAGGAT TAGGAAAATT CATTTCAGGA GCAATTTTAT TTGAAGAAAC ATTATTTCAA
TGATTTCCTA ATCCTTTTAA GTAAAGTCCT CGTTAAAATA AACTTCTTTG TAATAAAGTT

AAGAATGAAG CCGGTGTACC ACAGGTTAAT TTATTACACA ATGAAAATAT AATTCCAGGT
TTCTTACTTC GGCCACATGG TGTCCAATTA AATAATGTGT TACTTTTATA TTAAGGTCCA

ATTAAGGTTG ATAAAGGTTT GGTTAACATT CCATGCACAG ATGAAGAAAA ATCAACTCAA
TAATTCCAAC TATTTCCAAA CCAATTGTAA GGTACGTGTC TACTTCTTTT TAGTTGAGTT

TGTTTAGATG GATTAGCAGA AAGATGCAAA GAGTATTATA AAGCTGGTGC AAGGTTTGCT
ACAAATCTAC CTAATCGTCT TTCTACGTTT CTCATAATAT TTCGACCACG TTCCAAACGA

AAATGGAGAA CAGTTTTAGT TATTGACACA GCCAAAGGAA AACCAACTGA TTTATCAAAT
TTTACCTCTT GTCAAAATCA ATAACTGTGT CGGTTTCCTT TTGGTTGACT AAATAGTTTA

CACGAAACTG CATGGGGATT GGCTAGATAT GCATCTATTT GTCAACAAAA TAGATTAGTT
GTGCTTTGAC GTACCCCTAA CCGATCTATA CGTAGATAAA CAGTTGTTTT ATCTAATCAA

CCAATTGTTG AACCTGAAAT TTTAGCTGAT GGACCACACT CAATTGAAGT TTGCGCAGTT
GGTTAACAAC TTGGACTTTA AAATCGACTA CCTGGTGTGA GTTAACTTCA AACGCGTCAA

GTAACTCAAA AAGTTTTATC ATGTGTATTT AAAGCTTTAC AAGAAAATGG TGTATTATTA
CATTGAGTTT TTCAAAATAG TACACATAAA TTTCGAAATG TTCTTTTACC ACATAATAAT

GAAGGTGCAT TGTTAAAACC AAACATGGTT ACTGCTGGTT ATGAATGTAC TGCTAAAACC
CTTCCACGTA ACAATTTTGG TTTGTACCAA TGACGACCAA TACTTACATG ACGATTTTGG

ACTACTCAAG ATGTTGGTTT CTTAACTGTC AGAACCTTAA GGAGAACTGT ACCACCAGCC
TGATGAGTTC TACAACCAAA GAATTGACAG TCTTGGAATT CCTCTTGACA TGGTGGTCGG
```

# Fig. 11b

TTACCAGGTG TTGTATTTTT ATCTGGAGGA CAATCAGAAG AAGAGGCTTC TGTTAATTTA
AATGGTCCAC AACATAAAAA TAGACCTCCT GTTAGTCTTC TTCTCCGAAG ACAATTAAAT

AATTCAATCA ATGCTTTGGG TCCACACCCA TGGGCTTTAA CCTTCTCTTA CGGTAGAGCT
TTAAGTTAGT TACGAAACCC AGGTGTGGGT ACCCGAAATT GGAAGAGAAT GCCATCTCGA

TTACAAGCTT CAGTATTGAA CACATGGCAA GGAAAGAAAG AAAATGTTGC AAAGGCAAGA
AATGTTCGAA GTCATAACTT GTGTACCGTT CCTTTCTTTC TTTTACAACG TTTCCGTTCT

GAAGTTTTAT TACAAAGAGC TGAAGCCAAC TCCTTAGCAA CTTATGGTAA ATACAAAGGA
CTTCAAAATA ATGTTTCTCG ACTTCGGTTG AGGAATCGTT GAATACCATT TATGTTTCCT

(T)
GGTGCAGGTG GTGAAAATGC AGGTGCTTCA TTATATGAAA AGAAATATGT CTATTAAAAA
CCACGTCCAC CACTTTTACG TCCACGAAGT AATATACTTT TCTTTATACA GATAATTTTT

CTTCACCAAC CAAAAATGAA TAATAATAAT AATAAATAAA TTACTAAATG AATGGTACTA
GAAGTGGTTG GTTTTTACTT ATTATTATTA TTATTTATTT AATGATTTAC TTACCATGAT

TATTTTTAAA AATAAGGGTA ATATATTTTC TGTATGTATA TATATATATA TATATACAAA
ATAAAAATTT TTATTCCCAT TATATAAAAG ACATACATAT ATATATATAT ATATATGTTT

ATATGTGAAA TTATAAAAAA AAAAAAAAAA AAAAAAAGGA ATTCCGGATC CTCCGGCATC
TATACACTTT AATATTTTTT TTTTTTTTTT TTTTTTTCCT TAAGGCCTAG GAGGCCGTAG

ATGGTTCGAC CATTGAACTG CATCGTCGCC GTGTCCCAAA ATATGGGGAT TGGCAAGAAC
TACCAAGCTG GTAACTTGAC GTAGCAGCGG CACAGGGTTT TATACCCCTA ACCGTTCTTG

GGAGACCTAC CCTGGCCTCC GCTCAGGAAC GAGTTCAAGT ACTTCCAAAG AATGACCACA
CCTCTGGATG GGACCGGAGG CGAGTCCTTG CTCAAGTTCA TGAAGGTTTC TTACTGGTGT

ACCTCTTCAG TGGAAGGTAA ACAGAATCTG GTGATTATGG GTAGGAAAAC CTGGTTCTCC
TGGAGAAGTC ACCTTCCATT TGTCTTAGAC CACTAATACC CATCCTTTTG GACCAAGAGG

ATTCCTGAGA AGAATCGACC TTTAAAGGAC AGAATTAATA TAGTTCTCAG TAGAGAACTC
TAAGGACTCT TCTTAGCTGG AAATTTCCTG TCTTAATTAT ATCAAGAGTC ATCTCTTGAG

AAAGAACCAC CACGAGGAGC TCATTTTCTT GCCAAAAGTT TGGATGATGC CTTAAGACTT
TTTCTTGGTG GTGCTCCTCG AGTAAAAGAA CGGTTTTCAA ACCTACTACG GAATTCTGAA

ATTGAACAAC CGGAATTGGC AAGTAAAGTA GACATGGTTT GGATAGTCGG AGGCAGTTCT
TAACTTGTTG GCCTTAACCG TTCATTTCAT CTGTACCAAA CCTATCAGCC TCCGTCAAGA

GTTTACCAGG AAGCCATGAA TCAACCAGGC CACCTTAGAC TCTTTGTGAC AAGGATCATG
CAAATGGTCC TTCGGTACTT AGTTGGTCCG GTGGAATCTG AGAAACACTG TTCCTAGTAC

CAGGAATTTG AAAGTGACAC GTTTTTCCCA GAAATTGATT TGGGGAAATA TAAACTTCTC
GTCCTTAAAC TTTCACTGTG CAAAAAGGGT CTTTAACTAA ACCCCTTTAT ATTTGAAGAG

CCAGAATACC CAGGCGTCCT CTCTGAGGTC CAGGAGGAAA AAGGCATCAA GTATAAGTTT
GGTCTTATGG GTCCGCAGGA GAGACTCCAG GTCCTCCTTT TTCCGTAGTT CATATTCAAA

GAAGTCTACG AGAAGAAAGG TTCCAGATCT GTTAACCTAG TTTAACAGGA AGATGCTTTC
CTTCAGATGC TCTTCTTTCC AAGGTCTAGA CAATTGGATC AAATTGTCCT TCTACGAAAG

# Fig. 11c

AAGTTCTCTG CTCCCCTCCT AAAGCTATGC ATTTTTATAA GACCATGGGA CTTTTGCTGG
TTCAAGAGAC GAGGGGAGGA TTTCGATACG TAAAAATATT CTGGTACCCT GAAAACGACC

CTTTAGATCC GGCCAAGCTT GGACTCCTGT TGATAGATCC AGTAATGACC TCAGAACTCC
GAAATCTAGG CCGGTTCGAA CCTGAGGACA ACTATCTAGG TCATTACTGG AGTCTTGAGG

ATCTGGATTT GTTCAGAACG CTCGGTTGCC GCCGGGCGTT TTTTATTGGT GAGAATCCAA
TAGACCTAAA CAAGTCTTGC GAGCCAACGG CGGCCCGCAA AAAATAACCA CTCTTAGGTT

GCTAGCTTGG CGAGATTTTC AGGAGCTAAG GAAGCTAAAA TGGAGAAAAA AATCACTGGA
CGATCGAACC GCTCTAAAAG TCCTCGATTC CTTCGATTTT ACCTCTTTTT TTAGTGACCT

TATACCACCG TTGATATATC CCAATGGCAT CGTAAAGAAC ATTTTGAGGC ATTTCAGTCA
ATATGGTGGC AACTATATAG GGTTACCGTA GCATTTCTTG TAAAACTCCG TAAAGTCAGT

GTTGCTCAAT GTACCTATAA CCAGACCGTT CAGCTGGATA TTACGGCCTT TTTAAAGACC
CAACGAGTTA CATGGATATT GGTCTGGCAA GTCGACCTAT AATGCCGGAA AAATTTCTGG

GTAAAGAAAA ATAAGCACAA GTTTTATCCG GCCTTTATTC ACATTCTTGC CCGCCTGATG
CATTTCTTTT TATTCGTGTT CAAAATAGGC CGGAAATAAG TGTAAGAACG GGCGGACTAC

AATGCTCATC CGGAATTTCG TATGGCAATG AAAGACGGTG AGCTGGTGAT ATGGGATAGT
TTACGAGTAG GCCTTAAAGC ATACCGTTAC TTTCTGCCAC TCGACCACTA TACCCTATCA

GTTCACCCTT GTTACACCGT TTTCCATGAG CAAACTGAAA CGTTTTCATC GCTCTGGAGT
CAAGTGGGAA CAATGTGGCA AAAGGTACTC GTTTGACTTT GCAAAAGTAG CGAGACCTCA

GAATACCACG ACGATTTCCG GCAGTTTCTA CACATATATT CGCAAGATGT GGCGTGTTAC
CTTATGGTGC TGCTAAAGGC CGTCAAAGAT GTGTATATAA GCGTTCTACA CCGCACAATG

GGTGAAAACC TGGCCTATTT CCCTAAAGGG TTTATTGAGA ATATGTTTTT CGTCTCAGCC
CCACTTTTGG ACCGGATAAA GGGATTTCCC AAATAACTCT TATACAAAAA GCAGAGTCGG

AATCCCTGGG TGAGTTTCAC CAGTTTTGAT TTAAACGTGG CCAATATGGA CAACTTCTTC
TTAGGGACCC ACTCAAAGTG GTCAAAACTA AATTTGCACC GGTTATACCT GTTGAAGAAG

GCCCCCGTTT TCACCATGGG CAAATATTAT ACGCAAGGCG ACAAGGTGCT GATGCCGCTG
CGGGGGCAAA AGTGGTACCC GTTTATAATA TGCGTTCCGC TGTTCCACGA CTACGGCGAC

GCGATTCAGG TTCATCATGC CGTCTGTGAT GGCTTCCATG TCGGCAGAAT GCTTAATGAA
CGCTAAGTCC AAGTAGTACG GCAGACACTA CCGAAGGTAC AGCCGTCTTA CGAATTACTT

TTACAACAGT ACTGCGATGA GTGGCAGGGC GGGGCGTAAT TTTTTTAAGG CAGTTATTGG
AATGTTGTCA TGACGCTACT CACCGTCCCG CCCCGCATTA AAAAAATTCC GTCAATAACC

TGCCCTTAAA CGCCTGGGGT AATGACTCTC TAGCTTGAGG CATCAAATAA AACGAAAGGC
ACGGGAATTT GCGGACCCCA TTACTGAGAG ATCGAACTCC GTAGTTTATT TTGCTTTCCG

TCAGTCGAAA GACTGGGCCT TTCGTTTTAT CTGTTGTTTG TCGGTGAACG CTCTCCTGAG
AGTCAGCTTT CTGACCCGGA AAGCAAAATA GACAACAAAC AGCCACTTGC GAGAGGACTC

TAGGACAAAT CCGCCGCTCT AGAGCTGCCT CGCGCGTTTC GGTGATGACG GTGAAAACCT
ATCCTGTTTA GGCGGCGAGA TCTCGACGGA GCGCGCAAAG CCACTACTGC CACTTTTGGA

CTGACACATG CAGCTCCCGG AGACGGTCAC AGCTTGTCTG TAAGCGGATG CCGGGAGCAG

# Fig. 11d

```
GACTGTGTAC GTCGAGGGCC TCTGCCAGTG TCGAACAGAC ATTCGCCTAC GGCCCTCGTC

ACAAGCCCGT CAGGGCGCGT CAGCGGGTGT TGGCGGGTGT CGGGGCGCAG CCATGACCCA
TGTTCGGGCA GTCCCGCGCA GTCGCCCACA ACCGCCCACA GCCCCGCGTC GGTACTGGGT

GTCACGTAGC GATAGCGGAG TGTATACTGG CTTAACTATG CGGCATCAGA GCAGATTGTA
CAGTGCATCG CTATCGCCTC ACATATGACC GAATTGATAC GCCGTAGTCT CGTCTAACAT

CTGAGAGTGC ACCATATGCG GTGTGAAATA CCGCACAGAT GCGTAAGGAG AAAATACCGC
GACTCTCACG TGGTATACGC CACACTTTAT GGCGTGTCTA CGCATTCCTC TTTTATGGCG

ATCAGGCGCT CTTCCGCTTC CTCGCTCACT GACTCGCTGC GCTCGGTCTG TCGGCTGCGG
TAGTCCGCGA GAAGGCGAAG GAGCGAGTGA CTGAGCGACG CGAGCCAGAC AGCCGACGCC

CGAGCGGTAT CAGCTCACTC AAAGGCGGTA ATACGGTTAT CCACAGAATC AGGGGATAAC
GCTCGCCATA GTCGAGTGAG TTTCCGCCAT TATGCCAATA GGTGTCTTAG TCCCCTATTG

GCAGGAAAGA ACATGTGAGC AAAAGGCCAG CAAAAGGCCA GGAACCGTAA AAAGGCCGCG
CGTCCTTTCT TGTACACTCG TTTTCCGGTC GTTTTCCGGT CCTTGGCATT TTTCCGGCGC

TTGCTGGCGT TTTTCCATAG GCTCCGCCCC CCTGACGAGC ATCACAAAAA TCGACGCTCA
AACGACCGCA AAAAGGTATC CGAGGCGGGG GGACTGCTCG TAGTGTTTTT AGCTGCGAGT

AGTCAGAGGT GGCGAAACCC GACAGGACTA TAAAGATACC AGGCGTTTCC CCCTGGAAGC
TCAGTCTCCA CCGCTTTGGG CTGTCCTGAT ATTTCTATGG TCCGCAAAGG GGGACCTTCG

TCCCTCGTGC GCTCTCCTGT TCCGACCCTG CCGCTTACCG GATACCTGTC CGCCTTTCTC
AGGGAGCACG CGAGAGGACA AGGCTGGGAC GGCGAATGGC CTATGGACAG GCGGAAAGAG

CCTTCGGGAA GCGTGGCGCT TTCTCAATGC TCACGCTGTA GGTATCTCAG TTCGGTGTAG
GGAAGCCCTT CGCACCGCGA AAGAGTTACG AGTGCGACAT CCATAGAGTC AAGCCACATC

GTCGTTCGCT CCAAGCTGGG CTGTGTGCAC GAACCCCCCG TTCAGCCCGA CCGCTGCGCC
CAGCAAGCGA GGTTCGACCC GACACACGTG CTTGGGGGGC AAGTCGGGCT GGCGACGCGG

TTATCCGGTA ACTATCGTCT TGAGTCCAAC CCGGTAAGAC ACGACTTATC GCCACTGGCA
AATAGGCCAT TGATAGCAGA ACTCAGGTTG GGCCATTCTG TGCTGAATAG CGGTGACCGT

GCAGCCACTG GTAACAGGAT TAGCAGAGCG AGGTATGTAG GCGGTGCTAC AGAGTTCTTG
CGTCGGTGAC CATTGTCCTA ATCGTCTCGC TCCATACATC CGCCACGATG TCTCAAGAAC

AAGTGGTGGC CTAACTACGG CTACACTAGA AGGACAGTAT TTGGTATCTG CGCTCTGCTG
TTCACCACCG GATTGATGCC GATGTGATCT TCCTGTCATA AACCATAGAC GCGAGACGAC

AAGCCAGTTA CCTTCGGAAA AAGAGTTGGT AGCTCTTGAT CCGGCAAACA AACCACCGCT
TTCGGTCAAT GGAAGCCTTT TTCTCAACCA TCGAGAACTA GGCCGTTTGT TTGGTGGCGA

GGTAGCGGTG GTTTTTTTGT TTGCAAGCAG CAGATTACGC GCAGAAAAAA AGGATCTCAA
CCATCGCCAC CAAAAAAACA AACGTTCGTC GTCTAATGCG CGTCTTTTTT TCCTAGAGTT

GAAGATCCTT TGATCTTTTC TACGGGGTCT GACGCTCAGT GGAACGAAAA CTCACGTTAA
CTTCTAGGAA ACTAGAAAAG ATGCCCCAGA CTGCGAGTCA CCTTGCTTTT GAGTGCAATT

GGGATTTTGG TCATGAGATT ATCAAAAAGG ATCTTCACCT AGATCCTTTT AAATTAAAAA
CCCTAAAACC AGTACTCTAA TAGTTTTTCC TAGAAGTGGA TCTAGGAAAA TTTAATTTTT
```

# Fig. 11e

```
TGAAGTTTTA AATCAATCTA AAGTATATAT GAGTAAACTT GGTCTGACAG TTACCAATGC
ACTTCAAAAT TTAGTTAGAT TTCATATATA CTCATTTGAA CCAGACTGTC AATGGTTACG

TTAATCAGTG AGGCACCTAT CTCAGCGATC TGTCTATTTC GTTCATCCAT AGCTGCCTGA
AATTAGTCAC TCCGTGGATA GAGTCGCTAG ACAGATAAAG CAAGTAGGTA TCGACGGACT

CTCCCCGTCG TGTAGATAAC TACGATACGG GAGGGCTTAC CATCTGGCCC CAGTGCTGCA
GAGGGGCAGC ACATCTATTG ATGCTATGCC CTCCCGAATG GTAGACCGGG GTCACGACGT

ATGATACCGC GAGACCCACG CTCACCGGCT CCAGATTTAT CAGCAATAAA CCAGCCAGCC
TACTATGGCG CTCTGGGTGC GAGTGGCCGA GGTCTAAATA GTCGTTATTT GGTCGGTCGG

GGAAGGGCCG AGCGCAGAAG TGGTCCTGCA ACTTTATCCG CCTCCATCCA GTCTATTAAT
CCTTCCCGGC TCGCGTCTTC ACCAGGACGT TGAAATAGGC GGAGGTAGGT CAGATAATTA

TGTTGCCGGG AAGCTAGAGT AAGTAGTTCG CCAGTTAATA GTTTGCGCAA CGTTGTTGCC
ACAACGGCCC TTCGATCTCA TTCATCAAGC GGTCAATTAT CAAACGCGTT GCAACAACGG

ATTGCTACAG GCATCGTGGT GTCACGCTCG TCGTTTGGTA TGGCTTCATT CAGCTCCGGT
TAACGATGTC CGTAGCACCA CAGTGCGAGC AGCAAACCAT ACCGAAGTAA GTCGAGGCCA

TCCCAACGAT CAAGGCGAGT TACATGATCC CCCATGTTGT GCAAAAAAGC GGTTAGCTCC
AGGGTTGCTA GTTCCGCTCA ATGTACTAGG GGGTACAACA CGTTTTTTCG CCAATCGAGG

TTCGGTCCTC CGATCGTTGT CAGAAGTAAG TTGGCCGCAG TGTTATCACT CATGGTTATG
AAGCCAGGAG GCTAGCAACA GTCTTCATTC AACCGGCGTC ACAATAGTGA GTACCAATAC

GCAGCACTGC ATAATTCTCT TACTGTCATG CCATCCGTAA GATGCTTTTC TGTGACTGGT
CGTCGTGACG TATTAAGAGA ATGACAGTAC GGTAGGCATT CTACGAAAAG ACACTGACCA

GAGTACTCAA CCAAGTCATT CTGAGAATAG TGTATGCGGC GACCGAGTTG CTCTTGCCCG
CTCATGAGTT GGTTCAGTAA GACTCTTATC ACATACGCCG CTGGCTCAAC GAGAACGGGC

GCGTCAATAC GGGATAATAC CGCGCCACAT AGCAGAACTT TAAAAGTGCT CATCATTGGA
CGCAGTTATG CCCTATTATG GCGCGGTGTA TCGTCTTGAA ATTTTCACGA GTAGTAACCT

AAACGTTCTT CGGGGCGAAA ACTCTCAAGG ATCTTACCGC TGTTGAGATC CAGTTCGATG
TTTGCAAGAA GCCCCGCTTT TGAGAGTTCC TAGAATGGCG ACAACTCTAG GTCAAGCTAC

TAACCCACTC GTGCACCCAA CTGATCTTCA GCATCTTTTA CTTTCACCAG CGTTTCTGGG
ATTGGGTGAG CACGTGGGTT GACTAGAAGT CGTAGAAAAT GAAAGTGGTC GCAAAGACCC

TGAGCAAAAA CAGGAAGGCA AAATGCCGCA AAAAAGGGAA TAAGGGCGAC ACGGAAATGT
ACTCGTTTTT GTCCTTCCGT TTTACGGCGT TTTTTCCCTT ATTCCCGCTG TGCCTTTACA

TGAATACTCA TACTCTTCCT TTTTCAATAT TATTGAAGCA TTTATCAGGG TTATTGTCTC
ACTTATGAGT ATGAGAAGGA AAAAGTTATA ATAACTTCGT AAATAGTCCC AATAACAGAG

ATGAGCGGAT ACATATTTGA ATGTATTTAG AAAAATAAAC AAATAGGGGT TCCGCGCACA
TACTCGCCTA TGTATAAACT TACATAAATC TTTTTATTTG TTTATCCCCA AGGCGCGTGT

TTTCCCCGAA AAGTGCCACC TGACGTCTAA GAAACCATTA TTATCATGAC ATTAACCTAT
AAAGGGGCTT TTCACGGTGG ACTGCAGATT CTTTGGTAAT AATAGTACTG TAATTGGATA

AAAAATAGGC GTATCACGAG GCCCTTTCGT CTTCAC
TTTTTATCCG CATAGTGCTC CGGGAAAGCA GAAGTG
```

# Fig. 12a

ATATATATTATATACTATTATATATGTATTCATTTTATTCTGCTCACATTATTTATGCAT
ATGCTTCCTTTATAATAAATATATTCGTATTAACATTCAAGAAATGAGGAACGAAATATT
CCTTAATTTACATATGTATTTTTTTATTAATTGAAAAAAAAAAAAAAAATAGTAAAAATA
AGTATAGGCATATATTGAATAATGTGCTGTTGAATTGATTTATATATATATATATATATA
TATATGTATATTTATTTATATTTATACATATGGGAATATTATATATTTTCCTTTTTTCTT

M25

ATTTTTATATTTTTATATTTTTTTAGGCTCATTGCACTGAATAT

```
                     10                  20                  30                  40
ATG AAT GCC CCA AAA AAA TTA CCA GCA GAT GTT GCC GAA GAA TTA
MET Asn Ala Pro Lys Lys Leu Pro Ala Asp Val Ala Glu Glu Leu

         50                  60                  70                  80                  90
GCA ACC ACC GCC CAA AAG CTT GTT CAA GCT GGA AAG GGA ATT TTA
Ala Thr Thr Ala Gln Lys Leu Val Gln Ala Gly Lys Gly Ile Leu

                    100                 110                 120                 130
GCT GCT GAT GAA TCA ACA CAA ACC ATT AAG AAA AGA TTC GAC AAC
Ala Ala Asp Glu Ser Thr Gln Thr Ile Lys Lys Arg Phe Asp Asn

        140                 150                 160                 170                 180
ATC AAA TTA GAG AAC ACA ATA GAA AAC AGA GCT AGC TAC AGA GAT
Ile Lys Leu Glu Asn Thr Ile Glu Asn Arg Ala Ser Tyr Arg Asp

                    190                 200                 210                 220
TTA TTA TTT GGA ACT AAA GGA TTA GGA AAA TTC ATT TCA GGA GCA
Leu Leu Phe Gly Thr Lys Gly Leu Gly Lys Phe Ile Ser Gly Ala

        230                 240                 250                 260                 270
ATT TTA TTT GAA GAA ACA TTA TTT CAA AAG AAT GAA GCC GGT GTA
Ile Leu Phe Glu Glu Thr Leu Phe Gln Lys Asn Glu Ala Gly Val
```

# Fig. 12b

```
     Gln
     CAG  280         290         300         310
           |           |           |           |
CCA  ATG  GTT AAT TTA TTA CAC AAT GAA AAT ATA ATT CCA GGT ATT
Pro  MET  Val Asn Leu Leu His Asn Glu Asn Ile Ile Pro Gly Ile
      ▲

    320                     340         350         360
     |                       |           |           |
AAG GTT GAT AAA GGT TTG GTT AAC ATT CCA TGC ACA GAT GAA GAA
Lys Val Asp Lys Gly Leu Val Asn Ile Pro Cys Thr Asp Glu Glu

                    Cys
            370     TGT     380         390         400
             |               |           |           |
AAA TCA ACT CAA     GGT TTA GAT GGA TTA GCA GAA AGA TGC AAA GAG
Lys Ser Thr Gln     Gly Leu Asp Gly Leu Ala Glu Arg Cys Lys Glu
                     ▲

    410         420         430         440         450
     |           |           |           |           |
TAT TAT AAA GCT GGT GCA AGG TTT GCT AAA TGG AGA ACA GTT TTA
Tyr Tyr Lys Ala Gly Ala Arg Phe Ala Lys Trp Arg Thr Val Leu

            460         470         480         490
             |           |           |           |
GTT ATT GAC ACA GCC AAA GGA AAA CCA ACT GAT TTA TCA AAT CAC
Val Ile Asp Thr Ala Lys Gly Lys Pro Thr Asp Leu Ser Asn His

    500         510         520         530         540
     |           |           |           |           |
GAA ACT GCA TGG GGA TTG GCT AGA TAT GCA TCT ATT TGT CAA CAA
Glu Thr Ala Trp Gly Leu Ala Arg Tyr Ala Ser Ile Cys Gln Gln

            550         560         570         580
             |           |           |           |
AAT AGA TTA GTT CCA ATT GTT GAA CCT GAA ATT TTA GCT GAT GGA
Asn Arg Leu Val Pro Ile Val Glu Pro Glu Ile Leu Ala Asp Gly
```

# Fig. 12c

590 600 610 620 630

RO-33 (at 619)

CCA CAC TCA ATT GAA GTT TGT (TGC) GCA GTT GTA ACT CAA AAA GTT TTA

Pro His Ser Ile Glu Val Cys Ala Val Val Thr Gln Lys Val Leu

▲ (Cys) ● (Val)

640 650 660 670

TCA TGT GTA TTT AAA GCT TTA CAA GAA AAT GGT GTA TTA TTA GAA

Ser Cys Val Phe Lys Ala Leu Gln Glu Asn Gly Val Leu Leu Glu

680 690 700 710 720

GGT GCA TTG TTA AAA CCA AAC ATG GTT ACT GCT GGT TAT GAA TGT

Gly Ala Leu Leu Lys Pro Asn MET Val Thr Ala Gly Tyr Glu Cys

730 740 750 760

ACT GCT AAA ACC ACT ACT CAA GAT GTT GGT TTC TTA ACT GTC AGA

Thr Ala Lys Thr Thr Thr Gln Asp Val Gly Phe Leu Thr Val Arg

770 780 790 800 810

ACC TTA AGG AGA ACT GTA CCA CCA GCC TTA CCA GGT GTT GTA TTT

Thr Leu Arg Arg Thr Val Pro Pro Ala Leu Pro Gly Val Val Phe

820 830 840 850

TTA TCT GGA GGA CAA TCA GAA GAA GAG GCT TCT GTT AAT TTA AAT

Leu Ser Gly Gly Gln Ser Glu Glu Glu Ala Ser Val Asn Leu Asn

860 870 880 890 900

TCA ATC AAT GCT TTG GGT CCA CAC CCA TGG GCT TTA ACC TTC TCT

Ser Ile Asn Ala Leu Gly Pro His Pro Trp Ala Leu Thr Phe Ser

## Fig. 12d

```
            910                 920                 930                 940
             |                   |                   |                   |
TAC GGT AGA GCT TTA CAA GCT TCA GTA TTG AAC ACA TGG CAA GGA
Tyr Gly Arg Ala Leu Gln Ala Ser Val Leu Asn Thr Trp Gln Gly

    950                 960                 970                 980                 990
     |                   |                   |                   |                   |
AAG AAA GAA AAT GTT GCA AAG GCA AGA GAA GTT TTA TTA CAA AGA
Lys Lys Glu Asn Val Ala Lys Ala Arg Glu Val Leu Leu Gln Arg

            1000                1010                1020                1030
             |                   |                   |                   |
GCT GAA GCC AAC TCC TTA GCA ACT TAT GGT AAA TAC AAA GGA GGT
Ala Glu Ala Asn Ser Leu Ala Thr Tyr Gly Lys Tyr Lys Gly Gly

    1040                1050                1060                1070                1080
     |                   |                   |                   |                   |
GCA GGT GGT GAA AAT GCA GGT GCT TCA TTA TAT GAA AAG AAA TAT
Ala Gly Gly Glu Asn Ala Gly Ala Ser Leu Tyr Glu Lys Lys Tyr

GTC TAT TAAAAACTTCACCAACCAAAAATGAATAATAATAATAATAAATAAATTAC
Val Tyr ■ ——————————————————————————— M 25 ➜ ——————————

TAAATGAATGGTACTATATTTTTAAAAATAAGGGTAATATATTTTCTGTATGTATATAT
————————————————AAATTATCCTTATACATGAACCATGTACCATAAATTAAAATAT

ATATATATATATACAAAATATGTGAAATTATAAAAAAAAAAAAAAAAAAAAAAAGGAAT
TTACATGAACATGTACATAAATT
                     ◀— M25
```

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 88114016.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,E | EP - A1 - 0 283 829 (F.HOFFMANN-LA ROCHE & CO AKTIENGESELLSCHAFT)<br>* Patentansprüche *<br>-- | 1-25 | C 12 N 15/00<br>C 07 K 13/00<br>C 07 H 21/04<br>C 12 N 1/20<br>C 12 P 21/00<br>A 61 K 39/015<br>A 61 K 37/02 |
| A | WO - A1 - 86/04 922 (MEMORIAL SLOAN-KETTERING CANCER CENTER)<br>* Patentansprüche *<br>-- | 1-25 | |
| A | PROCEEDINGS OF THE NATIONAL ACADEMY OF SCIENCES OF THE UNITED STATES OF AMERICA, Band 81, Nr. 12, Juni 1984 (Baltimore, USA)<br>M.J.MCGARVEY et al. "Identification and expression in Escherichia coli of merozoite stage-specific genes of the human malarial parasite Plasmodium falciparum"<br>Seiten 3690-3694<br>* Gesamt *<br>-- | 1-10, 12,15, 17,20, 21 | |

**RECHERCHIERTE SACHGEBIETE (Int. Cl.4)**

C 12 N
C 07 K
C 07 H
C 12 P
A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-25
Unvollständig recherchierte Patentansprüche: -
Nicht recherchierte Patentansprüche: 26
Grund für die Beschränkung der Recherche:

(Artikel 52(4) EPÜ)

| Recherchenort | Abschlußdatum der Recherche | Prüfer |
|---|---|---|
| WIEN | 07-12-1988 | WOLF |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

# EUROPÄISCHER TEILRECHERCHENBERICHT

Nummer der Anmeldung

EP 88114016.4

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.4) |
|---|---|---|---|
| A | NUCLEIC ACIDS RESEARCH, Band 14, Nr. 8, 25. April 1986 (London)<br>J.L.WEBER et al. "Variation in the gene encoding a major merozoite surface antigen of the human malaria parasite Plasmodium falciparum"<br>Seiten 3311-3323<br>* Gesamt *<br>-- | 1-10, 12,15, 17,20, 21 | |
| A | THE EMBO JOURNAL, Band 4, Nr. 4, 1985 (Oxford, GB)<br>A.CHEUNG et al. "Cloning and expression in Escherichia coli of a surface antigen of Plasmodium falciparum merozoites"<br>Seiten 1007-1012<br>* Gesamt *<br>---- | 1-10, 12,15, 17,20, 21 | RECHERCHIERTE SACHGEBIETE (Int. Cl.4) |